# EUROPEAN PATENT APPLICATION

(11) **EP 3 181 579 A1**
(43) Date of publication of application: **21.06.2017**
(21) Application number: 17153422.5
(22) Date of filing: 16.10.2008
(51) Int. Cl.: C07K 14/325, C12N 15/82, A01H 5/00

(54) **AXMI-066 AND AXMI-076: DELTA-ENDOTOXIN PROTEINS AND METHODS FOR THEIR USE**

(30) Priority: 16.10.2007 US 980439 P
(62) Divisional of application: 08840217.7
(71) Applicant: Athenix Corporation, Research Triangle Park, NC 27709 (US)
(72) Inventor: TOMSO, Daniel J., Bahama, NC 27503 (US); SAMPSON, Kimberly S., Durham, NC 27713 (US); AGARWAL, Shruti, Durham, NC 27713 (US); VOLRATH, Sandra, Durham, NC 27707 (US); DESAI, Nalini, Chapel Hill, NC 27514 (US); HEINRICHS, Volker, Raleigh, NC 27614 (US)
(74) Representative: Lejeune, Anne Claire Marie

(57) **Abstract**

Compositions and methods for conferring pesticidal activity to bacteria, plants, plant cells, tissues and seeds are provided. Compositions comprising a coding sequence for pesticidal polypeptides are provided. The coding sequences can be used in DNA constructs or expression cassettes for transformation and expression in plants and bacteria. Compositions also comprise transformed bacteria, plants, plant cells, tissues, and seeds. In particular, isolated pesticidal nucleic acid molecules are provided. Additionally, amino acid sequences corresponding to the polynucleotides are encompassed. In particular, the present invention provides for isolated nucleic acid molecules comprising nucleotide sequences encoding the amino acid sequence shown in SEQ ID NO:5, 2, or 10, the nucleotide sequence set forth in SEQ ID NO:4, 1, 3, 4, 6, 9, or 11, or the nucleotide sequence deposited in a bacterial host as Accession No. B-50045, as well as variants and fragments thereof.

## Description

### FIELD OF THE INVENTION

This invention relates to the field of molecular biology. Provided are novel genes that encode pesticidal proteins. These proteins and the nucleic acid sequences that encode them are useful in preparing pesticidal formulations and in the production of transgenic pest-resistant plants.

### BACKGROUND OF THE INVENTION

*Bacillus thuringiensis* is a Gram-positive spore forming soil bacterium characterized by its ability to produce crystalline inclusions that are specifically toxic to certain orders and species of insects, but are harmless to plants and other non-targeted organisms. For this reason, compositions including *Bacillus thuringiensis* strains or their insecticidal proteins can be used as environmentally-acceptable insecticides to control agricultural insect pests or insect vectors for a variety of human or animal diseases.

Crystal (Cry) proteins (delta-endotoxins) from *Bacillus thuringiensis* have potent insecticidal activity against predominantly Lepidopteran, Dipteran, and Coleopteran larvae. These proteins also have shown activity against *Hymenoptera, Homoptera, Phthiraptera, Mallophaga,* and *Acari* pest orders, as well as other invertebrate orders such as *Nemathelminthes, Platyhelminthes,* and *Sarcomastigorphora* (Feitelson (1993) The Bacillus Thuringiensis family tree. In Advanced Engineered Pesticides, Marcel Dekker, Inc., New York, N.Y.) These proteins were originally classified as CryI to CryV based primarily on their insecticidal activity. The major classes were *Lepidoptera-*specific (I), *Lepidoptera-* and *Diptera-specific* (II), *Coleoptera-specific* (III), *Diptera-*specific (IV), and nematode-specific (V) and (VI). The proteins were further classified into subfamilies; more highly related proteins within each family were assigned divisional letters such as *Cry1A, Cry1B, Cry1C,* etc. Even more closely related proteins within each division were given names such as *Cry1C1, Cry1C2,* etc.

A new nomenclature was recently described for the Cry genes based upon amino acid sequence homology rather than insect target specificity (Crickmore et al. (1998) Microbiol. Mol. Biol. Rev. 62:807-813). In the new classification, each toxin is assigned a unique name incorporating a primary rank (an Arabic number), a secondary rank (an uppercase letter), a tertiary rank (a lowercase letter), and a quaternary rank (another Arabic number). In the new classification, Roman numerals have been exchanged for Arabic numerals in the primary rank. Proteins with less than 45% sequence identity have different primary ranks, and the criteria for secondary and tertiary ranks are 78% and 95%, respectively.

The crystal protein does not exhibit insecticidal activity until it has been ingested and solubilized in the insect midgut. The ingested protoxin is hydrolyzed by proteases in the insect digestive tract to an active toxic molecule. (Höfte and Whiteley (1989) Microbiol. Rev. 53:242-255). This toxin binds to apical brush border receptors in the midgut of the target larvae and inserts into the apical membrane creating ion channels or pores, resulting in larval death.

Delta-endotoxins generally have five conserved sequence domains, and three conserved structural domains (see, for example, de Maagd et al. (2001) Trends Genetics 17:193-199). The first conserved structural domain consists of seven alpha helices and is involved in membrane insertion and pore formation. Domain II consists of three beta-sheets arranged in a Greek key configuration, and domain III consists of two antiparallel beta-sheets in "jelly-roll" formation (de Maagd *et al.,* 2001, supra). Domains II and III are involved in receptor recognition and binding, and are therefore considered determinants of toxin specificity.

Because of the devastation that insects can confer, and the improvement in yield by controlling insect pests, there is a continual need to discover new forms of pesticidal toxins.

### SUMMARY OF INVENTION

Compositions and methods for conferring pesticidal activity to bacteria, plants, plant cells, tissues and seeds are provided. Compositions include nucleic acid molecules encoding sequences for pesticidal and insectidal polypeptides, vectors comprising those nucleic acid molecules, and host cells comprising the vectors. Compositions also include the pesticidal polypeptide sequences and antibodies to those polypeptides. The nucleotide sequences can be used in DNA constructs or expression cassettes for transformation and expression in organisms, including microorganisms and plants. The nucleotide or amino acid sequences may be synthetic sequences that have been designed for expression in an organism including, but not limited to, a microorganism or a plant. Compositions also comprise transformed bacteria, plants, plant cells, tissues, and seeds.

In particular, isolated nucleic acid molecules are provided that encode a pesticidal protein. Additionally, amino acid sequences corresponding to the pesticidal protein are encompassed. In particular, the present invention provides for an isolated nucleic acid molecule comprising a nucleotide sequence encoding the amino acid sequence shown in SEQ ID NO:2 or 5, a nucleotide sequence set forth in SEQ ID NO:1, 3, 4, 6, 9, or 11, or the delta-endotoxin nucleotide sequence of the DNA insert of the plasmid deposited in a bacterial host as Accession No. NRRL B-50045, as well as variants and fragments thereof. Nucleotide sequences that are complementary to a nucleotide sequence of the invention, or that hybridize to a sequence of the invention are also encompassed.

Methods are provided for producing the polypeptides of the invention, and for using those polypeptides for controlling or killing a lepidopteran, coleopteran, nematode, or dipteran pest. Methods and kits for detecting the nucleic acids and polypeptides of the invention in a sample are also included.

The compositions and methods of the invention are useful for the production of organisms with enhanced pest resistance or tolerance. These organisms and compositions comprising the organisms are desirable for agricultural purposes. The compositions of the invention are also useful for generating altered or improved proteins that have pesticidal activity, or for detecting the presence of pesticidal proteins or nucleic acids in products or organisms.

### DESCRIPTION OF FIGURES

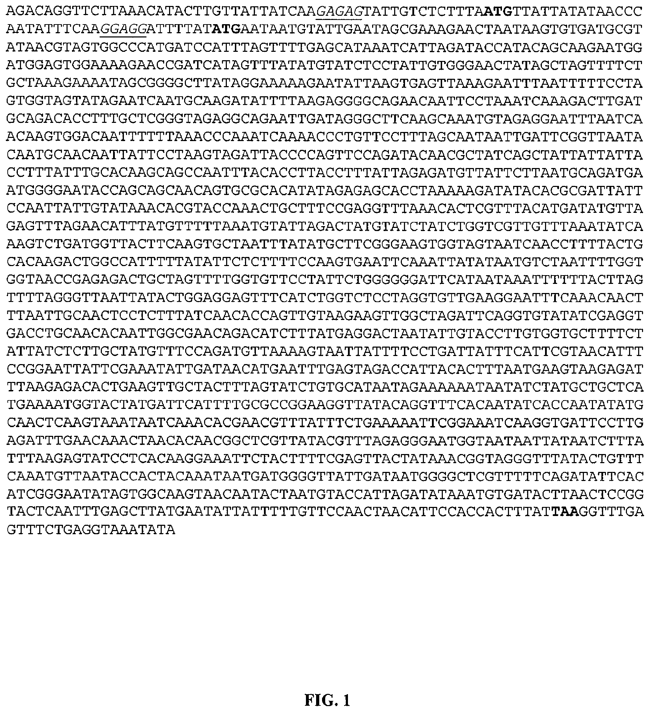

Figure 1 shows the DNA sequence of the *axmi-066* gene and its surrounding DNA region (SEQ ID NO:8). The first ATG (corresponding to the start site of SEQ ID NO:1; translation of which encodes AXMI-066 (SEQ ID NO:2)) is at nucleotide position 52 of the sequence shown in this figure. The second internal methionine (whose translation encodes residues 14 through 637 of SEQ ID NO:2) is at position 91 of this figure. The TAA stop codon begins at position 1963 of the sequence in this figure. The ATG start codons and the TAA stop codon are shown in bold type. Two putative ribosome binding sites are shown in italics and underlined.

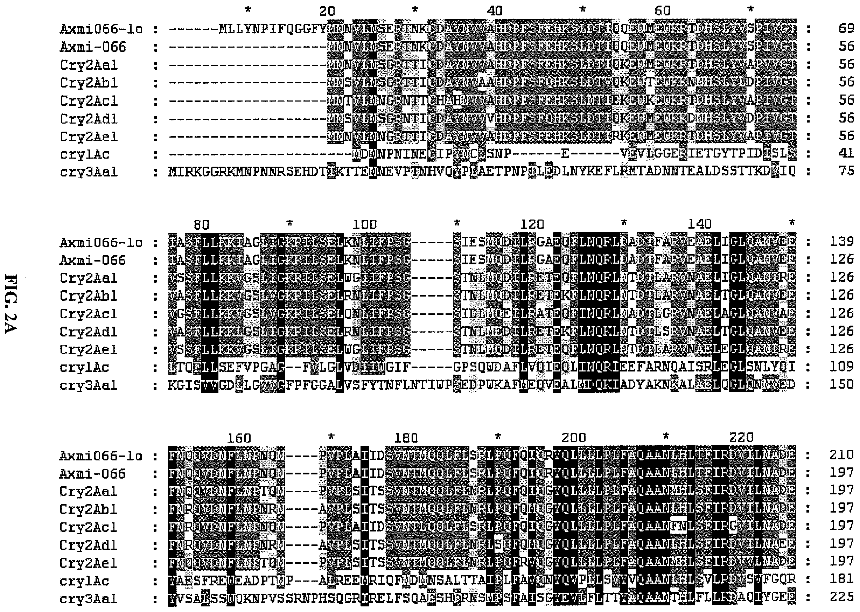

Figures 2A-2D show an alignment of AXMI-066_long (SEQ ID NO:2), AXMI-066 (SEQ ID NO:10), Cry2Aa1 (SEQ ID NO:14), Cry2Ab1 (SEQ ID NO:15), Cry2Ac1 (SEQ ID NO:16), Cry2Ad1 (SEQ ID NO:17), Cry2Ae1 (SEQ ID NO:18), Cry1Ac (SEQ ID NO:19), and Cry3Aa1 (SEQ ID NO:20). The alignment shows the most highly conserved amino acid residues highlighted in black, and highly conserved amino acid residues highlighted in gray.

### DETAILED DESCRIPTION

The present invention is drawn to compositions and methods for regulating pest resistance or tolerance in organisms, particularly plants or plant cells. By "resistance" is intended that the pest (e.g., insect) is killed upon ingestion or other contact with the polypeptides of the invention. By "tolerance" is intended an impairment or reduction in the movement, feeding, reproduction, or other functions of the pest. The methods involve transforming organisms with a nucleotide sequence encoding a pesticidal protein of the invention. In particular, the nucleotide sequences of the invention are useful for preparing plants and microorganisms that possess pesticidal activity. Thus, transformed bacteria, plants, plant cells, plant tissues and seeds are provided. Compositions are pesticidal nucleic acids and proteins of *Bacillus* or other species. The sequences find use in the construction of expression vectors for subsequent transformation into organisms of interest, as probes for the isolation of other homologous (or partially homologous) genes, and for the generation of altered pesticidal proteins by methods known in the art, such as domain swapping or DNA shuffling. The proteins find use in controlling or killing lepidopteran, coleopteran, dipteran, and nematode pest populations and for producing compositions with pesticidal activity.

A plasmid containing the *axmi-066* nucleotide sequence of the invention was deposited in the permanent collection of the Agricultural Research Service Culture Collection, Northern Regional Research Laboratory (NRRL), 1815 North University Street, Peoria, Illinois 61604, United States of America, on May 29, 2007 under Accession No. NRRL B-50045. This deposit will be maintained under the terms of the Budapest Treaty on the International Recognition of the Deposit of Microorganisms for the Purposes of Patent Procedure. Access to these deposits will be available during the pendency of the application to the Commissioner of Patents and Trademarks and persons determined by the Commissioner to be entitled thereto upon request. Upon allowance of any claims in the application, the Applicants will make available to the public, pursuant to 37 C.F.R. § 1.808, sample(s) of the deposit with the NRRL. This deposit was made merely as a convenience for those of skill in the art and is not an admission that a deposit is required under 35 U.S.C. §112.

By "pesticidal toxin" or "pesticidal protein" is intended a toxin that has toxic activity against one or more pests, including, but not limited to, members of the *Lepidoptera, Diptera,* and *Coleoptera* orders, or the *Nematoda* phylum, or a protein that has homology to such a protein. Pesticidal proteins have been isolated from organisms including, for example, *Bacillus sp., Clostridium bifermentans* and *Paenibacillus popilliae.* Pesticidal proteins include amino acid sequences deduced from the full-length nucleotide sequences disclosed herein, and amino acid sequences that are shorter than the full-length sequences, either due to the use of an alternate downstream start site, or due to processing that produces a shorter protein having pesticidal activity. Processing may occur in the organism the protein is expressed in, or in the pest after ingestion of the protein.

Pesticidal proteins encompass delta-endotoxins. Delta-endotoxins include proteins identified as *cry1* through *cry43, cyt1* and *cyt2,* and Cyt-like toxin. There are currently over 250 known species of delta-endotoxins with a wide range of specificities and toxicities. For an expansive list see Crickmore et al. (1998), Microbiol. Mol. Biol. Rev. 62:807-813, and for regular updates see Crickmore et al. (2003) "Bacillus thuringiensis toxin nomenclature," at www.biols.susx.ac.uk/Home/Neil_Crickmore/Bt/index.

Thus, provided herein are novel isolated nucleotide sequences that confer pesticidal activity. These isolated nucleotide sequences encode polypeptides with homology to known delta-endotoxins or binary toxins. Also provided are the amino acid sequences of the pesticidal proteins. The protein resulting from translation of this gene allows cells to control or kill pests that ingest it.

### Isolated Nucleic Acid Molecules, and Variants and Fragments Thereof

One aspect of the invention pertains to isolated or recombinant nucleic acid molecules comprising nucleotide sequences encoding pesticidal proteins and polypeptides or biologically active portions thereof, as well as nucleic acid molecules sufficient for use as hybridization probes to identify nucleic acid molecules encoding proteins with regions of sequence homology. As used herein, the term "nucleic acid molecule" is intended to include DNA molecules (e.g., recombinant DNA, cDNA or genomic DNA) and RNA molecules (e.g., mRNA) and analogs of the DNA or RNA generated using nucleotide analogs. The nucleic acid molecule can be single-stranded or double-stranded, but preferably is double-stranded DNA.

An "isolated" or "purified" nucleic acid molecule or protein, or biologically active portion thereof, is substantially free of other cellular material, or culture medium when produced by recombinant techniques, or substantially free of chemical precursors or other chemicals when chemically synthesized. Preferably, an "isolated" nucleic acid is free of sequences (preferably protein encoding sequences) that naturally flank the nucleic acid (i.e., sequences located at the 5' and 3' ends of the nucleic acid) in the genomic DNA of the organism from which the nucleic acid is derived. For purposes of the invention, "isolated" when used to refer to nucleic acid molecules excludes isolated chromosomes. For example, in various embodiments, the isolated nucleic acid molecule encoding a pesticidal protein can contain less than about 5 kb, 4 kb, 3 kb, 2 kb, 1 kb, 0.5 kb, or 0.1 kb of nucleotide sequences that naturally flank the nucleic acid molecule in genomic DNA of the cell from which the nucleic acid is derived. A pesticidal protein that is substantially free of cellular material includes preparations of protein having less than about 30%, 20%, 10%, or 5% (by dry weight) of non-pesticidal protein (also referred to herein as a "contaminating protein").

Nucleotide sequences encoding the proteins of the present invention include the sequence set forth in SEQ ID NO:1, 3, 4, 6, 9, or 11, or the nucleotide sequence deposited in a bacterial host as Accession No. B-50045, and variants, fragments, and complements thereof. By "complement" is intended a nucleotide sequence that is sufficiently complementary to a given nucleotide sequence such that it can hybridize to the given nucleotide sequence to thereby form a stable duplex. The corresponding amino acid sequence for the pesticidal protein encoded by this nucleotide sequence are set forth in SEQ ID NO:2 or 5.

Nucleic acid molecules that are fragments of these nucleotide sequences encoding pesticidal proteins are also encompassed by the present invention. By "fragment" is intended a portion of the nucleotide sequence encoding a pesticidal protein. A fragment of a nucleotide sequence may encode a biologically active portion of a pesticidal protein, or it may be a fragment that can be used as a hybridization probe or PCR primer using methods disclosed below. Nucleic acid molecules that are fragments of a nucleotide sequence encoding a pesticidal protein comprise at least about 50, 100, 200, 300, 400, 500, 600, 700, 800, 900, 1000, 1100, 1200, 1300, 1350, 1400 contiguous nucleotides, or up to the number of nucleotides present in a full-length nucleotide sequence encoding a pesticidal protein disclosed herein, depending upon the intended use. By "contiguous" nucleotides is intended nucleotide residues that are immediately adjacent to one another. Fragments of the nucleotide sequences of the present invention will encode protein fragments that retain the biological activity of the pesticidal protein and, hence, retain pesticidal activity. By "retains activity" is intended that the fragment will have at least about 30%, at least about 50%, at least about 70%, 80%, 90%, 95% or higher of the pesticidal activity of the pesticidal protein. In one embodiment, the pesticidal activity is coleoptericidal activity. In another embodiment, the pesticidal activity is lepidoptericidal activity. In another embodiment, the pesticidal activity is nematocidal activity. In another embodiment, the pesticidal activity is diptericidal activity. Methods for measuring pesticidal activity are well known in the art. See, for example, Czapla and Lang (1990) J. Econ. Entomol. 83:2480-2485; Andrews et al. (1988) Biochem. J. 252:199-206; Marrone et al. (1985) J. of Economic Entomology 78:290-293; and U.S. Patent No. 5,743,477, all of which are herein incorporated by reference in their entirety.

A fragment of a nucleotide sequence encoding a pesticidal protein that encodes a biologically active portion of a protein of the invention will encode at least about 15, 25, 30, 50, 75, 100, 125, 150, 175, 200, 250, 300, 350, 400, 450 contiguous amino acids, or up to the total number of amino acids present in a full-length pesticidal protein of the invention.

Preferred pesticidal proteins of the present invention are encoded by a nucleotide sequence sufficiently identical to the nucleotide sequence of SEQ ID NO:1, 3, 4, 6, 9, or 11. By "sufficiently identical" is intended an amino acid or nucleotide sequence that has at least about 60% or 65% sequence identity, about 70% or 75% sequence identity, about 80% or 85% sequence identity, about 90%, 91%, 92%, 93%, 94%, 95%, 96%, 97%, 98%, 99% or greater sequence identity compared to a reference sequence using one of the alignment programs described herein using standard parameters. One of skill in the art will recognize that these values can be appropriately adjusted to determine corresponding identity of proteins encoded by two nucleotide sequences by taking into account codon degeneracy, amino acid similarity, reading frame positioning, and the like.

To determine the percent identity of two amino acid sequences or of two nucleic acids, the sequences are aligned for optimal comparison purposes. The percent identity between the two sequences is a function of the number of identical positions shared by the sequences (i.e., percent identity = number of identical positions/total number of positions (e.g., overlapping positions) x 100). In one embodiment, the two sequences are the same length. In another embodiment, the percent identity is calculated across the entirety of the reference sequence (i.e., the sequence disclosed herein as any of SEQ ID NO:1-13). The percent identity between two sequences can be determined using techniques similar to those described below, with or without allowing gaps. In calculating percent identity, typically exact matches are counted.

The determination of percent identity between two sequences can be accomplished using a mathematical algorithm. A nonlimiting example of a mathematical algorithm utilized for the comparison of two sequences is the algorithm of Karlin and Altschul (1990) Proc. Natl. Acad. Sci. USA 87:2264, modified as in Karlin and Altschul (1993) Proc. Natl. Acad. Sci. USA 90:5873-5877. Such an algorithm is incorporated into the BLASTN and BLASTX programs of Altschul et al. (1990) J. Mol. Biol. 215:403. BLAST nucleotide searches can be performed with the BLASTN program, score = 100, wordlength = 12, to obtain nucleotide sequences homologous to pesticidal-like nucleic acid molecules of the invention. BLAST protein searches can be performed with the BLASTX program, score = 50, wordlength = 3, to obtain amino acid sequences homologous to pesticidal protein molecules of the invention. To obtain gapped alignments for comparison purposes, Gapped BLAST (in BLAST 2.0) can be utilized as described in Altschul et al. (1997) Nucleic Acids Res. 25:3389. Alternatively, PSI-Blast can be used to perform an iterated search that detects distant relationships between molecules. See Altschul *et al.* (1997) *supra.* When utilizing BLAST, Gapped BLAST, and PSI-Blast programs, the default parameters of the respective programs (e.g., BLASTX and BLASTN) can be used. Alignment may also be performed manually by inspection.

Another non-limiting example of a mathematical algorithm utilized for the comparison of sequences is the ClustalW algorithm (Higgins et al. (1994) Nucleic Acids Res. 22:4673-4680). ClustalW compares sequences and aligns the entirety of the amino acid or DNA sequence, and thus can provide data about the sequence conservation of the entire amino acid sequence. The ClustalW algorithm is used in several commercially available DNA/amino acid analysis software packages, such as the ALIGNX module of the Vector NTI Program Suite (Invitrogen Corporation, Carlsbad, CA). After alignment of amino acid sequences with ClustalW, the percent amino acid identity can be assessed. A non-limiting example of a software program useful for analysis of ClustalW alignments is GENEDOC™. GENEDOC™ (Karl Nicholas) allows assessment of amino acid (or DNA) similarity and identity between multiple proteins. Another non-limiting example of a mathematical algorithm utilized for the comparison of sequences is the algorithm of Myers and Miller (1988) CABIOS 4:11-17. Such an algorithm is incorporated into the ALIGN program (version 2.0), which is part of the GCG Wisconsin Genetics Software Package, Version 10 (available from Accelrys, Inc., 9685 Scranton Rd., San Diego, CA, USA). When utilizing the ALIGN program for comparing amino acid sequences, a PAM120 weight residue table, a gap length penalty of 12, and a gap penalty of 4 can be used.

Unless otherwise stated, GAP Version 10, which uses the algorithm of Needleman and Wunsch (1970) J. Mol. Biol. 48(3):443-453, will be used to determine sequence identity or similarity using the following parameters: % identity and % similarity for a nucleotide sequence using GAP Weight of 50 and Length Weight of 3, and the nwsgapdna.cmp scoring matrix; % identity or % similarity for an amino acid sequence using GAP weight of 8 and length weight of 2, and the BLOSUM62 scoring program. Equivalent programs may also be used. By "equivalent program" is intended any sequence comparison program that, for any two sequences in question, generates an alignment having identical nucleotide residue matches and an identical percent sequence identity when compared to the corresponding alignment generated by GAP Version 10.

The invention also encompasses variant nucleic acid molecules. "Variants" of the pesticidal protein encoding nucleotide sequences include those sequences that encode the pesticidal proteins disclosed herein but that differ conservatively because of the degeneracy of the genetic code as well as those that are sufficiently identical as discussed above. Naturally occurring allelic variants can be identified with the use of well-known molecular biology techniques, such as polymerase chain reaction (PCR) and hybridization techniques as outlined below. Variant nucleotide sequences also include synthetically derived nucleotide sequences that have been generated, for example, by using site-directed mutagenesis but which still encode the pesticidal proteins disclosed in the present invention as discussed below. Variant proteins encompassed by the present invention are biologically active, that is they continue to possess the desired biological activity of the native protein, that is, pesticidal activity. By "retains activity" is intended that the variant will have at least about 30%, at least about 50%, at least about 70%, or at least about 80% of the pesticidal activity of the native protein. Methods for measuring pesticidal activity are well known in the art. See, for example, Czapla and Lang (1990) J. Econ. Entomol. 83: 2480-2485; Andrews et al. (1988) Biochem. J. 252:199-206; Marrone et al. (1985) J. of Economic Entomology 78:290-293; and U.S. Patent No. 5,743,477, all of which are herein incorporated by reference in their entirety.

In one embodiment, the variants encompass insertion of one or more amino acids into SEQ ID NO:2, 5, or 10. In another embodiment, the variants encompass insertion of one or more amino acids in apical loop 2 of SEQ ID NO:10. In another embodiment, the variants encompass insertion of one or more amino acids between residues 379 and 380 of SEQ ID NO:10. In another embodiment, the variants encompass insertion of at least a glycine residue between residues 379 and 380 of SEQ ID NO:10. In another embodiment, the variants encompass insertion of a glycine residue and one additional residue between residues 379 and 380 of SEQ ID NO:10. In another embodiment, the variants encompass insertion of two glycine residues, of a glycine and a threonine, a glycine and a serine, a glycine and a leucine, an arginine and a glycine, a glycine and an asparagine, a glycine and a lysine, a histidine and a glycine, a phenylalanine and a glycine, a leucine and a glycine, or an asparagine and a glycine residue between residues 379 and 380 of SEQ ID NO:10.

In yet another embodiment, the variant is selected from the group consisting of P83T, L250I, G319K, G319F, I322S, I322V, I322Q, I322A, L323F, Y376N, Y376I, Y376R, Y376S, Y376V, Y376A, R377E, R377Q, R377L, G378S, G378A, G378W, D379V, D379E, L380M, L380P, L380Y, Q381L, L401I, M406H, M406V, M406K, M406E, M406T, M406S, M406A, M406V, M406N, F407W, and F407R relative to SEQ ID NO:10.

The skilled artisan will further appreciate that changes can be introduced by mutation of the nucleotide sequences of the invention thereby leading to changes in the amino acid sequence of the encoded pesticidal proteins, without altering the biological activity of the proteins. Thus, variant isolated nucleic acid molecules can be created by introducing one or more nucleotide substitutions, additions, or deletions into the corresponding nucleotide sequence disclosed herein, such that one or more amino acid substitutions, additions or deletions are introduced into the encoded protein. Mutations can be introduced by standard techniques, such as site-directed mutagenesis and PCR-mediated mutagenesis. Such variant nucleotide sequences are also encompassed by the present invention.

For example, conservative amino acid substitutions may be made at one or more, predicted, nonessential amino acid residues. A "nonessential" amino acid residue is a residue that can be altered from the wild-type sequence of a pesticidal protein without altering the biological activity, whereas an "essential" amino acid residue is required for biological activity. A "conservative amino acid substitution" is one in which the amino acid residue is replaced with an amino acid residue having a similar side chain. Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine).

Delta-endotoxins generally have five conserved sequence domains, and three conserved structural domains (see, for example, de Maagd et al. (2001) Trends Genetics 17:193-199). The first conserved structural domain consists of seven alpha helices and is involved in membrane insertion and pore formation. Domain II consists of three beta-sheets arranged in a Greek key configuration, and domain III consists of two antiparallel beta-sheets in "jelly-roll" formation (de Maagd *et al.,* 2001, supra). Domains II and III are involved in receptor recognition and binding, and are therefore considered determinants of toxin specificity.

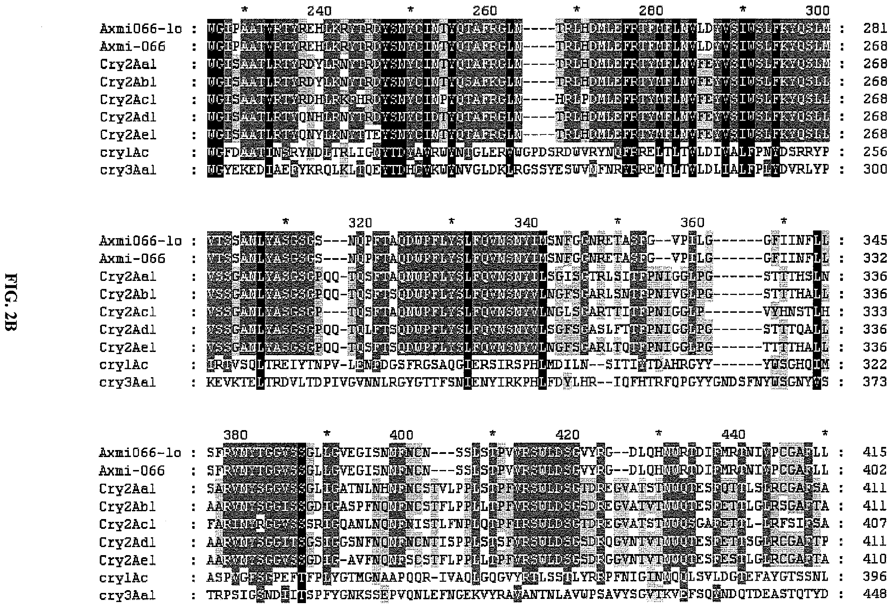

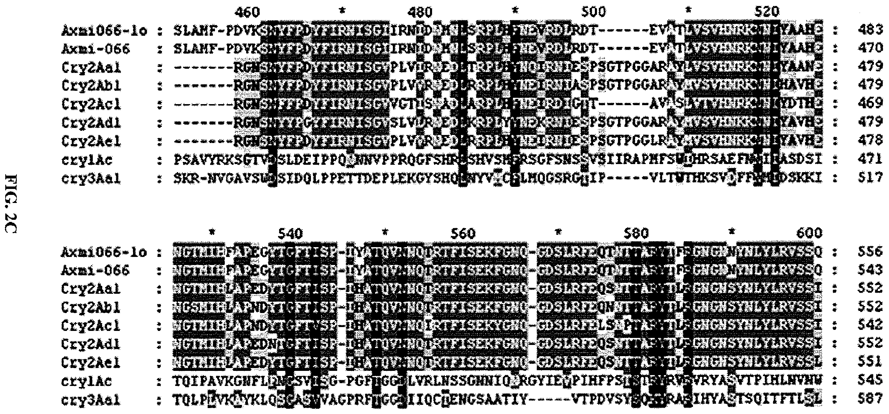

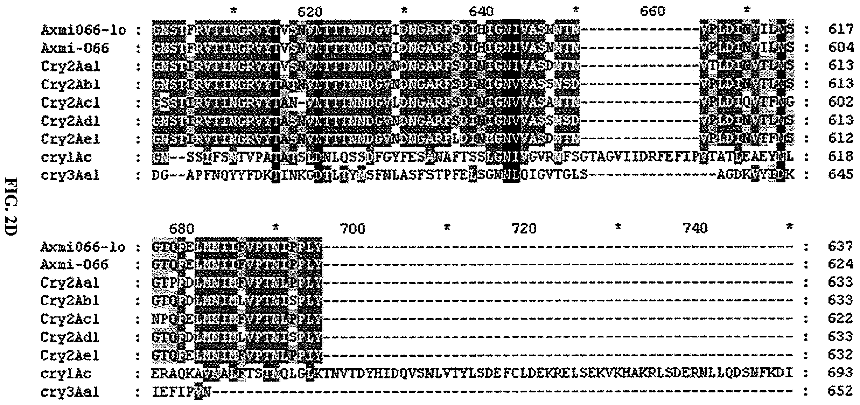

AXMI-066 shows homology Cry2A family of proteins. The 3D structure of Cry2Aa has been determined (see, Morse et al. (2001) Structure 9:409-417), and domain swapping experiments between Cry2A and Cry2B have lead to the identification of specificity regions (see, for example, Liang and Dean (1994) Molecular Microbiology, 13 (4):569-575; Widner and Whiteley (1989) J.Bacteriology. 171(2)965-974; and Widner and Whiteley (1990) J.Bacteriology., 172(6):2826-2832, each of which is herein incorporated by reference in its entirety). Apical loops in Cry toxins have been implicated in receptor recognition, and Cry2Aa contains 2 apical loops. Loop 1 is found from about position 316 to about position 335 of SEQ ID NO:14. Loop 2 is found from about position 370 to about position 394 of SEQ ID NO:14. The corresponding residues in AXMI-066 can be found in the alignment of Figure 2.

Amino acid substitutions may be made in nonconserved regions that retain function. In general, such substitutions would not be made for conserved amino acid residues, or for amino acid residues residing within a conserved motif, where such residues are essential for protein activity. Examples of residues that are conserved and that may be essential for protein activity include, for example, residues that are identical between all proteins contained in an alignment of similar or related toxins to the sequences of the invention (e.g., residues that are identical between all proteins contained in the alignment in Figure 7). Examples of residues that are conserved but that may allow conservative amino acid substitutions and still retain activity include, for example, residues that have only conservative substitutions between all proteins contained in an alignment of similar or related toxins to the sequences of the invention (e.g., residues that have only conservative substitutions between all proteins contained in the alignment in Figure 7). However, one of skill in the art would understand that functional variants may have minor conserved or nonconserved alterations in the conserved residues.

Alternatively, variant nucleotide sequences can be made by introducing mutations randomly along all or part of the coding sequence, such as by saturation mutagenesis, and the resultant mutants can be screened for ability to confer pesticidal activity to identify mutants that retain activity. Following mutagenesis, the encoded protein can be expressed recombinantly, and the activity of the protein can be determined using standard assay techniques.

Using methods such as PCR, hybridization, and the like corresponding pesticidal sequences can be identified, such sequences having substantial identity to the sequences of the invention. See, for example, Sambrook and Russell (2001) Molecular Cloning: A Laboratory Manual. (Cold Spring Harbor Laboratory Press, Cold Spring Harbor, NY) and Innis, et al. (1990) PCR Protocols: A Guide to Methods and Applications (Academic Press, NY).

In a hybridization method, all or part of the pesticidal nucleotide sequence can be used to screen cDNA or genomic libraries. Methods for construction of such cDNA and genomic libraries are generally known in the art and are disclosed in Sambrook and Russell, 2001, *supra.* The so-called hybridization probes may be genomic DNA fragments, cDNA fragments, RNA fragments, or other oligonucleotides, and may be labeled with a detectable group such as ³²P, or any other detectable marker, such as other radioisotopes, a fluorescent compound, an enzyme, or an enzyme co-factor. Probes for hybridization can be made by labeling synthetic oligonucleotides based on the known pesticidal protein-encoding nucleotide sequence disclosed herein. Degenerate primers designed on the basis of conserved nucleotides or amino acid residues in the nucleotide sequence or encoded amino acid sequence can additionally be used. The probe typically comprises a region of nucleotide sequence that hybridizes under stringent conditions to at least about 12, at least about 25, at least about 50, 75, 100, 125, 150, 175, or 200 consecutive nucleotides of nucleotide sequence encoding a pesticidal protein of the invention or a fragment or variant thereof. Methods for the preparation of probes for hybridization are generally known in the art and are disclosed in Sambrook and Russell, 2001, *supra* herein incorporated by reference.

For example, an entire pesticidal protein sequence disclosed herein, or one or more portions thereof, may be used as a probe capable of specifically hybridizing to corresponding pesticidal protein-like sequences and messenger RNAs. To achieve specific hybridization under a variety of conditions, such probes include sequences that are unique and are preferably at least about 10 nucleotides in length, or at least about 20 nucleotides in length. Such probes may be used to amplify corresponding pesticidal sequences from a chosen organism by PCR. This technique may be used to isolate additional coding sequences from a desired organism or as a diagnostic assay to determine the presence of coding sequences in an organism. Hybridization techniques include hybridization screening of plated DNA libraries (either plaques or colonies; see, for example, Sambrook et al. (1989) Molecular Cloning: A Laboratory Manual (2d ed., Cold Spring Harbor Laboratory Press, Cold Spring Harbor, New York).

Hybridization of such sequences may be carried out under stringent conditions. By "stringent conditions" or "stringent hybridization conditions" is intended conditions under which a probe will hybridize to its target sequence to a detectably greater degree than to other sequences (e.g., at least 2-fold over background). Stringent conditions are sequence-dependent and will be different in different circumstances. By controlling the stringency of the hybridization and/or washing conditions, target sequences that are 100% complementary to the probe can be identified (homologous probing). Alternatively, stringency conditions can be adjusted to allow some mismatching in sequences so that lower degrees of similarity are detected (heterologous probing). Generally, a probe is less than about 1000 nucleotides in length, preferably less than 500 nucleotides in length.

Typically, stringent conditions will be those in which the salt concentration is less than about 1.5 M Na ion, typically about 0.01 to 1.0 M Na ion concentration (or other salts) at pH 7.0 to 8.3 and the temperature is at least about 30°C for short probes (e.g., 10 to 50 nucleotides) and at least about 60°C for long probes (e.g., greater than 50 nucleotides). Stringent conditions may also be achieved with the addition of destabilizing agents such as formamide. Exemplary low stringency conditions include hybridization with a buffer solution of 30 to 35% formamide, 1 M NaCl, 1% SDS (sodium dodecyl sulphate) at 37°C, and a wash in 1X to 2X SSC (20X SSC = 3.0 M NaCl/0.3 M trisodium citrate) at 50 to 55°C. Exemplary moderate stringency conditions include hybridization in 40 to 45% formamide, 1.0 M NaCl, 1% SDS at 37°C, and a wash in 0.5X to 1X SSC at 55 to 60°C. Exemplary high stringency conditions include hybridization in 50% formamide, 1 M NaCl, 1% SDS at 37°C, and a wash in 0.1X SSC at 60 to 65°C. Optionally, wash buffers may comprise about 0.1% to about 1% SDS. Duration of hybridization is generally less than about 24 hours, usually about 4 to about 12 hours.

Specificity is typically the function of post-hybridization washes, the critical factors being the ionic strength and temperature of the final wash solution. For DNA-DNA hybrids, the Tₘ can be approximated from the equation of Meinkoth and Wahl (1984) Anal. Biochem. 138:267-284: Tₘ = 81.5°C + 16.6 (log M) + 0.41 (%GC) - 0.61 (% form) - 500/L; where M is the molarity of monovalent cations, %GC is the percentage of guanosine and cytosine nucleotides in the DNA, % form is the percentage of formamide in the hybridization solution, and L is the length of the hybrid in base pairs. The Tₘ is the temperature (under defined ionic strength and pH) at which 50% of a complementary target sequence hybridizes to a perfectly matched probe. Tₘ is reduced by about 1°C for each 1% of mismatching; thus, Tₘ, hybridization, and/or wash conditions can be adjusted to hybridize to sequences of the desired identity. For example, if sequences with ≥90% identity are sought, the Tₘ can be decreased 10°C. Generally, stringent conditions are selected to be about 5°C lower than the thermal melting point (Tₘ) for the specific sequence and its complement at a defined ionic strength and pH. However, severely stringent conditions can utilize a hybridization and/or wash at 1, 2, 3, or 4°C lower than the thermal melting point (Tₘ); moderately stringent conditions can utilize a hybridization and/or wash at 6, 7, 8, 9, or 10°C lower than the thermal melting point (Tₘ); low stringency conditions can utilize a hybridization and/or wash at 11, 12, 13, 14, 15, or 20°C lower than the thermal melting point (Tₘ). Using the equation, hybridization and wash compositions, and desired Tₘ, those of ordinary skill will understand that variations in the stringency of hybridization and/or wash solutions are inherently described. If the desired degree of mismatching results in a Tₘ of less than 45°C (aqueous solution) or 32°C (formamide solution), it is preferred to increase the SSC concentration so that a higher temperature can be used. An extensive guide to the hybridization of nucleic acids is found in Tijssen (1993) Laboratory Techniques in Biochemistry and Molecular Biology-Hybridization with Nucleic Acid Probes, Part I, Chapter 2 (Elsevier, New York); and Ausubel et al., eds. (1995) Current Protocols in Molecular Biology, Chapter 2 (Greene Publishing and Wiley-Interscience, New York). See Sambrook et al. (1989) Molecular Cloning: A Laboratory Manual (2d ed., Cold Spring Harbor Laboratory Press, Cold Spring Harbor, New York).

### Isolated Proteins and Variants and Fragments Thereof

Pesticidal proteins are also encompassed within the present invention. By "pesticidal protein" is intended a protein having the amino acid sequence set forth in SEQ ID NO:2 or 5. Fragments, biologically active portions, and variants thereof are also provided, and may be used to practice the methods of the present invention.

"Fragments" or "biologically active portions" include polypeptide fragments comprising amino acid sequences sufficiently identical to the amino acid sequence set forth in SEQ ID NO:2 or 5, and that exhibit pesticidal activity. A biologically active portion of a pesticidal protein can be a polypeptide that is, for example, 10, 25, 50, 100, 150, 200, 250 or more amino acids in length. Such biologically active portions can be prepared by recombinant techniques and evaluated for pesticidal activity. Methods for measuring pesticidal activity are well known in the art. See, for example, Czapla and Lang (1990) J. Econ. Entomol. 83:2480-2485; Andrews et al. (1988) Biochem. J. 252:199-206; Marrone et al. (1985) J. of Economic Entomology 78:290-293; and U.S. Patent No. 5,743,477, all of which are herein incorporated by reference in their entirety. As used here, a fragment comprises at least 8 contiguous amino acids of SEQ ID NO:2 or 5. The invention encompasses other fragments, however, such as any fragment in the protein greater than about 10, 20, 30, 50, 100, 150, 200, 250, or 300 amino acids.

By "variants" is intended proteins or polypeptides having an amino acid sequence that is at least about 60%, 65%, about 70%, 75%, about 80%, 85%, about 90%, 91%, 92%, 93%, 94%, 95%, 96%, 97%, 98% or 99% identical to the amino acid sequence of SEQ ID NO:2 or 5. Variants also include polypeptides encoded by a nucleic acid molecule that hybridizes to the nucleic acid molecule of SEQ ID NO:1, 3, 4, 6, 9, or 11, or a complement thereof, under stringent conditions. Variants include polypeptides that differ in amino acid sequence due to mutagenesis. Variant proteins encompassed by the present invention are biologically active, that is they continue to possess the desired biological activity of the native protein, that is, retaining pesticidal activity. Methods for measuring pesticidal activity are well known in the art. See, for example, Czapla and Lang (1990) J. Econ. Entomol. 83:2480-2485; Andrews et al. (1988) Biochem. J. 252:199-206; Marrone et al. (1985) J. of Economic Entomology 78:290-293; and U.S. Patent No. 5,743,477, all of which are herein incorporated by reference in their entirety.

Bacterial genes, such as the *axmi* genes of this invention, quite often possess multiple methionine initiation codons in proximity to the start of the open reading frame. Often, translation initiation at one or more of these start codons will lead to generation of a functional protein. These start codons can include ATG codons. However, bacteria such as *Bacillus sp.* also recognize the codon GTG as a start codon, and proteins that initiate translation at GTG codons contain a methionine at the first amino acid. Furthermore, it is not often determined *a priori* which of these codons are used naturally in the bacterium. Thus, it is understood that use of one of the alternate methionine codons may also lead to generation of pesticidal proteins. These pesticidal proteins are encompassed in the present invention and may be used in the methods of the present invention.

Antibodies to the polypeptides of the present invention, or to variants or fragments thereof, are also encompassed. Methods for producing antibodies are well known in the art (see, for example, Harlow and Lane (1988) Antibodies: A Laboratory Manual, Cold Spring Harbor Laboratory, Cold Spring Harbor, NY; U.S. Patent No. 4,196,265).

### Altered or Improved Variants

It is recognized that DNA sequences of a pesticidal protein may be altered by various methods, and that these alterations may result in DNA sequences encoding proteins with amino acid sequences different than that encoded by a pesticidal protein of the present invention. This protein may be altered in various ways including amino acid substitutions, deletions, truncations, and insertions of one or more amino acids of SEQ ID NO:2 or 5, including up to about 2, about 3, about 4, about 5, about 6, about 7, about 8, about 9, about 10, about 15, about 20, about 25, about 30, about 35, about 40, about 45, about 50, about 55, about 60, about 65, about 70, about 75, about 80, about 85, about 90, about 100, about 105, about 110, about 115, about 120, about 125, about 130, about 135, about 140, about 145, about 150, about 155, or more amino acid substitutions, deletions or insertions. Methods for such manipulations are generally known in the art. For example, amino acid sequence variants of a pesticidal protein can be prepared by mutations in the DNA. This may also be accomplished by one of several forms of mutagenesis and/or in directed evolution. In some aspects, the changes encoded in the amino acid sequence will not substantially affect the function of the protein. Such variants will possess the desired pesticidal activity. However, it is understood that the ability of a pesticidal protein to confer pesticidal activity may be improved by the use of such techniques upon the compositions of this invention. For example, one may express a pesticidal protein in host cells that exhibit high rates of base misincorporation during DNA replication, such as XL-1 Red (Stratagene, La Jolla, CA). After propagation in such strains, one can isolate the DNA (for example by preparing plasmid DNA, or by amplifying by PCR and cloning the resulting PCR fragment into a vector), culture the pesticidal protein mutations in a non-mutagenic strain, and identify mutated genes with pesticidal activity, for example by performing an assay to test for pesticidal activity. Generally, the protein is mixed and used in feeding assays. See, for example Marrone et al. (1985) J. of Economic Entomology 78:290-293. Such assays can include contacting plants with one or more pests and determining the plant's ability to survive and/or cause the death of the pests. Examples of mutations that result in increased toxicity are found in Schnepf et al. (1998) Microbiol. Mol. Biol. Rev. 62:775-806.

Alternatively, alterations may be made to the protein sequence of many proteins at the amino or carboxy terminus without substantially affecting activity. This can include insertions, deletions, or alterations introduced by modern molecular methods, such as PCR, including PCR amplifications that alter or extend the protein coding sequence by virtue of inclusion of amino acid encoding sequences in the oligonucleotides utilized in the PCR amplification. Alternatively, the protein sequences added can include entire protein-coding sequences, such as those used commonly in the art to generate protein fusions. Such fusion proteins are often used to (1) increase expression of a protein of interest (2) introduce a binding domain, enzymatic activity, or epitope to facilitate either protein purification, protein detection, or other experimental uses known in the art (3) target secretion or translation of a protein to a subcellular organelle, such as the periplasmic space of Gram-negative bacteria, or the endoplasmic reticulum of eukaryotic cells, the latter of which often results in glycosylation of the protein.

Variant nucleotide and amino acid sequences of the present invention also encompass sequences derived from mutagenic and recombinogenic procedures such as DNA shuffling. With such a procedure, one or more different pesticidal protein coding regions can be used to create a new pesticidal protein possessing the desired properties. In this manner, libraries of recombinant polynucleotides are generated from a population of related sequence polynucleotides comprising sequence regions that have substantial sequence identity and can be homologously recombined *in vitro* or *in vivo.* For example, using this approach, sequence motifs encoding a domain of interest may be shuffled between a pesticidal gene of the invention and other known pesticidal genes to obtain a new gene coding for a protein with an improved property of interest, such as an increased insecticidal activity. Strategies for such DNA shuffling are known in the art. See, for example, Stemmer (1994) Proc. Natl. Acad. Sci. USA 91:10747-10751; Stemmer (1994) Nature 370:389-391; Crameri et al. (1997) Nature Biotech. 15:436-438; Moore et al. (1997) J. Mol. Biol. 272:336-347; Zhang et al. (1997) Proc. Natl. Acad. Sci. USA 94:4504-4509; Crameri et al. (1998) Nature 391:288-291; and U.S. Patent Nos. 5,605,793 and 5,837,458.

Domain swapping or shuffling is another mechanism for generating altered pesticidal proteins. Domains may be swapped between pesticidal proteins, resulting in hybrid or chimeric toxins with improved pesticidal activity or target spectrum. Methods for generating recombinant proteins and testing them for pesticidal activity are well known in the art (see, for example, Naimov et al. (2001) Appl. Environ. Microbiol. 67:5328-5330; de Maagd et al. (1996) Appl. Environ. Microbiol. 62:1537-1543; Ge et al. (1991) J. Biol. Chem. 266:17954-17958; Schnepf et al. (1990) J. Biol. Chem. 265:20923-20930; Rang et al. 91999) Appl. Environ. Microbiol. 65:2918-2925).

### Vectors

A pesticidal sequence of the invention may be provided in an expression cassette for expression in a plant of interest. By "plant expression cassette" is intended a DNA construct that is capable of resulting in the expression of a protein from an open reading frame in a plant cell. Typically these contain a promoter and a coding sequence. Often, such constructs will also contain a 3' untranslated region. Such constructs may contain a "signal sequence" or "leader sequence" to facilitate co-translational or post-translational transport of the peptide to certain intracellular structures such as the chloroplast (or other plastid), endoplasmic reticulum, or Golgi apparatus.

By "signal sequence" is intended a sequence that is known or suspected to result in cotranslational or post-translational peptide transport across the cell membrane. In eukaryotes, this typically involves secretion into the Golgi apparatus, with some resulting glycosylation. Insecticidal toxins of bacteria are often synthesized as protoxins, which are protolytically activated in the gut of the target pest (Chang (1987) Methods Enzymol. 153:507-516). In some embodiments of the present invention, the signal sequence is located in the native sequence, or may be derived from a sequence of the invention. By "leader sequence" is intended any sequence that when translated, results in an amino acid sequence sufficient to trigger co-translational transport of the peptide chain to a subcellular organelle. Thus, this includes leader sequences targeting transport and/or glycosylation by passage into the endoplasmic reticulum, passage to vacuoles, plastids including chloroplasts, mitochondria, and the like.

By "plant transformation vector" is intended a DNA molecule that is necessary for efficient transformation of a plant cell. Such a molecule may consist of one or more plant expression cassettes, and may be organized into more than one "vector" DNA molecule. For example, binary vectors are plant transformation vectors that utilize two non-contiguous DNA vectors to encode all requisite cis- and trans-acting functions for transformation of plant cells (Hellens and Mullineaux (2000) Trends in Plant Science 5:446-451). "Vector" refers to a nucleic acid construct designed for transfer between different host cells. "Expression vector" refers to a vector that has the ability to incorporate, integrate and express heterologous DNA sequences or fragments in a foreign cell. The cassette will include 5' and 3' regulatory sequences operably linked to a sequence of the invention. By "operably linked" is intended a functional linkage between a promoter and a second sequence, wherein the promoter sequence initiates and mediates transcription of the DNA sequence corresponding to the second sequence. Generally, operably linked means that the nucleic acid sequences being linked are contiguous and, where necessary to join two protein coding regions, contiguous and in the same reading frame. The cassette may additionally contain at least one additional gene to be cotransformed into the organism. Alternatively, the additional gene(s) can be provided on multiple expression cassettes.

"Promoter" refers to a nucleic acid sequence that functions to direct transcription of a downstream coding sequence. The promoter together with other transcriptional and translational regulatory nucleic acid sequences (also termed "control sequences") are necessary for the expression of a DNA sequence of interest.

Such an expression cassette is provided with a plurality of restriction sites for insertion of the pesticidal sequence to be under the transcriptional regulation of the regulatory regions.

The expression cassette will include in the 5'-3' direction of transcription, a transcriptional and translational initiation region (i.e., a promoter), a DNA sequence of the invention, and a translational and transcriptional termination region (i.e., termination region) functional in plants. The promoter may be native or analogous, or foreign or heterologous, to the plant host and/or to the DNA sequence of the invention. Additionally, the promoter may be the natural sequence or alternatively a synthetic sequence. Where the promoter is "native" or "homologous" to the plant host, it is intended that the promoter is found in the native plant into which the promoter is introduced. Where the promoter is "foreign" or "heterologous" to the DNA sequence of the invention, it is intended that the promoter is not the native or naturally occurring promoter for the operably linked DNA sequence of the invention.

The termination region may be native with the transcriptional initiation region, may be native with the operably linked DNA sequence of interest, may be native with the plant host, or may be derived from another source (i.e., foreign or heterologous to the promoter, the DNA sequence of interest, the plant host, or any combination thereof). Convenient termination regions are available from the Ti-plasmid of *A. tumefaciens,* such as the octopine synthase and nopaline synthase termination regions. See also Guerineau et al. (1991) Mol. Gen. Genet. 262:141-144; Proudfoot (1991) Cell 64:671-674; Sanfacon et al. (1991) Genes Dev. 5:141-149; Mogen et al. (1990) Plant Cell 2:1261-1272; Munroe et al. (1990) Gene 91:151-158; Ballas et al. (1989) Nucleic Acids Res. 17:7891-7903; and Joshi et al. (1987) Nucleic Acid Res. 15:9627-9639.

Where appropriate, the gene(s) may be optimized for increased expression in the transformed host cell. That is, the genes can be synthesized using host cell-preferred codons for improved expression, or may be synthesized using codons at a host-preferred codon usage frequency. Generally, the GC content of the gene will be increased. See, for example, Campbell and Gowri (1990) Plant Physiol. 92:1-11 for a discussion of host-preferred codon usage. Methods are available in the art for synthesizing plant-preferred genes. See, for example, U.S. Patent Nos. 5,380,831, and 5,436,391, and Murray et al. (1989) Nucleic Acids Res. 17:477-498, herein incorporated by reference.

In one embodiment, the pesticidal protein is targeted to the chloroplast for expression. In this manner, where the pesticidal protein is not directly inserted into the chloroplast, the expression cassette will additionally contain a nucleic acid encoding a transit peptide to direct the pesticidal protein to the chloroplasts. Such transit peptides are known in the art. See, for example, Von Heijne et al. (1991) Plant Mol. Biol. Rep. 9:104-126; Clark et al. (1989) J. Biol. Chem. 264:17544-17550; Della-Cioppa et al. (1987) Plant Physiol. 84:965-968; Romer et al. (1993) Biochem. Biophys. Res. Commun. 196:1414-1421; and Shah et al. (1986) Science 233:478-481.

The pesticidal gene to be targeted to the chloroplast may be optimized for expression in the chloroplast to account for differences in codon usage between the plant nucleus and this organelle. In this manner, the nucleic acids of interest may be synthesized using chloroplast-preferred codons. See, for example, U.S. Patent No. 5,380,831, herein incorporated by reference.

### Plant Transformation

Methods of the invention involve introducing a nucleotide construct into a plant. By "introducing" is intended to present to the plant the nucleotide construct in such a manner that the construct gains access to the interior of a cell of the plant. The methods of the invention do not require that a particular method for introducing a nucleotide construct to a plant is used, only that the nucleotide construct gains access to the interior of at least one cell of the plant. Methods for introducing nucleotide constructs into plants are known in the art including, but not limited to, stable transformation methods, transient transformation methods, and virus-mediated methods.

By "plant" is intended whole plants, plant organs (e.g., leaves, stems, roots, etc.), seeds, plant cells, propagules, embryos and progeny of the same. Plant cells can be differentiated or undifferentiated (e.g. callus, suspension culture cells, protoplasts, leaf cells, root cells, phloem cells, pollen).

"Transgenic plants" or "transformed plants" or "stably transformed" plants or cells or tissues refers to plants that have incorporated or integrated exogenous nucleic acid sequences or DNA fragments into the plant cell. These nucleic acid sequences include those that are exogenous, or not present in the untransformed plant cell, as well as those that may be endogenous, or present in the untransformed plant cell. "Heterologous" generally refers to the nucleic acid sequences that are not endogenous to the cell or part of the native genome in which they are present, and have been added to the cell by infection, transfection, microinjection, electroporation, microprojection, or the like.

Transformation of plant cells can be accomplished by one of several techniques known in the art. The pesticidal gene of the invention may be modified to obtain or enhance expression in plant cells. Typically a construct that expresses such a protein would contain a promoter to drive transcription of the gene, as well as a 3' untranslated region to allow transcription termination and polyadenylation. The organization of such constructs is well known in the art. In some instances, it may be useful to engineer the gene such that the resulting peptide is secreted, or otherwise targeted within the plant cell. For example, the gene can be engineered to contain a signal peptide to facilitate transfer of the peptide to the endoplasmic reticulum. It may also be preferable to engineer the plant expression cassette to contain an intron, such that mRNA processing of the intron is required for expression.

Typically this "plant expression cassette" will be inserted into a "plant transformation vector". This plant transformation vector may be comprised of one or more DNA vectors needed for achieving plant transformation. For example, it is a common practice in the art to utilize plant transformation vectors that are comprised of more than one contiguous DNA segment. These vectors are often referred to in the art as "binary vectors". Binary vectors as well as vectors with helper plasmids are most often used for *Agrobacterium-*mediated transformation, where the size and complexity of DNA segments needed to achieve efficient transformation is quite large, and it is advantageous to separate functions onto separate DNA molecules. Binary vectors typically contain a plasmid vector that contains the cis-acting sequences required for T-DNA transfer (such as left border and right border), a selectable marker that is engineered to be capable of expression in a plant cell, and a "gene of interest" (a gene engineered to be capable of expression in a plant cell for which generation of transgenic plants is desired). Also present on this plasmid vector are sequences required for bacterial replication. The cis-acting sequences are arranged in a fashion to allow efficient transfer into plant cells and expression therein. For example, the selectable marker gene and the pesticidal gene are located between the left and right borders. Often a second plasmid vector contains the trans-acting factors that mediate T-DNA transfer from *Agrobacterium* to plant cells. This plasmid often contains the virulence functions (Vir genes) that allow infection of plant cells by *Agrobacterium,* and transfer of DNA by cleavage at border sequences and vir-mediated DNA transfer, as is understood in the art (Hellens and Mullineaux (2000) Trends in Plant Science 5:446-451). Several types of *Agrobacterium* strains (e.g. LBA4404, GV3101, EHA101, EHA105, etc.) can be used for plant transformation. The second plasmid vector is not necessary for transforming the plants by other methods such as microprojection, microinjection, electroporation, polyethylene glycol, etc.

In general, plant transformation methods involve transferring heterologous DNA into target plant cells (e.g. immature or mature embryos, suspension cultures, undifferentiated callus, protoplasts, etc.), followed by applying a maximum threshold level of appropriate selection (depending on the selectable marker gene) to recover the transformed plant cells from a group of untransformed cell mass. Explants are typically transferred to a fresh supply of the same medium and cultured routinely. Subsequently, the transformed cells are differentiated into shoots after placing on regeneration medium supplemented with a maximum threshold level of selecting agent. The shoots are then transferred to a selective rooting medium for recovering rooted shoot or plantlet. The transgenic plantlet then grows into a mature plant and produces fertile seeds (e.g. Hiei et al. (1994) The Plant Journal 6:271-282; Ishida et al. (1996) Nature Biotechnology 14:745-750). Explants are typically transferred to a fresh supply of the same medium and cultured routinely. A general description of the techniques and methods for generating transgenic plants are found in Ayres and Park (1994) Critical Reviews in Plant Science 13:219-239 and Bommineni and Jauhar (1997) Maydica 42:107-120. Since the transformed material contains many cells; both transformed and non-transformed cells are present in any piece of subjected target callus or tissue or group of cells. The ability to kill non-transformed cells and allow transformed cells to proliferate results in transformed plant cultures. Often, the ability to remove non-transformed cells is a limitation to rapid recovery of transformed plant cells and successful generation of transgenic plants.

Transformation protocols as well as protocols for introducing nucleotide sequences into plants may vary depending on the type of plant or plant cell, i.e., monocot or dicot, targeted for transformation. Generation of transgenic plants may be performed by one of several methods, including, but not limited to, microinjection, electroporation, direct gene transfer, introduction of heterologous DNA by *Agrobacterium* into plant cells (*Agrobacterium-*mediated transformation), bombardment of plant cells with heterologous foreign DNA adhered to particles, ballistic particle acceleration, aerosol beam transformation (U.S. Published Application No. 20010026941; U.S. Patent No. 4,945,050; International Publication No. WO 91/00915; U.S. Published Application No. 2002015066), Lec1 transformation, and various other non-particle direct-mediated methods to transfer DNA.

Methods for transformation of chloroplasts are known in the art. See, for example, Svab et al. (1990) Proc. Natl. Acad. Sci. USA 87:8526-8530; Svab and Maliga (1993) Proc. Natl. Acad. Sci. USA 90:913-917; Svab and Maliga (1993) EMBO J. 12:601-606. The method relies on particle gun delivery of DNA containing a selectable marker and targeting of the DNA to the plastid genome through homologous recombination. Additionally, plastid transformation can be accomplished by transactivation of a silent plastid-borne transgene by tissue-preferred expression of a nuclear-encoded and plastid-directed RNA polymerase. Such a system has been reported in McBride et al. (1994) Proc. Natl. Acad. Sci. USA 91:7301-7305.

Following integration of heterologous foreign DNA into plant cells, one then applies a maximum threshold level of appropriate selection in the medium to kill the untransformed cells and separate and proliferate the putatively transformed cells that survive from this selection treatment by transferring regularly to a fresh medium. By continuous passage and challenge with appropriate selection, one identifies and proliferates the cells that are transformed with the plasmid vector. Molecular and biochemical methods can then be used to confirm the presence of the integrated heterologous gene of interest into the genome of the transgenic plant.

The cells that have been transformed may be grown into plants in accordance with conventional ways. See, for example, McCormick et al. (1986) Plant Cell Reports 5:81-84. These plants may then be grown, and either pollinated with the same transformed strain or different strains, and the resulting hybrid having constitutive expression of the desired phenotypic characteristic identified. Two or more generations may be grown to ensure that expression of the desired phenotypic characteristic is stably maintained and inherited and then seeds harvested to ensure expression of the desired phenotypic characteristic has been achieved. In this manner, the present invention provides transformed seed (also referred to as "transgenic seed") having a nucleotide construct of the invention, for example, an expression cassette of the invention, stably incorporated into their genome.

### Evaluation of Plant Transformation

Following introduction of heterologous foreign DNA into plant cells, the transformation or integration of heterologous gene in the plant genome is confirmed by various methods such as analysis of nucleic acids, proteins and metabolites associated with the integrated gene.

PCR analysis is a rapid method to screen transformed cells, tissue or shoots for the presence of incorporated gene at the earlier stage before transplanting into the soil (Sambrook and Russell (2001) Molecular Cloning: A Laboratory Manual. Cold Spring Harbor Laboratory Press, Cold Spring Harbor, NY). PCR is carried out using oligonucleotide primers specific to the gene of interest or *Agrobacterium* vector background, etc.

Plant transformation may be confirmed by Southern blot analysis of genomic DNA (Sambrook and Russell, 2001, *supra*)*.* In general, total DNA is extracted from the transformant, digested with appropriate restriction enzymes, fractionated in an agarose gel and transferred to a nitrocellulose or nylon membrane. The membrane or "blot" is then probed with, for example, radiolabeled ³²P target DNA fragment to confirm the integration of introduced gene into the plant genome according to standard techniques (Sambrook and Russell, 2001, *supra*)*.*

In Northern blot analysis, RNA is isolated from specific tissues of transformant, fractionated in a formaldehyde agarose gel, and blotted onto a nylon filter according to standard procedures that are routinely used in the art (Sambrook and Russell, 2001, *supra*)*.* Expression of RNA encoded by the pesticidal gene is then tested by hybridizing the filter to a radioactive probe derived from a pesticidal gene, by methods known in the art (Sambrook and Russell, 2001, *supra*)*.*

Western blot, biochemical assays and the like may be carried out on the transgenic plants to confirm the presence of protein encoded by the pesticidal gene by standard procedures (Sambrook and Russell, 2001, *supra*) using antibodies that bind to one or more epitopes present on the pesticidal protein.

### Pesticidal Activity in Plants

In another aspect of the invention, one may generate transgenic plants expressing a pesticidal protein that has pesticidal activity. Methods described above by way of example may be utilized to generate transgenic plants, but the manner in which the transgenic plant cells are generated is not critical to this invention. Methods known or described in the art such as *Agrobacterium*-mediated transformation, biolistic transformation, and non-particle-mediated methods may be used at the discretion of the experimenter. Plants expressing a pesticidal protein may be isolated by common methods described in the art, for example by transformation of callus, selection of transformed callus, and regeneration of fertile plants from such transgenic callus. In such process, one may use any gene as a selectable marker so long as its expression in plant cells confers ability to identify or select for transformed cells.

A number of markers have been developed for use with plant cells, such as resistance to chloramphenicol, the aminoglycoside G418, hygromycin, or the like. Other genes that encode a product involved in chloroplast metabolism may also be used as selectable markers. For example, genes that provide resistance to plant herbicides such as glyphosate, bromoxynil, or imidazolinone may find particular use. Such genes have been reported (Stalker et al. (1985) J. Biol. Chem. 263:6310-6314 (bromoxynil resistance nitrilase gene); and Sathasivan et al. (1990) Nucl. Acids Res. 18:2188 (AHAS imidazolinone resistance gene). Additionally, the genes disclosed herein are useful as markers to assess transformation of bacterial or plant cells. Methods for detecting the presence of a transgene in a plant, plant organ (e.g., leaves, stems, roots, etc.), seed, plant cell, propagule, embryo or progeny of the same are well known in the art. In one embodiment, the presence of the transgene is detected by testing for pesticidal activity.

Fertile plants expressing a pesticidal protein may be tested for pesticidal activity, and the plants showing optimal activity selected for further breeding. Methods are available in the art to assay for pest activity. Generally, the protein is mixed and used in feeding assays. See, for example Marrone et al. (1985) J. of Economic Entomology 78:290-293.

The present invention may be used for transformation of any plant species, including, but not limited to, monocots and dicots. Examples of plants of interest include, but are not limited to, corn (maize), sorghum, wheat, sunflower, tomato, crucifers, peppers, potato, cotton, rice, soybean, sugarbeet, sugarcane, tobacco, barley, and oilseed rape, *Brassica* sp., alfalfa, rye, millet, safflower, peanuts, sweet potato, cassava, coffee, coconut, pineapple, citrus trees, cocoa, tea, banana, avocado, fig, guava, mango, olive, papaya, cashew, macadamia, almond, oats, vegetables, ornamentals, and conifers.

Vegetables include, but are not limited to, tomatoes, lettuce, green beans, lima beans, peas, and members of the genus *Curcumis* such as cucumber, cantaloupe, and musk melon. Ornamentals include, but are not limited to, azalea, hydrangea, hibiscus, roses, tulips, daffodils, petunias, carnation, poinsettia, and chrysanthemum. Preferably, plants of the present invention are crop plants (for example, maize, sorghum, wheat, sunflower, tomato, crucifers, peppers, potato, cotton, rice, soybean, sugarbeet, sugarcane, tobacco, barley, oilseed rape., etc.).

### Use in Pesticidal Control

General methods for employing strains comprising a nucleotide sequence of the present invention, or a variant thereof, in pesticide control or in engineering other organisms as pesticidal agents are known in the art. See, for example U.S. Patent No. 5,039,523 and EP 0480762A2.

The *Bacillus* strains containing a nucleotide sequence of the present invention, or a variant thereof, or the microorganisms that have been genetically altered to contain a pesticidal gene and protein may be used for protecting agricultural crops and products from pests. In one aspect of the invention, whole, i.e., unlysed, cells of a toxin (pesticide)-producing organism are treated with reagents that prolong the activity of the toxin produced in the cell when the cell is applied to the environment of target pest(s).

Alternatively, the pesticide is produced by introducing a pesticidal gene into a cellular host. Expression of the pesticidal gene results, directly or indirectly, in the intracellular production and maintenance of the pesticide. In one aspect of this invention, these cells are then treated under conditions that prolong the activity of the toxin produced in the cell when the cell is applied to the environment of target pest(s). The resulting product retains the toxicity of the toxin. These naturally encapsulated pesticides may then be formulated in accordance with conventional techniques for application to the environment hosting a target pest, e.g., soil, water, and foliage of plants. See, for example EPA 0192319, and the references cited therein. Alternatively, one may formulate the cells expressing a gene of this invention such as to allow application of the resulting material as a pesticide.

The active ingredients of the present invention are normally applied in the form of compositions and can be applied to the crop area or plant to be treated, simultaneously or in succession, with other compounds. These compounds can be fertilizers, weed killers, cryoprotectants, surfactants, detergents, pesticidal soaps, dormant oils, polymers, and/or time-release or biodegradable carrier formulations that permit long-term dosing of a target area following a single application of the formulation. They can also be selective herbicides, chemical insecticides, virucides, microbicides, amoebicides, pesticides, fungicides, bacteriocides, nematocides, molluscicides or mixtures of several of these preparations, if desired, together with further agriculturally acceptable carriers, surfactants or application-promoting adjuvants customarily employed in the art of formulation. Suitable carriers and adjuvants can be solid or liquid and correspond to the substances ordinarily employed in formulation technology, e.g. natural or regenerated mineral substances, solvents, dispersants, wetting agents, tackifiers, binders or fertilizers. Likewise the formulations may be prepared into edible "baits" or fashioned into pest "traps" to permit feeding or ingestion by a target pest of the pesticidal formulation.

Methods of applying an active ingredient of the present invention or an agrochemical composition of the present invention that contains at least one of the pesticidal proteins produced by the bacterial strains of the present invention include leaf application, seed coating and soil application. The number of applications and the rate of application depend on the intensity of infestation by the corresponding pest.

The composition may be formulated as a powder, dust, pellet, granule, spray, emulsion, colloid, solution, or such like, and may be prepared by such conventional means as desiccation, lyophilization, homogenation, extraction, filtration, centrifugation, sedimentation, or concentration of a culture of cells comprising the polypeptide. In all such compositions that contain at least one such pesticidal polypeptide, the polypeptide may be present in a concentration of from about 1% to about 99% by weight.

Lepidopteran, dipteran, or coleopteran pests may be killed or reduced in numbers in a given area by the methods of the invention, or may be prophylactically applied to an environmental area to prevent infestation by a susceptible pest. Preferably the pest ingests, or is contacted with, a pesticidally-effective amount of the polypeptide. By "pesticidally-effective amount" is intended an amount of the pesticide that is able to bring about death to at least one pest, or to noticeably reduce pest growth, feeding, or normal physiological development. This amount will vary depending on such factors as, for example, the specific target pests to be controlled, the specific environment, location, plant, crop, or agricultural site to be treated, the environmental conditions, and the method, rate, concentration, stability, and quantity of application of the pesticidally-effective polypeptide composition. The formulations may also vary with respect to climatic conditions, environmental considerations, and/or frequency of application and/or severity of pest infestation.

The pesticide compositions described may be made by formulating either the bacterial cell, crystal and/or spore suspension, or isolated protein component with the desired agriculturally-acceptable carrier. The compositions may be formulated prior to administration in an appropriate means such as lyophilized, freeze-dried, desiccated, or in an aqueous carrier, medium or suitable diluent, such as saline or other buffer. The formulated compositions may be in the form of a dust or granular material, or a suspension in oil (vegetable or mineral), or water or oil/water emulsions, or as a wettable powder, or in combination with any other carrier material suitable for agricultural application. Suitable agricultural carriers can be solid or liquid and are well known in the art. The term "agriculturally-acceptable carrier" covers all adjuvants, inert components, dispersants, surfactants, tackifiers, binders, etc. that are ordinarily used in pesticide formulation technology; these are well known to those skilled in pesticide formulation. The formulations may be mixed with one or more solid or liquid adjuvants and prepared by various means, e.g., by homogeneously mixing, blending and/or grinding the pesticidal composition with suitable adjuvants using conventional formulation techniques. Suitable formulations and application methods are described in U.S. Patent No. 6,468,523, herein incorporated by reference.

"Pest" includes but is not limited to, insects, fungi, bacteria, nematodes, mites, ticks, and the like. Insect pests include insects selected from the orders *Coleoptera, Diptera, Hymenoptera, Lepidopteran, Mallophaga, Homoptera, Hemiptera, Orthroptera, Thysanoptera, Dermaptera, Isoptera, Anoplura, Siphonaptera, Trichoptera,* etc., particularly *Coleoptera, Lepidopteran,* and *Diptera.*

The order *Coleoptera* includes the suborders *Adephaga* and *Polyphaga.* Suborder *Adephaga* includes the superfamilies *Caraboidea* and *Gyrinoidea,* while suborder *Polyphaga* includes the superfamilies *Hydrophiloidea, Staphylinoidea, Cantharoidea, Cleroidea, Elateroidea, Dascilloidea, Dryopoidea, Byrrhoidea, Cucujoidea, Meloidea, Mordelloidea, Tenebrionoidea, Bostrichoidea, Scarabaeoidea, Cerambycoidea, Chrysomeloidea,* and *Curculionoidea.* Superfamily *Caraboidea* includes the families *Cicindelidae, Carabidae,* and *Dytiscidae.* Superfamily *Gyrinoidea* includes the family *Gyrinidae.* Superfamily *Hydrophiloidea* includes the family *Hydrophilidae.* Superfamily *Staphylinoidea* includes the families *Silphidae* and *Staphylinidae.* Superfamily *Cantharoidea* includes the families *Cantharidae* and *Lampyridae.* Superfamily *Cleroidea* includes the families *Cleridae* and *Dermestidae.* Superfamily *Elateroidea* includes the families *Elateridae* and *Buprestidae.* Superfamily *Cucujoidea* includes the family *Coccinellidae.* Superfamily *Meloidea* includes the family *Meloidae.* Superfamily *Tenebrionoidea* includes the family *Tenebrionidae.* Superfamily *Scarabaeoidea* includes the families *Passalidae* and *Scarabaeidae.* Superfamily *Cerambycoidea* includes the family *Cerambycidae.* Superfamily *Chrysomeloidea* includes the family *Chrysomelidae.* Superfamily *Curculionoidea* includes the families *Curculionidae* and *Scolytidae.*

The order *Diptera* includes the Suborders *Nematocera, Brachycera,* and *Cyclorrhapha.* Suborder *Nematocera* includes the families *Tipulidae, Psychodidae, Culicidae, Ceratopogonidae, Chironomidae, Simuliidae, Bibionidae,* and *Cecidomyiidae.* Suborder *Brachycera* includes the families *Stratiomyidae, Tabanidae, Therevidae, Asilidae, Mydidae, Bombyliidae,* and *Dolichopodidae.* Suborder *Cyclorrhapha* includes the Divisions *Aschiza* and *Aschiza.* Division *Aschiza* includes the families *Phoridae, Syrphidae,* and *Conopidae.* Division *Aschiza* includes the Sections *Acalyptratae* and *Calyptratae.* Section *Acalyptratae* includes the families *Otitidae, Tephritidae, Agromyzidae,* and *Drosophilidae.* Section *Calyptratae* includes the families *Hippoboscidae,* Oestridae, *Tachinidae, Anthomyiidae, Muscidae, Calliphoridae,* and *Sarcophagidae.*

The order *Lepidopteran* includes the families *Papilionidae, Pieridae, Lycaenidae, Nymphalidae, Danaidae, Satyridae, Hesperiidae, Sphingidae, Saturniidae, Geometridae, Arctiidae, Noctuidae, Lymantriidae, Sesiidae,* and *Tineidae.*

Insect pests of the invention for the major crops include: Maize: *Ostrinia nubilalis,* European corn borer; *Agrotis ipsilon,* black cutworm; *Helicoverpa zea,* corn earworm; *Spodoptera frugiperda,* fall armyworm; *Diatraea grandiosella,* southwestern corn borer; *Elasmopalpus lignosellus,* lesser cornstalk borer; *Diatraea saccharalis,* surgarcane borer; *Diabrotica virgifera,* western corn rootworm; *Diabrotica longicornis barberi,* northern corn rootworm; *Diabrotica undecimpunctata howardi,* southern corn rootworm; *Melanotus* spp., wireworms; *Cyclocephala borealis,* northern masked chafer (white grub); *Cyclocephala immaculata,* southern masked chafer (white grub); *Popillia japonica,* Japanese beetle; *Chaetocnema pulicaria,* corn flea beetle; *Sphenophorus maidis,* maize billbug; *Rhopalosiphum maidis,* corn leaf aphid; *Anuraphis maidiradicis,* corn root aphid; *Blissus leucopterus leucopterus,* chinch bug; *Melanoplus femurrubrum*, redlegged grasshopper; *Melanoplus sanguinipes,* migratory grasshopper; *Hylemya platura,* seedcorn maggot; *Agromyza parvicornis,* corn blot leafminer; *Anaphothrips obscrurus,* grass thrips; *Solenopsis milesta,* thief ant; *Tetranychus urticae,* twospotted spider mite; Sorghum: *Chilo partellus,* sorghum borer; *Spodoptera frugiperda,* fall armyworm; *Helicoverpa zea,* corn earworm; *Elasmopalpus lignosellus,* lesser cornstalk borer; *Feltia subterranea,* granulate cutworm; *Phyllophaga crinita,* white grub; *Eleodes, Conoderus,* and *Aeolus* spp., wireworms; *Oulema melanopus,* cereal leaf beetle; *Chaetocnema pulicaria,* corn flea beetle; *Sphenophorus maidis,* maize billbug; *Rhopalosiphum maidis*; corn leaf aphid; *Sipha flava,* yellow sugarcane aphid; *Blissus leucopterus leucopterus,* chinch bug; *Contarinia sorghicola,* sorghum midge; *Tetranychus cinnabarinus,* carmine spider mite; *Tetranychus urticae,* twospotted spider mite; Wheat: *Pseudaletia unipunctata,* army worm; *Spodoptera frugiperda,* fall armyworm; *Elasmopalpus lignosellus,* lesser cornstalk borer; *Agrotis orthogonia,* western cutworm; *Elasmopalpus lignosellus,* lesser cornstalk borer; *Oulema melanopus,* cereal leaf beetle; *Hypera punctata,* clover leaf weevil; *Diabrotica undecimpunctata howardi,* southern corn rootworm; Russian wheat aphid; *Schizaphis graminum,* greenbug; *Macrosiphum avenae,* English grain aphid; *Melanoplus femurrubrum*, redlegged grasshopper; *Melanoplus differential,* differential grasshopper; *Melanoplus sanguinipes,* migratory grasshopper; *Mayetiola destructor,* Hessian fly; *Sitodiplosis mosellana,* wheat midge; *Meromyza americana,* wheat stem maggot; *Hylemya coarctata,* wheat bulb fly; *Frankliniella fusca,* tobacco thrips; *Cephus cinctus,* wheat stem sawfly; *Aceria tulipae,* wheat curl mite; Sunflower: *Suleima helianthana,* sunflower bud moth; *Homoeosoma electellum,* sunflower moth; *zygogramma exclamationis,* sunflower beetle; *Bothyrus gibbosus,* carrot beetle; *Neolasioptera murtfeldtiana,* sunflower seed midge; Cotton: *Heliothis virescens,* cotton budworm; *Helicoverpa zea,* cotton bollworm; *Spodoptera exigua,* beet armyworm; *Pectinophora gossypiella,* pink bollworm; *Anthonomus grands,* boll weevil; *Aphis gossypii,* cotton aphid; *Pseudatomoscelis seriatus,* cotton fleahopper; *Trialeurodes abutilonea,* bandedwinged whitefly; *Lygus lineolaris,* tarnished plant bug; *Melanoplus femurrubrum*, redlegged grasshopper; *Melanoplus differentialis,* differential grasshopper; *Thrips tabaci,* onion thrips; *Franklinkiella fusca,* tobacco thrips; *Tetranychus cinnabarinus,* carmine spider mite; *Tetranychus urticae,* twospotted spider mite; Rice: *Diatraea saccharalis,* sugarcane borer; *Spodoptera frugiperda,* fall armyworm; *Helicoverpa zea,* corn earworm; *Colaspis brunnea,* grape colaspis; *Lissorhoptrus oryzophilus,* rice water weevil; *Sitophilus oryzae,* rice weevil; *Nephotettix nigropictus,* rice leafhopper; *Blissus leucopterus leucopterus,* chinch bug; *Acrosternum hilare,* green stink bug; Soybean: *Pseudoplusia includens,* soybean looper; *Anticarsia gemmatalis,* velvetbean caterpillar; *Plathypena scabra,* green cloverworm; *Ostrinia nubilalis,* European corn borer; *Agrotis ipsilon,* black cutworm; *Spodoptera exigua,* beet armyworm; *Heliothis virescens,* cotton budworm; *Helicoverpa zea,* cotton bollworm; *Epilachna varivestis,* Mexican bean beetle; *Myzus persicae,* green peach aphid; *Empoasca fabae,* potato leafhopper; *Acrosternum hilare,* green stink bug; *Melanoplus femurrubrum,* redlegged grasshopper; *Melanoplus differentials,* differential grasshopper; *Hylemya platura,* seedcorn maggot; *Sericothrips variabilis,* soybean thrips; *Thrips tabaci,* onion thrips; *Tetranychus turkestani,* strawberry spider mite; *Tetranychus urticae,* twospotted spider mite; Barley: *Ostrinia nubilalis,* European corn borer; *Agrotis ipsilon,* black cutworm; *Schizaphis graminum,* greenbug; *Blissus leucopterus leucopterus,* chinch bug; *Acrosternum hilare,* green stink bug; *Euschistus servus,* brown stink bug; *Delia platura,* seedcorn maggot; *Mayetiola destructor,* Hessian fly; *Petrobia latens,* brown wheat mite; Oil Seed Rape: *Brevicoryne brassicae,* cabbage aphid; *Phyllotreta cruciferae,* Flea beetle; *Mamestra configurata,* Bertha armyworm; *Plutella xylostella,* Diamond-back moth; *Delia* ssp., Root maggots.

Nematodes include parasitic nematodes such as root-knot, cyst, and lesion nematodes, including *Heterodera* spp., *Meloidogyne* spp., and *Globodera* spp.; particularly members of the cyst nematodes, including, but not limited to, *Heterodera glycines* (soybean cyst nematode); *Heterodera schachtii* (beet cyst nematode); *Heterodera avenae* (cereal cyst nematode); and *Globodera rostochiensis* and *Globodera pailida* (potato cyst nematodes). Lesion nematodes include *Pratylenchus* spp.

### Methods for increasing plant yield

Methods for increasing plant yield are provided. The methods comprise introducing into a plant or plant cell a polynucleotide comprising a pesticidal sequence disclosed herein. As defined herein, the "yield" of the plant refers to the quality and/or quantity of biomass produced by the plant. By "biomass" is intended any measured plant product. An increase in biomass production is any improvement in the yield of the measured plant product. Increasing plant yield has several commercial applications. For example, increasing plant leaf biomass may increase the yield of leafy vegetables for human or animal consumption. Additionally, increasing leaf biomass can be used to increase production of plant-derived pharmaceutical or industrial products. An increase in yield can comprise any statistically significant increase including, but not limited to, at least a 1% increase, at least a 3% increase, at least a 5% increase, at least a 10% increase, at least a 20% increase, at least a 30%, at least a 50%, at least a 70%, at least a 100% or a greater increase in yield compared to a plant not expressing the pesticidal sequence.

The application discloses the following items:
Item 1. An isolated nucleic acid molecule comprising a nucleotide sequence encoding an amino acid sequence having pesticidal activity, wherein said nucleotide sequence is selected from the group consisting of:
   a) the nucleotide sequence set forth in SEQ ID NO:4, 1, 3, 4, 6, 9, or 11;
   b) a nucleotide sequence having at least 90% sequence identity to the nucleotide sequence of SEQ ID NO:4, 1, 3, 4, 6, 9, or 11;
   c) a nucleotide sequence having at least 95% sequence identity to the nucleotide sequence of SEQ ID NO:4;
   d) a nucleotide sequence that encodes a polypeptide comprising the amino acid sequence of SEQ ID NO:5, 2, or 10;
   e) a nucleotide sequence that encodes a polypeptide comprising an amino acid sequence having at least 90% sequence identity to the amino acid sequence of SEQ ID NO:2 or 10;
   f) a nucleotide sequence that encodes a polypeptide comprising an amino acid sequence having at least 95% sequence identity to the amino acid sequence of SEQ ID NO:5; and,
   g) the nucleotide sequence of the DNA insert of the plasmid deposited as Accession No. B-50045.

Item 2. The nucleic acid molecule of item 1, wherein said nucleic acid molecule encodes a sequence having at least 90% sequence identity to the amino acid sequence set forth in SEQ ID NO:10, and wherein said amino acid sequence comprises an insertion of one or two amino acids between resides 379 and 380 of SEQ ID NO:10.

Item 3. The nucleic acid molecule of item 2, wherein said amino acid sequence comprises an insertion of two amino acids between resides 379 and 380 of SEQ ID NO:10, and wherein said insertion is selected from the group consisting of a glycine and a glycine, a glycine and a threonine, a glycine and a serine, a glycine and a leucine, an arginine and a glycine, a glycine and an asparagine, a glycine and a lysine, a histidine and a glycine, a phenylalanine and a glycine, a leucine and a glycine, and an asparagine and a glycine residue.

Item 4. The isolated nucleic acid molecule of item 1, wherein said nucleotide sequence is a synthetic sequence that has been designed for expression in a plant.

Item 5. A vector comprising the nucleic acid molecule of item 1.

Item 6. The vector of item 5, further comprising a nucleic acid molecule encoding a heterologous polypeptide.

Item 7. A host cell that contains the vector of item 5.

Item 8. The host cell of item 7 that is a bacterial host cell.

Item 9. The host cell of item 7 that is a plant cell.

Item 10. A transgenic plant comprising the host cell of item 9.

Item 11. The transgenic plant of item 10, wherein said plant is selected from the group consisting of maize, sorghum, wheat, cabbage, sunflower, tomato, crucifers, peppers, potato, cotton, rice, soybean, sugarbeet, sugarcane, tobacco, barley, and oilseed rape.

Item 12. A transgenic seed comprising the nucleic acid molecule of item 1.

Item 13. An isolated polypeptide with pesticidal activity, selected from the group consisting of:
a) a polypeptide comprising the amino acid sequence of SEQ ID NO:5, 2, or 10;
b) a polypeptide comprising an amino acid sequence having at least 90% sequence identity to the amino acid sequence of SEQ ID NO:2 or 10;
c) a polypeptide comprising an amino acid sequence having at least 95% sequence identity to the amino acid sequence of SEQ ID NO:5;
d) a polypeptide that is encoded by SEQ ID NO:4, 1, 3, 4, 6, 9, or 11;
e) a polypeptide that is encoded by a nucleotide sequence that is at least 90% identical to the nucleotide sequence of SEQ ID NO:9, 1, 3, 6, or 11;
f) a polypeptide that is encoded by a nucleotide sequence that is at least 95% identical to the nucleotide sequence of SEQ ID NO:4; and,
f) a polypeptide encoded by the nucleotide sequence of the DNA insert of the plasmid deposited as Accession No. B-50045.

Item 14. The polypeptide of item 13, wherein said polypeptide comprises a sequence having at least 90% sequence identity to the amino acid sequence set forth in SEQ ID NO:10, and wherein said amino acid sequence comprises an insertion of one or two amino acids between resides 379 and 380 of SEQ ID NO:10.

Item 15. The polypeptide of item 14, wherein said amino acid sequence comprises an insertion of two amino acids between resides 379 and 380 of SEQ ID NO:10, and wherein said insertion is selected from the group consisting of a glycine and a glycine, a glycine and a threonine, a glycine and a serine, a glycine and a leucine, an arginine and a glycine, a glycine and an asparagine, a glycine and a lysine, a histidine and a glycine, a phenylalanine and a glycine, a leucine and a glycine, and an asparagine and a glycine residue.

Item 16. The polypeptide of item 13 further comprising heterologous amino acid sequences.

Item 17. A composition comprising the polypeptide of item 13.

Item 18. The composition of item 17, wherein said composition is selected from the group consisting of a powder, dust, pellet, granule, spray, emulsion, colloid, and solution.

Item 19. The composition of item 17, wherein said composition is prepared by desiccation, lyophilization, homogenization, extraction, filtration, centrifugation, sedimentation, or concentration of a culture of bacterial cells.

Item 20. The composition of item 17, comprising from about 1% to about 99% by weight of said polypeptide.

Item 21. A method for controlling a lepidopteran, coleopteran, nematode, or dipteran pest population comprising contacting said population with a pesticidally-effective amount of a polypeptide of item 13.

Item 22. A method for killing a lepidopteran, coleopteran, nematode, or dipteran pest, comprising contacting said pest with, or feeding to said pest, a pesticidally-effective amount of a polypeptide of item 13.

Item 23. A method for producing a polypeptide with pesticidal activity, comprising culturing the host cell of item 6 under conditions in which the nucleic acid molecule encoding the polypeptide is expressed.

Item 24. A plant having stably incorporated into its genome a DNA construct comprising a nucleotide sequence that encodes a protein having pesticidal activity, wherein said nucleotide sequence is selected from the group consisting of:
a) the nucleotide sequence set forth in SEQ ID NO:4, 1, 3, 4, 6, 9, or 11;
b) a nucleotide sequence having at least 90% sequence identity to the nucleotide sequence of SEQ ID NO:4, 1, 3, 4, 6, 9, or 11;
c) a nucleotide sequence having at least 95% sequence identity to the nucleotide sequence of SEQ ID NO:4;
d) a nucleotide sequence that encodes a polypeptide comprising the amino acid sequence of SEQ ID NO:5, 2, or 10;
e) a nucleotide sequence that encodes a polypeptide comprising an amino acid sequence having at least 90% sequence identity to the amino acid sequence of SEQ ID NO:2 or 10;
f) a nucleotide sequence that encodes a polypeptide comprising an amino acid sequence having at least 95% sequence identity to the amino acid sequence of SEQ ID NO:5; and,
g) the nucleotide sequence of the DNA insert of the plasmid deposited as Accession No. B-50045;
wherein said nucleotide sequence is operably linked to a promoter that drives expression of a coding sequence in a plant cell.

Item 25. The plant of item 24, wherein said nucleic acid molecule encodes a sequence having at least 90% sequence identity to the amino acid sequence set forth in SEQ ID NO:10, and wherein said amino acid sequence comprises an insertion of one or two amino acids between resides 379 and 380 of SEQ ID NO:10.

Item 26. The plant of item 25, wherein said amino acid sequence comprises an insertion of two amino acids between resides 379 and 380 of SEQ ID NO:10, and wherein said insertion is selected from the group consisting of a glycine and a glycine, a glycine and a threonine, a glycine and a serine, a glycine and a leucine, an arginine and a glycine, a glycine and an asparagine, a glycine and a lysine, a histidine and a glycine, a phenylalanine and a glycine, a leucine and a glycine, and an asparagine and a glycine residue.

Item 27. The plant of item 24, wherein said plant is a plant cell.

Item 28. A method for protecting a plant from a pest, comprising introducing into said plant or cell thereof at least one expression vector comprising a nucleotide sequence that encodes a pesticidal polypeptide, wherein said nucleotide sequence is selected from the group consisting of:
a) the nucleotide sequence set forth in SEQ ID NO:4, 1, 3, 4, 6, 9, or 11;
b) a nucleotide sequence having at least 90% sequence identity to the nucleotide sequence of SEQ ID NO:4, 1, 3, 4, 6, 9, or 11;
c) a nucleotide sequence having at least 95% sequence identity to the nucleotide sequence of SEQ ID NO:4;
d) a nucleotide sequence that encodes a polypeptide comprising the amino acid sequence of SEQ ID NO:5, 2, or 10;
e) a nucleotide sequence that encodes a polypeptide comprising an amino acid sequence having at least 90% sequence identity to the amino acid sequence of SEQ ID NO:2 or 10;
f) a nucleotide sequence that encodes a polypeptide comprising an amino acid sequence having at least 95% sequence identity to the amino acid sequence of SEQ ID NO:5; and,
g) the nucleotide sequence of the DNA insert of the plasmid deposited as Accession No. B-50045.

Item 29. The method of item 28, wherein said plant produces a pesticidal polypeptide having pesticidal activity against a lepidopteran, coleopteran, nematode, or dipteran pest.

The following examples are offered by way of illustration and not by way of limitation.

### EXPERIMENTAL

### Example 1. Extraction of Plasmid DNA

A pure culture of strain ATX13046 was grown in large quantities of rich media. The culture was spun to harvest the cell pellet. The cell pellet was then prepared by treatment with SDS by methods known in the art, resulting in breakage of the cell wall and release of DNA. Proteins and large genomic DNA were then precipitated by a high salt concentration. The plasmid DNA was then precipitated by standard ethanol precipitation. The plasmid DNA was separated from any remaining chromosomal DNA by high-speed centrifugation through a cesium chloride gradient. The DNA was visualized in the gradient by UV light and the band of lower density (i.e. the lower band) was extracted using a syringe. This band contained the plasmid DNA from Strain ATX13046. The quality of the DNA was checked by visualization on an agarose gel.

### Example 2. Cloning of Genes

The purified plasmid DNA was sheared into 5-10 kb sized fragments and the 5' and 3' single stranded overhangs repaired using T4 DNA polymerase and Klenow fragment in the presence of all four dNTPs. Phosphates were then attached to the 5' ends by treatment with T4 polynucleotide kinase. The repaired DNA fragments were then ligated overnight into a standard high copy vector (i.e. pBluescript SK+), suitably prepared to accept the inserts as known in the art (for example by digestion with a restriction enzyme producing blunt ends).

The quality of the library was analyzed by digesting a subset of clones with a restriction enzyme known to have a cleavage site flanking the cloning site. A high percentage of clones were determined to contain inserts, with an average insert size of 5-6 kb.

### Example 3. High Throughput Sequencing of Library Plates

Once the clone library quality was checked and confirmed, colonies were grown in a rich broth in 2 ml 96-well blocks overnight at 37°C at a shaking speed of 350 rpm. The blocks were spun to harvest the cells to the bottom of the block. The blocks were then prepared by standard alkaline lysis prep in a high throughput format.

The end sequences of clones from this library were then determined for a large number of clones from each block in the following way: The DNA sequence of each clone chosen for analysis was determined using the fluorescent dye terminator sequencing technique (Applied Biosystems) and standard primers flanking each side of the cloning site. Once the reactions had been carried out in the thermocycler, the DNA was precipitated using standard ethanol precipitation. The DNA was resuspended in water and loaded onto a capillary sequencing machine. Each library plate of DNA was sequenced from either end of the cloning site, yielding two reads per plate over each insert.

### Example 4. Assembly and Screening of Sequencing Data

DNA sequences obtained were compiled into an assembly project and aligned together to form contigs. This can be done efficiently using a computer program, such as Vector NTI, or alternatively by using the Phred/Phrap suite of DNA alignment and analysis programs. These contigs, along with any individual read that may not have been added to a contig, were compared to a compiled database of all classes of known pesticidal genes. Contigs or individual reads identified as having identity to a known endotoxin or pesticidal gene were analyzed further. Among the sequences obtained, DNA clones were identified as having homology to known endotoxin genes. Therefore, these clones were selected for further sequencing.

### Example 5. Cloning of axmi-076

A fragment of DNA with homology to endotoxin genes was identified from Strain ATX14775. The full open reading frame was identified by Tail (Thermal Asymmetric Interlaced) PCR based methods as known in the art. Finally, using the DNA sequence of the full length open reading frame from the Tail (Thermal Asymmetric Interlaced) PCR product, the open reading frame was amplified directly by PCR from strain ATX14775 and cloned into a vector.

### Example 6. Sequencing of clones

Primers were designed to anneal to the clones of interest in a manner such that DNA sequences generated from such primers will overlap existing DNA sequence of the clone(s). This process, known as "oligo walking," is well known in the art. This process was utilized to determine the entire DNA sequence of the region exhibiting homology to a known endotoxin gene. In the case of the genes of the invention, this process was used to determine the DNA sequence of the entire open reading frame, resulting in a single nucleotide sequence for each. The completed DNA sequence was then placed back into the original large assembly for further validation. This allowed incorporation of more DNA sequence reads into the contig, resulting in multiple reads of coverage over the entire region.

Analysis of the DNA sequence of each clone by methods known in the art identified an open reading frame on each insert with homology to known delta endotoxin genes. The open reading frames were designated as *axmi-066* and *axmi-076,* respectively. The DNA sequence of *axmi-066* is provided in SEQ ID NO:1, and the amino acid sequence of the predicted protein is provided in SEQ ID NO:2. The DNA sequence of axmi-076 is provided in SEQ ID NO:4 and its predicted protein sequence is provided in SEQ ID NO:5.

The predicted open reading frame of *axmi-066* is 637 amino acids long. However, alignment with its closest endotoxin homologs suggests that the start of translation of *axmi-066* in *Bacillus* is likely to be at the internal ATG start codon thirty nine nucleotides downstream of the first ATG start codon (corresponding to nucleotide position 39 of SEQ ID NO:1). This coding sequence is set forth in SEQ ID NO:9. Translational initiation at this internal start codon will result in a 624 amino acid protein with a molecular weight of 71kD (SEQ ID NO:10). A possible but strong Shine-Dalgarno sequence is present 6 nucleotides upstream of this internal start codon, which supports the results of protein alignments.

### Example 7. Synthetic nucleotide sequences encoding AXMI-066 and AXMI-076

The *optaxmi-066* gene (SEQ ID NO:3) represents a synthetic nucleotide sequence that upon translation, will encode the AXMI-066 protein.

The *optaxmi-076* gene (SEQ ID NO:6) and the *optaxmi-076v04* (SEQ ID NO:11) gene represent synthetic nucleotide sequences that upon translation, will encode the AXMI-076 protein.

### Example 8. Homology of AXMI-066 and AXMI-076 to Known Endotoxin Genes

A search of DNA and protein databases with the DNA sequences and amino acid sequences of AXMI-066 and AXMI-076 revealed that they are homologous to known delta-endotoxin proteins.

Figure 2 shows an alignment of AXMI-066 with several endotoxins. Blast searches identified members of the *cry2* endotoxin family as having the strongest homology to AXMI-066. Alignment of the AXMI-066 protein (SEQ ID NO:2) to a large set of endotoxin proteins confirmed that AXMI-066 has 74.8% identity to the Cry2Aa1 toxin.

Alignment of the AXMI-076 protein (SEQ ID NO:5) to a large set of endotoxin proteins confirmed that AXMI-076 has 93.1 % identity to the Cry2Ae1 toxin and 91.0% identity to the Cry2Aa1 toxin.

### Example 9. Expression of synthetic sequences in Bacillus

The *axmi-066, optaxmi-066, axmi-076, optaxmi-076v,* or *optaxmi-076v04* sequences (SEQ ID NO:1, 3, 4, 6, and 11, respectively) are amplified by PCR and cloned into the *Bacillus* expression vector such as pAX916 by methods well known in the art. The resulting clone is assayed for expression of the AXMI protein after transformation into cells of a *cry(-) Bacillus thuringiensis* strain. A *Bacillus* strain containing the *axmi* clone and expressing the AXMI insecticidal protein is grown in, for example, CYS media (10 g/l Bacto-casitone; 3 g/l yeast extract; 6 g/l KH₂PO₄; 14 g/l K₂HPO₄; 0.5 mM MgSO₄; 0.05 mM MnCl₂; 0.05 mM FeSO₄), until sporulation is evident by microscopic examination. Samples are prepared, and analyzed by polyacrylamide gel electrophoresis (PAGE).

In the case of AXMI-066, the *axmi066* open reading frame starting from the internal ATG (corresponding to nucleotide positions 39-41 of SEQ ID NO:1) was amplified by PCR. The product was cloned into a Bacillus vector based on pAX916 as well as *E. coli* expression vector based on pRSF1b (Invitrogen). The resulting clones were confirmed by restriction analysis and finally by complete sequencing of the cloned gene. The resulting constructs are called pAX2755 and pAX2757 respectively.

### Example 10. Insecticidal Activity of AXMI-066 and AXMI-076

AXMI-066 and AXMI-076 were tested for activity against important lepidopteran pests by bioassay. Cultures of a Bacillus strain containing pAX2755 were grown to sporulation, pelleted, and tested on insect pests with appropriate controls. In these tests AXMI-066 and AXMI-076 demonstrated activity on several Lepidopteran pests, as summarized Table 1.

**Table 1. Insect activity of AXMI-066 and AXMI-076**

| Insect | AXMI-066 | AXMI-076 |
|---|---|---|
| European corn borer (*Ostrinia nubilalis*) | +/- | ++++ |
| Corn earworm (*Helicoverpa zea*) | ++ | +++ |
| Tobacco budworm (*Heliothis virescens*) | ++ | ++++ |
| Fall armyworm (*Spodoptera frugiperda*) | ++ | ++ |
| Velvetbean caterpillar (*Anticarsia gemmatalis*) | +++ | ++++ |
| Black cutworm (*Agrotis ipsilon*) | - | - |

### Example 11. AXMI-066 variants

Alignment of the AXMI-066 (SEQ ID NO:10) and Cry2Aa protein sequences indicates that the apical loops 1 and 2 of axmi-066 are both 2 amino acids shorter than loops 1 and 2 of Cry2Aa. AXMI-066 contains an additional loop 3 that is missing in Cry2Aa. Variant libraries of AXMI-066 have been generated containing insertions of 2 amino acids in loops 1 and 2, respectively. A deletion of loop 3 in AXMI-066 has also been generated. AXMI-066 variants have been expressed and assayed for insecticidal activity on several Lepidopteran insects. Eleven AXMI-066 variants carrying insertions into loop 2 containing glycines have been identified that are active on several Lepidopteran insects.

Variant libraries of axmi-66 were generated using the Quickchange Lightening kit (Stratagene). Construct pAX5435 (His6-axmi-66 in pRSF1b) was mutagenized. The 2 libraries consist of 2 codon insertions between Val320 and Pro321 (loop 1) and Gly378 and Asp379 (loop 2), respectively. Each library contains permutations of all 64 codons for the two inserted positions. A deletion of loop 3 was also carried out. The mutagenic sense oligos are as follows:
Loop 1:
   1. CTTCCTTCGGCGTGNWNNWNCCCATCCTCGGCGGC (SEQ ID NO:21)
   2. CTTCCTTCGGCGTGNSNNSNCCCATCCTCGGCGGC (SEQ ID NO:22)
   3. CTTCCTTCGGCGTGNWNNSNCCCATCCTCGGCGGC (SEQ ID NO:23)
   4. CTTCCTTCGGCGTGNSNNWNCCCATCCTCGGCGGC (SEQ ID NO:24)
Loop 2:
   1. CGGCGTCTACAGAGGANWNNWNGATCTTCAGCACAACTGG (SEQ ID NO:25)
   2. CGGCGTCTACAGAGGANSNNSNGATCTTCAGCACAACTGG (SEQ ID NO:26)
   3. CGGCGTCTACAGAGGANSNNWNGATCTTCAGCACAACTGG (SEQ ID NO:27)
   4. CGGCGTCTACAGAGGANWNNSNGATCTTCAGCACAACTGG (SEQ ID NO:28)
Loop 3:
   CGCCTTCCTCCTCTCAGTGAAGAGCAACTACTTCC (SEQ ID NO:29)

Mutagenesis reactions contain a sense oligo as described above and the corresponding antisense oligo. The libraries were cloned, and a number of clones were sequenced. Clones selected for functional characterization contained various combinations of positively charged, negatively charged, aromatic, polar and apolar amino acids. The selected insertion variants were expressed in *E. coli* and soluble extracts were prepared by bead beating in 50 mM Na-Carbonate pH 10.5, 1 mM DTT. The extracts were assayed for activity against Corn Earworm "Hz" (*Helicoverpa zea*)*,* European Corn Borer "ECB" (*Ostrinia nubilalis*)*,* Tobacco budworm "Hv" (*Heliothis virescens*)*,* Fall Armyworm "FAW" (*Spodoptera frugiperda*)*,* Black Cutworm "BCW" (*Agrotis ipsilon),* and Velvetbean caterpillar "VBC" (*Anticarsia gemmatalis*)*.* Loop 2 insertion variants containing glycines were active against lepidopteran insects. The loop 2 GT insertion showed the highest toxicity of the variants tested. No activity was detected in the loop 1 insertion variants and the loop 3 deletion variants.

Single point mutations of AXMI-066 (SEQ ID NO:10) were also created and tested against lepidopteran pests. Table 2 lists the position and mutations that resulted in polypeptides having pesticidal activity equal to or greater than the pesticidal activity SEQ ID NO:10.

**Table 2**

| Position relative to SEQ ID NO:10 | Residue in SEQ ID NO:10 | Active mutants |
|---|---|---|
| 83 | P | T |
| 250 | L | I |
| 319 | G | K,F |
| 322 | I | S, V, Q, A |
| 323 | L | F |
| 376 | Y | N, I, R, S, V, A |
| 377 | R | E, Q, L |
| 378 | G | S, A, W |
| 379 | D | V, E |
| 380 | L | M, P, Y |
| 381 | Q | L |
| 401 | L | I |
| 406 | M | H, V, K, E, T, S, A, V, N |
| 407 | F | W, R |

### Example 12. Assays for Pesticidal Activity

The *axmi-066, optaxmi-066, axmi-076, and optaxmi-076* nucleotide sequences of the invention can be tested for their ability to produce pesticidal proteins. The ability of a pesticidal protein to act as a pesticide upon a pest is often assessed in a number of ways. One way well known in the art is to perform a feeding assay. In such a feeding assay, one exposes the pest to a sample containing either compounds to be tested or control samples. Often this is performed by placing the material to be tested, or a suitable dilution of such material, onto a material that the pest will ingest, such as an artificial diet. The material to be tested may be composed of a liquid, solid, or slurry. The material to be tested may be placed upon the surface and then allowed to dry. Alternatively, the material to be tested may be mixed with a molten artificial diet, then dispensed into the assay chamber. The assay chamber may be, for example, a cup, a dish, or a well of a microtiter plate.

Assays for sucking pests (for example aphids) may involve separating the test material from the insect by a partition, ideally a portion that can be pierced by the sucking mouth parts of the sucking insect, to allow ingestion of the test material. Often the test material is mixed with a feeding stimulant, such as sucrose, to promote ingestion of the test compound.

Other types of assays can include microinjection of the test material into the mouth, or gut of the pest, as well as development of transgenic plants, followed by test of the ability of the pest to feed upon the transgenic plant. Plant testing may involve isolation of the plant parts normally consumed, for example, small cages attached to a leaf, or isolation of entire plants in cages containing insects.

Other methods and approaches to assay pests are known in the art, and can be found, for example in Robertson and Preisler, eds. (1992) Pesticide bioassays with arthropods, CRC, Boca Raton, FL. Alternatively, assays are commonly described in the journals *Arthropod Management Tests* and *Journal of Economic Entomology* or by discussion with members of the Entomological Society of America (ESA).

### Example 13. Vectoring of Genes for Plant Expression

The coding regions of the invention are connected with appropriate promoter and terminator sequences for expression in plants. Such sequences are well known in the art and may include the rice actin promoter or maize ubiquitin promoter for expression in monocots, the *Arabidopsis* UBQ3 promoter or CaMV 35S promoter for expression in dicots, and the nos or PinII terminators. Techniques for producing and confirming promoter - gene - terminator constructs also are well known in the art.

In one aspect of the invention, synthetic DNA sequences are designed and generated. These synthetic sequences have altered nucleotide sequence relative to the parent sequence, but encode proteins that are essentially identical to the parent AXMI-066 or AXMI-076 protein (e.g., SEQ ID NO:3 or 6).

In another aspect of the invention, modified versions of the synthetic genes are designed such that the resulting peptide is targeted to a plant organelle, such as the endoplasmic reticulum or the apoplast. Peptide sequences known to result in targeting of fusion proteins to plant organelles are known in the art. For example, the N-terminal region of the acid phosphatase gene from the White Lupin *Lupinus albus* (GENBANK® ID GI:14276838, Miller et al. (2001) Plant Physiology 127: 594-606) is known in the art to result in endoplasmic reticulum targeting of heterologous proteins. If the resulting fusion protein also contains an endoplasmic reticulum retention sequence comprising the peptide N-terminus-lysine-aspartic acid-glutamic acid-leucine (i.e., the "KDEL" motif, SEQ ID NO:7) at the C-terminus, the fusion protein will be targeted to the endoplasmic reticulum. If the fusion protein lacks an endoplasmic reticulum targeting sequence at the C-terminus, the protein will be targeted to the endoplasmic reticulum, but will ultimately be sequestered in the apoplast.

Thus, this gene encodes a fusion protein that contains the N-terminal thirty-one amino acids of the acid phosphatase gene from the White Lupin *Lupinus albus* (GENBANK® ID GI:14276838 , Miller *et al.,* 2001, *supra*) fused to the N-terminus of the AXMI-066 or AXMI-076 sequence, as well as the KDEL sequence at the C-terminus. Thus, the resulting protein is predicted to be targeted the plant endoplasmic reticulum upon expression in a plant cell.

A construct comprising a nucleotide sequence encoding a chloroplast transit peptide derived from *Chlamydomonas reinhardtii* linked to the *optaxmi-076v04* sequence is set forth in SEQ ID NO:12 (nucleotide sequence) and SEQ ID NO:13 (amino acid sequence).

The plant expression cassettes described above are combined with an appropriate plant selectable marker to aid in the selection of transformed cells and tissues, and ligated into plant transformation vectors. These may include binary vectors from *Agrobacterium-*mediated transformation or simple plasmid vectors for aerosol or biolistic transformation.

### Example 14. Vectoring of axmi-066, optaxmi-066, axmi-076, and optaxmi-076 genes for Plant Expression

The coding region DNA of the *axmi-066, optaxmi-066, axmi-076, and optaxmi-*076 genes of the invention are operably connected with appropriate promoter and terminator sequences for expression in plants. Such sequences are well known in the art and may include the rice actin promoter or maize ubiquitin promoter for expression in monocots, the *Arabidopsis* UBQ3 promoter or CaMV 35S promoter for expression in dicots, and the nos or PinII terminators. Techniques for producing and confirming promoter - gene - terminator constructs also are well known in the art.

The plant expression cassettes described above are combined with an appropriate plant selectable marker to aid in the selections of transformed cells and tissues, and ligated into plant transformation vectors. These may include binary vectors from *Agrobacterium-*mediated transformation or simple plasmid vectors for aerosol or biolistic transformation.

### Example 15. Transformation of Maize Cells with the pesticidal protein genes described herein

Maize ears are best collected 8-12 days after pollination. Embryos are isolated from the ears, and those embryos 0.8-1.5 mm in size are preferred for use in transformation. Embryos are plated scutellum side-up on a suitable incubation media, such as DN62A5S media (3.98 g/L N6 Salts; 1 mL/L (of 1000x Stock) N6 Vitamins; 800 mg/L L-Asparagine; 100 mg/L Myo-inositol; 1.4 g/L L-Proline; 100 mg/L Casamino acids; 50 g/L sucrose; 1 mL/L (of 1 mg/mL Stock) 2,4-D). However, media and salts other than DN62A5S are suitable and are known in the art. Embryos are incubated overnight at 25°C in the dark. However, it is not necessary *per se* to incubate the embryos overnight.

The resulting explants are transferred to mesh squares (30-40 per plate), transferred onto osmotic media for about 30-45 minutes, then transferred to a beaming plate (see, for example, PCT Publication No. WO/0138514 and U.S. Patent No. 5,240,842).

DNA constructs designed to the genes of the invention in plant cells are accelerated into plant tissue using an aerosol beam accelerator, using conditions essentially as described in PCT Publication No. WO/0138514. After beaming, embryos are incubated for about 30 min on osmotic media, and placed onto incubation media overnight at 25°C in the dark. To avoid unduly damaging beamed explants, they are incubated for at least 24 hours prior to transfer to recovery media. Embryos are then spread onto recovery period media, for about 5 days, 25°C in the dark, then transferred to a selection media. Explants are incubated in selection media for up to eight weeks, depending on the nature and characteristics of the particular selection utilized. After the selection period, the resulting callus is transferred to embryo maturation media, until the formation of mature somatic embryos is observed. The resulting mature somatic embryos are then placed under low light, and the process of regeneration is initiated by methods known in the art. The resulting shoots are allowed to root on rooting media, and the resulting plants are transferred to nursery pots and propagated as transgenic plants.

### Materials

**DN62A5S Media**

| Components | Per Liter | Source |
|---|---|---|
| Chu's N6 Basal Salt Mixture (Prod. No. C 416) | 3.98 g/L | Phytotechnology Labs |
| Chu's N6 Vitamin Solution (Prod. No. C 149) | 1 mL/L (of 1000x Stock) | Phytotechnology Labs |
| L-Asparagine | 800 mg/L | Phytotechnology Labs |
| Myo-inositol | 100 mg/L | Sigma |
| L-Proline | 1.4 g/L | Phytotechnology Labs |
| Casamino acids | 100 mg/L | Fisher Scientific |
| Sucrose | 50 g/L | Phytotechnology Labs |
| 2,4-D (Prod. No. D-7299) | 1 mL/L (of 1 mg/mL Stock) | Sigma |

The pH of the solution is adjusted to pH 5.8 with 1N KOH/1N KCl, Gelrite (Sigma) is added at a concentration up to 3g/L, and the media is autoclaved. After cooling to 50°C, 2 ml/L of a 5 mg/ml stock solution of silver nitrate (Phytotechnology Labs) is added.

### Example 16. Transformation of the axmi-066, optaxmi-066, axmi-076, and optaxmi-076 genes of the invention in Plant Cells by Agrobacterium-Mediated Transformation

Ears are best collected 8-12 days after pollination. Embryos are isolated from the ears, and those embryos 0.8-1.5 mm in size are preferred for use in transformation. Embryos are plated scutellum side-up on a suitable incubation media, and incubated overnight at 25°C in the dark. However, it is not necessary *per se* to incubate the embryos overnight. Embryos are contacted with an *Agrobacterium* strain containing the appropriate vectors for Ti plasmid mediated transfer for about 5-10 min, and then plated onto co-cultivation media for about 3 days (25°C in the dark). After co-cultivation, explants are transferred to recovery period media for about five days (at 25°C in the dark). Explants are incubated in selection media for up to eight weeks, depending on the nature and characteristics of the particular selection utilized. After the selection period, the resulting callus is transferred to embryo maturation media, until the formation of mature somatic embryos is observed. The resulting mature somatic embryos are then placed under low light, and the process of regeneration is initiated as known in the art.

All publications and patent applications mentioned in the specification are indicative of the level of skill of those skilled in the art to which this invention pertains. All publications and patent applications are herein incorporated by reference to the same extent as if each individual publication or patent application was specifically and individually indicated to be incorporated by reference.

Although the foregoing invention has been described in some detail by way of illustration and example for purposes of clarity of understanding, it will be obvious that certain changes and modifications may be practiced within the scope of the appended claims.

## Claims

1. A nucleic acid molecule comprising a nucleotide sequence encoding an amino acid sequence having pesticidal activity, wherein said nucleotide sequence is selected from the group consisting of:
a) the nucleotide sequence set forth in SEQ ID NO: 4, 6, or 11; and
b) a nucleotide sequence that encodes a polypeptide comprising the amino acid sequence of SEQ ID NO: 5.

2. The nucleic acid molecule of claim 1, wherein said nucleotide sequence is a synthetic sequence that has been designed for expression in a plant.

3. An expression cassette comprising the nucleic acid molecule of claim 1.

4. The expression cassette of claim 3, further comprising a nucleic acid molecule encoding a heterologous polypeptide.

5. A host cell that contains the expression cassette of claim 3.

6. The host cell of claim 5 that is a bacterial host cell or a plant cell.

7. A transgenic plant comprising the host plant cell of claim 6.

8. The transgenic plant of claim 7, wherein said plant is selected from the group consisting of maize, sorghum, wheat, cabbage, sunflower, tomato, crucifers, peppers, potato, cotton, rice, soybean, sugarbeet, sugarcane, tobacco, barley, and oilseed rape.

9. A transgenic seed comprising the nucleic acid molecule of claim 1.

10. A polypeptide with pesticidal activity comprising the amino acid sequence of SEQ ID NO: 5.

11. The polypeptide of claim 10 further comprising heterologous amino acid sequences.

12. A composition comprising the polypeptide of claim 10.

13. The composition of claim 12, wherein said composition:
a) is selected from the group consisting of a powder, dust, pellet, granule, spray, emulsion, colloid, and solution, or
b) is prepared by desiccation, lyophilization, homogenization, extraction, filtration, centrifugation, sedimentation, or concentration of a culture of bacterial cells, or
c) comprises from about 1% to about 99% by weight of said polypeptide.

14. A method for killing or controlling a lepidopteran pest population comprising contacting said population with a pesticidally-effective amount of a polypeptide of claim 10.

15. A method for producing a polypeptide with pesticidal activity, comprising culturing the host cell of claim 5 under conditions in which the nucleic acid molecule encoding the polypeptide is expressed.

16. A plant or a plant cell having stably incorporated into its genome a DNA construct comprising a nucleotide sequence that encodes a protein having pesticidal activity, wherein said nucleotide sequence is selected from the group consisting of:
a) the nucleotide sequence set forth in SEQ ID NO: 4, 6, or 11; and
b) a nucleotide sequence that encodes a polypeptide comprising the amino acid sequence of SEQ ID NO: 5.

17. A method for protecting a plant from a pest, comprising introducing into said plant or cell thereof at least one expression cassette comprising a nucleotide sequence that encodes a pesticidal polypeptide, wherein said nucleotide sequence is selected from the group consisting of:
a) the nucleotide sequence set forth in SEQ ID NO: 4, 6, or 11; and
b) a nucleotide sequence that encodes a polypeptide comprising the amino acid sequence of SEQ ID NO: 5.


SEQUENCE LISTING

<110> Daniel J. Tomso
      Kimberly S. Sampson
      Nalini Desai
      Shruti Agarwal
      Sandra Volrath
      Volker Heinrichs

<120> AXMI-066 AND AXMI-076: DELTA-ENDOTOXIN
  PROTEINS AND METHODS FOR THEIR USE


<130> B08862A JAZ/AFL/BA

<140> EPXXXXXXXX
<151> 2017-01-26

<150> EP08840217.7
<151> 2008-10-16

<150> PCT/US2008/080090
<151> 2008-10-16

<150> 60/980,439
<151> 2007-10-16

<160> 29

<170> FastSEQ for Windows Version 4.0

<210> 1
<211> 1911
<212> DNA
<213> Bacillus thuringiensis

<220>
<221> CDS
<222> (1)...(1911)

<400> 1
atg tta tta tat aac cca ata ttt caa gga gga ttt tat atg aat aat    48
Met Leu Leu Tyr Asn Pro Ile Phe Gln Gly Gly Phe Tyr Met Asn Asn
 1               5                  10                  15

gta ttg aat agc gaa aga act aat aag tgt gat gcg tat aac gta gtg    96
Val Leu Asn Ser Glu Arg Thr Asn Lys Cys Asp Ala Tyr Asn Val Val
            20                  25                  30

gcc cat gat cca ttt agt ttt gag cat aaa tca tta gat acc ata cag    144
Ala His Asp Pro Phe Ser Phe Glu His Lys Ser Leu Asp Thr Ile Gln
        35                  40                  45

caa gaa tgg atg gag tgg aaa aga acc gat cat agt tta tat gta tct    192
Gln Glu Trp Met Glu Trp Lys Arg Thr Asp His Ser Leu Tyr Val Ser
    50                  55                  60

cct att gtg gga act ata gct agt ttt ctg cta aag aaa ata gcg ggg    240
Pro Ile Val Gly Thr Ile Ala Ser Phe Leu Leu Lys Lys Ile Ala Gly
 65                  70                  75                  80

ctt ata gga aaa aga ata tta agt gag tta aag aat tta att ttt cct    288

Leu Ile Gly Lys Arg Ile Leu Ser Glu Leu Lys Asn Leu Ile Phe Pro
                85                  90                  95

agt ggt agt ata gaa tca atg caa gat att tta aga ggg gca gaa caa     336
Ser Gly Ser Ile Glu Ser Met Gln Asp Ile Leu Arg Gly Ala Glu Gln
            100                 105                 110

ttc cta aat caa aga ctt gat gca gac acc ttt gct cgg gta gag gca     384
Phe Leu Asn Gln Arg Leu Asp Ala Asp Thr Phe Ala Arg Val Glu Ala
        115                 120                 125

gaa ttg ata ggg ctt caa gca aat gta gag gaa ttt aat caa caa gtg     432
Glu Leu Ile Gly Leu Gln Ala Asn Val Glu Glu Phe Asn Gln Gln Val
    130                 135                 140

gac aat ttt tta aac cca aat caa aac cct gtt cct tta gca ata att     480
Asp Asn Phe Leu Asn Pro Asn Gln Asn Pro Val Pro Leu Ala Ile Ile
145                 150                 155                 160

gat tcg gtt aat aca atg caa caa tta ttc cta agt aga tta ccc cag     528
Asp Ser Val Asn Thr Met Gln Gln Leu Phe Leu Ser Arg Leu Pro Gln
                165                 170                 175

ttc cag ata caa cgc tat cag cta tta tta tta cct tta ttt gca caa     576
Phe Gln Ile Gln Arg Tyr Gln Leu Leu Leu Leu Pro Leu Phe Ala Gln
            180                 185                 190

gca gcc aat tta cac ctt acc ttt att aga gat gtt att ctt aat gca     624
Ala Ala Asn Leu His Leu Thr Phe Ile Arg Asp Val Ile Leu Asn Ala
        195                 200                 205

gat gaa tgg gga ata cca gca gca aca gtg cgc aca tat aga gag cac     672
Asp Glu Trp Gly Ile Pro Ala Ala Thr Val Arg Thr Tyr Arg Glu His
    210                 215                 220

cta aaa aga tat aca cgc gat tat tcc aat tat tgt ata aac acg tac     720
Leu Lys Arg Tyr Thr Arg Asp Tyr Ser Asn Tyr Cys Ile Asn Thr Tyr
225                 230                 235                 240

caa act gct ttc cga ggt tta aac act cgt tta cat gat atg tta gag     768
Gln Thr Ala Phe Arg Gly Leu Asn Thr Arg Leu His Asp Met Leu Glu
                245                 250                 255

ttt aga aca ttt atg ttt tta aat gta tta gac tat gta tct atc tgg     816
Phe Arg Thr Phe Met Phe Leu Asn Val Leu Asp Tyr Val Ser Ile Trp
            260                 265                 270

tcg ttg ttt aaa tat caa agt ctg atg gtt act tca agt gct aat tta     864
Ser Leu Phe Lys Tyr Gln Ser Leu Met Val Thr Ser Ser Ala Asn Leu
        275                 280                 285

tat gct tcg gga agt ggt agt aat caa cct ttt act gca caa gac tgg     912
Tyr Ala Ser Gly Ser Gly Ser Asn Gln Pro Phe Thr Ala Gln Asp Trp
    290                 295                 300

cca ttt tta tat tct ctt ttc caa gtg aat tca aat tat ata atg tct     960
Pro Phe Leu Tyr Ser Leu Phe Gln Val Asn Ser Asn Tyr Ile Met Ser
305                 310                 315                 320

aat ttt ggt ggt aac cga gag act gct agt ttt ggt gtt cct att ctg     1008
Asn Phe Gly Gly Asn Arg Glu Thr Ala Ser Phe Gly Val Pro Ile Leu
                325                 330                 335

ggg gga ttc ata ata aat ttt tta ctt agt ttt agg gtt aat tat act 1056
Gly Gly Phe Ile Ile Asn Phe Leu Leu Ser Phe Arg Val Asn Tyr Thr
            340                 345                 350

gga gga gtt tca tct ggt ctc cta ggt gtt gaa gga att tca aac aac 1104
Gly Gly Val Ser Ser Gly Leu Leu Gly Val Glu Gly Ile Ser Asn Asn
        355                 360                 365

ttt aat tgc aac tcc tct tta tca aca cca gtt gta aga agt tgg cta 1152
Phe Asn Cys Asn Ser Ser Leu Ser Thr Pro Val Val Arg Ser Trp Leu
    370                 375                 380

gat tca ggt gta tat cga ggt gac ctg caa cac aat tgg cga aca gac 1200
Asp Ser Gly Val Tyr Arg Gly Asp Leu Gln His Asn Trp Arg Thr Asp
385                 390                 395                 400

atc ttt atg agg act aat att gta cct tgt ggt gct ttt cta tta tct 1248
Ile Phe Met Arg Thr Asn Ile Val Pro Cys Gly Ala Phe Leu Leu Ser
                405                 410                 415

ctt gct atg ttt cca gat gtt aaa agt aat tat ttt cct gat tat ttc 1296
Leu Ala Met Phe Pro Asp Val Lys Ser Asn Tyr Phe Pro Asp Tyr Phe
            420                 425                 430

att cgt aac att tcc gga att att cga aat att gat aac atg aat ttg 1344
Ile Arg Asn Ile Ser Gly Ile Ile Arg Asn Ile Asp Asn Met Asn Leu
        435                 440                 445

agt aga cca tta cac ttt aat gaa gta aga gat tta aga gac act gaa 1392
Ser Arg Pro Leu His Phe Asn Glu Val Arg Asp Leu Arg Asp Thr Glu
    450                 455                 460

gtt gct act tta gta tct gtg cat aat aga aaa aat aat atc tat gct 1440
Val Ala Thr Leu Val Ser Val His Asn Arg Lys Asn Asn Ile Tyr Ala
465                 470                 475                 480

gct cat gaa aat ggt act atg att cat ttt gcg ccg gaa ggt tat aca 1488
Ala His Glu Asn Gly Thr Met Ile His Phe Ala Pro Glu Gly Tyr Thr
                485                 490                 495

ggt ttc aca ata tca cca ata tat gca act caa gta aat aat caa aca 1536
Gly Phe Thr Ile Ser Pro Ile Tyr Ala Thr Gln Val Asn Asn Gln Thr
            500                 505                 510

cga acg ttt att tct gaa aaa ttc gga aat caa ggt gat tcc ttg aga 1584
Arg Thr Phe Ile Ser Glu Lys Phe Gly Asn Gln Gly Asp Ser Leu Arg
        515                 520                 525

ttt gaa caa act aac aca acg gct cgt tat acg ttt aga ggg aat ggt 1632
Phe Glu Gln Thr Asn Thr Thr Ala Arg Tyr Thr Phe Arg Gly Asn Gly
    530                 535                 540

aat aat tat aat ctt tat tta aga gta tcc tca caa gga aat tct act 1680
Asn Asn Tyr Asn Leu Tyr Leu Arg Val Ser Ser Gln Gly Asn Ser Thr
545                 550                 555                 560

ttt cga gtt act ata aac ggt agg gtt tat act gtt tca aat gtt aat 1728
Phe Arg Val Thr Ile Asn Gly Arg Val Tyr Thr Val Ser Asn Val Asn
                565                 570                 575

acc act aca aat aat gat ggg gtt att gat aat ggg gct cgt ttt tca 1776
Thr Thr Thr Asn Asn Asp Gly Val Ile Asp Asn Gly Ala Arg Phe Ser
            580                 585                 590

gat att cac atc ggg aat ata gtg gca agt aac aat act aat gta cca     1824
Asp Ile His Ile Gly Asn Ile Val Ala Ser Asn Asn Thr Asn Val Pro
        595                 600                 605

tta gat ata aat gtg ata ctt aac tcc ggt act caa ttt gag ctt atg     1872
Leu Asp Ile Asn Val Ile Leu Asn Ser Gly Thr Gln Phe Glu Leu Met
    610                 615                 620

aat att att ttt gtt cca act aac att cca cca ctt tat                 1911
Asn Ile Ile Phe Val Pro Thr Asn Ile Pro Pro Leu Tyr
625                 630                 635


<210> 2
<211> 637
<212> PRT
<213> Bacillus thuringiensis

<400> 2
Met Leu Leu Tyr Asn Pro Ile Phe Gln Gly Gly Phe Tyr Met Asn Asn
 1               5                  10                  15
Val Leu Asn Ser Glu Arg Thr Asn Lys Cys Asp Ala Tyr Asn Val Val
            20                  25                  30
Ala His Asp Pro Phe Ser Phe Glu His Lys Ser Leu Asp Thr Ile Gln
        35                  40                  45
Gln Glu Trp Met Glu Trp Lys Arg Thr Asp His Ser Leu Tyr Val Ser
    50                  55                  60
Pro Ile Val Gly Thr Ile Ala Ser Phe Leu Leu Lys Lys Ile Ala Gly
65                  70                  75                  80
Leu Ile Gly Lys Arg Ile Leu Ser Glu Leu Lys Asn Leu Ile Phe Pro
                85                  90                  95
Ser Gly Ser Ile Glu Ser Met Gln Asp Ile Leu Arg Gly Ala Glu Gln
            100                 105                 110
Phe Leu Asn Gln Arg Leu Asp Ala Asp Thr Phe Ala Arg Val Glu Ala
        115                 120                 125
Glu Leu Ile Gly Leu Gln Ala Asn Val Glu Glu Phe Asn Gln Gln Val
    130                 135                 140
Asp Asn Phe Leu Asn Pro Asn Gln Asn Pro Val Pro Leu Ala Ile Ile
145                 150                 155                 160
Asp Ser Val Asn Thr Met Gln Gln Leu Phe Leu Ser Arg Leu Pro Gln
                165                 170                 175
Phe Gln Ile Gln Arg Tyr Gln Leu Leu Leu Leu Pro Leu Phe Ala Gln
            180                 185                 190
Ala Ala Asn Leu His Leu Thr Phe Ile Arg Asp Val Ile Leu Asn Ala
        195                 200                 205
Asp Glu Trp Gly Ile Pro Ala Ala Thr Val Arg Thr Tyr Arg Glu His
    210                 215                 220
Leu Lys Arg Tyr Thr Arg Asp Tyr Ser Asn Tyr Cys Ile Asn Thr Tyr
225                 230                 235                 240
Gln Thr Ala Phe Arg Gly Leu Asn Thr Arg Leu His Asp Met Leu Glu
                245                 250                 255
Phe Arg Thr Phe Met Phe Leu Asn Val Leu Asp Tyr Val Ser Ile Trp
            260                 265                 270
Ser Leu Phe Lys Tyr Gln Ser Leu Met Val Thr Ser Ser Ala Asn Leu
        275                 280                 285
Tyr Ala Ser Gly Ser Gly Ser Asn Gln Pro Phe Thr Ala Gln Asp Trp
    290                 295                 300
Pro Phe Leu Tyr Ser Leu Phe Gln Val Asn Ser Asn Tyr Ile Met Ser
305                 310                 315                 320
Asn Phe Gly Gly Asn Arg Glu Thr Ala Ser Phe Gly Val Pro Ile Leu
                325                 330                 335
Gly Gly Phe Ile Ile Asn Phe Leu Leu Ser Phe Arg Val Asn Tyr Thr

340                 345                 350
Gly Gly Val Ser Ser Gly Leu Leu Gly Val Glu Gly Ile Ser Asn Asn
        355                 360                 365
Phe Asn Cys Asn Ser Ser Leu Ser Thr Pro Val Val Arg Ser Trp Leu
    370                 375                 380
Asp Ser Gly Val Tyr Arg Gly Asp Leu Gln His Asn Trp Arg Thr Asp
385                 390                 395                 400
Ile Phe Met Arg Thr Asn Ile Val Pro Cys Gly Ala Phe Leu Leu Ser
                405                 410                 415
Leu Ala Met Phe Pro Asp Val Lys Ser Asn Tyr Phe Pro Asp Tyr Phe
            420                 425                 430
Ile Arg Asn Ile Ser Gly Ile Ile Arg Asn Ile Asp Asn Met Asn Leu
        435                 440                 445
Ser Arg Pro Leu His Phe Asn Glu Val Arg Asp Leu Arg Asp Thr Glu
    450                 455                 460
Val Ala Thr Leu Val Ser Val His Asn Arg Lys Asn Asn Ile Tyr Ala
465                 470                 475                 480
Ala His Glu Asn Gly Thr Met Ile His Phe Ala Pro Glu Gly Tyr Thr
                485                 490                 495
Gly Phe Thr Ile Ser Pro Ile Tyr Ala Thr Gln Val Asn Asn Gln Thr
            500                 505                 510
Arg Thr Phe Ile Ser Glu Lys Phe Gly Asn Gln Gly Asp Ser Leu Arg
        515                 520                 525
Phe Glu Gln Thr Asn Thr Thr Ala Arg Tyr Thr Phe Arg Gly Asn Gly
    530                 535                 540
Asn Asn Tyr Asn Leu Tyr Leu Arg Val Ser Ser Gln Gly Asn Ser Thr
545                 550                 555                 560
Phe Arg Val Thr Ile Asn Gly Arg Val Tyr Thr Val Ser Asn Val Asn
                565                 570                 575
Thr Thr Thr Asn Asn Asp Gly Val Ile Asp Asn Gly Ala Arg Phe Ser
            580                 585                 590
Asp Ile His Ile Gly Asn Ile Val Ala Ser Asn Asn Thr Asn Val Pro
        595                 600                 605
Leu Asp Ile Asn Val Ile Leu Asn Ser Gly Thr Gln Phe Glu Leu Met
    610                 615                 620
Asn Ile Ile Phe Val Pro Thr Asn Ile Pro Pro Leu Tyr
625                 630                 635


<210> 3
<211> 1875
<212> DNA
<213> Artificial Sequence

<220>
<223> Synthetic sequence encoding AXMI-066 (optaxmi-066)

<221> CDS
<222> (1)...(1875)

<400> 3
atg aac aat gtg ctc aac agc gag agg acc aac aaa tgt gat gcc tac    48
Met Asn Asn Val Leu Asn Ser Glu Arg Thr Asn Lys Cys Asp Ala Tyr
 1               5                  10                  15

aat gtg gtg gct cat gat ccc ttc agc ttc gag cac aag agc ttg gac    96
Asn Val Val Ala His Asp Pro Phe Ser Phe Glu His Lys Ser Leu Asp
            20                  25                  30

acc atc cag caa gaa tgg atg gag tgg aag agg aca gat cac agc ctc    144
Thr Ile Gln Gln Glu Trp Met Glu Trp Lys Arg Thr Asp His Ser Leu
        35                  40                  45

tat gtt tca cca att gtt ggc acc atc gcc agc ttc ctg ctg aag aag 192
Tyr Val Ser Pro Ile Val Gly Thr Ile Ala Ser Phe Leu Leu Lys Lys
    50                  55                  60

atc gcc ggc ctc atc ggc aag agg att ctt tct gag ctg aag aac ctc 240
Ile Ala Gly Leu Ile Gly Lys Arg Ile Leu Ser Glu Leu Lys Asn Leu
65                  70                  75                  80

atc ttc cca tca gga agc atc gag agc atg caa gac atc ctc cgc ggc 288
Ile Phe Pro Ser Gly Ser Ile Glu Ser Met Gln Asp Ile Leu Arg Gly
                85                  90                  95

gcc gag cag ttc ctc aac cag agg ctg gat gct gac acc ttc gcc agg 336
Ala Glu Gln Phe Leu Asn Gln Arg Leu Asp Ala Asp Thr Phe Ala Arg
            100                 105                 110

gtg gaa gct gag ctc atc ggc ctt caa gca aat gtg gag gag ttc aac 384
Val Glu Ala Glu Leu Ile Gly Leu Gln Ala Asn Val Glu Glu Phe Asn
        115                 120                 125

cag cag gtg gac aac ttc ctc aac ccc aac cag aac ccg gtg ccg ctg 432
Gln Gln Val Asp Asn Phe Leu Asn Pro Asn Gln Asn Pro Val Pro Leu
    130                 135                 140

gcc atc att gat tct gtg aac acc atg cag cag ctc ttc ctc tca agg 480
Ala Ile Ile Asp Ser Val Asn Thr Met Gln Gln Leu Phe Leu Ser Arg
145                 150                 155                 160

ctg ccg cag ttc cag atc caa aga tac cag ctg ctg ctg ctg ccg ctc 528
Leu Pro Gln Phe Gln Ile Gln Arg Tyr Gln Leu Leu Leu Leu Pro Leu
                165                 170                 175

ttt gct caa gct gcc aac ctc cac ctc acc ttc atc aga gat gtc atc 576
Phe Ala Gln Ala Ala Asn Leu His Leu Thr Phe Ile Arg Asp Val Ile
            180                 185                 190

ctc aat gct gat gaa tgg ggc atc ccg gcg gcg acg gtg agg acc tac 624
Leu Asn Ala Asp Glu Trp Gly Ile Pro Ala Ala Thr Val Arg Thr Tyr
        195                 200                 205

agg gag cac ctc aag aga tac aca agg gac tac tca aac tac tgc atc 672
Arg Glu His Leu Lys Arg Tyr Thr Arg Asp Tyr Ser Asn Tyr Cys Ile
    210                 215                 220

aac acc tac cag acg gcc ttc cgc ggc ctc aac aca agg cta cat gac 720
Asn Thr Tyr Gln Thr Ala Phe Arg Gly Leu Asn Thr Arg Leu His Asp
225                 230                 235                 240

atg ctg gag ttc aga acc ttc atg ttc ctc aat gtg ctg gac tat gtg 768
Met Leu Glu Phe Arg Thr Phe Met Phe Leu Asn Val Leu Asp Tyr Val
                245                 250                 255

agc atc tgg agc ctc ttc aag tac cag agc ttg atg gtg aca agc tcc 816
Ser Ile Trp Ser Leu Phe Lys Tyr Gln Ser Leu Met Val Thr Ser Ser
            260                 265                 270

gcc aac ctc tat gct tct gga agc ggc agc aac cag ccc ttc acc gct 864
Ala Asn Leu Tyr Ala Ser Gly Ser Gly Ser Asn Gln Pro Phe Thr Ala
        275                 280                 285

caa gat tgg ccc ttc ctc tac agc ctc ttc caa gtg aac tca aac tac 912
Gln Asp Trp Pro Phe Leu Tyr Ser Leu Phe Gln Val Asn Ser Asn Tyr
    290                 295                 300

atc atg agc aac ttc ggc ggc aac cgt gaa act gct tcc ttc ggc gtg 960
Ile Met Ser Asn Phe Gly Gly Asn Arg Glu Thr Ala Ser Phe Gly Val
305                 310                 315                 320

ccc atc ctc ggc ggc ttc atc atc aac ttc ctg ctg agc ttc cgc gtc 1008
Pro Ile Leu Gly Gly Phe Ile Ile Asn Phe Leu Leu Ser Phe Arg Val
                325                 330                 335

aac tac act gga gga gtt tct tct ggg ctg ctg gga gtt gaa ggc atc 1056
Asn Tyr Thr Gly Gly Val Ser Ser Gly Leu Leu Gly Val Glu Gly Ile
            340                 345                 350

agc aac aac ttc aac tgc aac tca agc ctc agc acg ccg gtg gtg agg 1104
Ser Asn Asn Phe Asn Cys Asn Ser Ser Leu Ser Thr Pro Val Val Arg
        355                 360                 365

agc tgg ctg gac agc ggc gtc tac aga gga gat ctt cag cac aac tgg 1152
Ser Trp Leu Asp Ser Gly Val Tyr Arg Gly Asp Leu Gln His Asn Trp
    370                 375                 380

agg acg gac atc ttc atg agg acc aac atc gtg cca tgc ggc gcc ttc 1200
Arg Thr Asp Ile Phe Met Arg Thr Asn Ile Val Pro Cys Gly Ala Phe
385                 390                 395                 400

ctc ctc tca ttg gcc atg ttc cct gat gtg aag agc aac tac ttc ccg 1248
Leu Leu Ser Leu Ala Met Phe Pro Asp Val Lys Ser Asn Tyr Phe Pro
                405                 410                 415

gac tac ttc atc agg aac atc agc ggc atc atc agg aac atc gac aac 1296
Asp Tyr Phe Ile Arg Asn Ile Ser Gly Ile Ile Arg Asn Ile Asp Asn
            420                 425                 430

atg aac ctc tca agg ccg ctg cac ttc aac gag gtg aga gat ctt cga 1344
Met Asn Leu Ser Arg Pro Leu His Phe Asn Glu Val Arg Asp Leu Arg
        435                 440                 445

gac acc gag gtg gcc acc ttg gtg agc gtc cac aac agg aag aac aac 1392
Asp Thr Glu Val Ala Thr Leu Val Ser Val His Asn Arg Lys Asn Asn
    450                 455                 460

atc tat gct gct cat gaa aat ggc acc atg atc cac ttc gcg cca gaa 1440
Ile Tyr Ala Ala His Glu Asn Gly Thr Met Ile His Phe Ala Pro Glu
465                 470                 475                 480

ggc tac acc ggc ttc acc atc tca cca att tat gca act caa gtc aac 1488
Gly Tyr Thr Gly Phe Thr Ile Ser Pro Ile Tyr Ala Thr Gln Val Asn
                485                 490                 495

aac caa aca agg acc ttc atc tcc gag aag ttt gga aat caa gga gat 1536
Asn Gln Thr Arg Thr Phe Ile Ser Glu Lys Phe Gly Asn Gln Gly Asp
            500                 505                 510

tct ttg aga ttt gag cag acc aac acc acg gcg cgc tac acc ttc aga 1584
Ser Leu Arg Phe Glu Gln Thr Asn Thr Thr Ala Arg Tyr Thr Phe Arg
        515                 520                 525

gga aat ggc aac aac tac aac ctc tac ctc cgc gtc agc agc caa ggc 1632
Gly Asn Gly Asn Asn Tyr Asn Leu Tyr Leu Arg Val Ser Ser Gln Gly
    530                 535                 540

aac agc acc ttc cgc gtg acc atc aat ggc cgc gtc tac acc gtc tca 1680
Asn Ser Thr Phe Arg Val Thr Ile Asn Gly Arg Val Tyr Thr Val Ser

545                 550                 555                 560

aat gtc aac acc acc acc aac aat gat ggc gtc atc gac aat gga gca    1728
Asn Val Asn Thr Thr Thr Asn Asn Asp Gly Val Ile Asp Asn Gly Ala
                565                 570                 575

aga ttc tcc gac atc cac atc ggc aac atc gtc gcc agc aac aac acc    1776
Arg Phe Ser Asp Ile His Ile Gly Asn Ile Val Ala Ser Asn Asn Thr
            580                 585                 590

aac gtg ccg ctg gac atc aat gtc atc ctc aac agc ggc acc caa ttt    1824
Asn Val Pro Leu Asp Ile Asn Val Ile Leu Asn Ser Gly Thr Gln Phe
        595                 600                 605

gag ctg atg aac atc atc ttc gtg cca acc aac atc ccg ccg ctc tac    1872
Glu Leu Met Asn Ile Ile Phe Val Pro Thr Asn Ile Pro Pro Leu Tyr
    610                 615                 620

tag                                                                1875
 *

<210> 4
<211> 1902
<212> DNA
<213> Bacillus thuringiensis

<220>
<221> CDS
<222> (1)...(1902)

<400> 4
atg aat aat gta tta aat aac gga aga act act aat tgt gat gcg tat    48
Met Asn Asn Val Leu Asn Asn Gly Arg Thr Thr Asn Cys Asp Ala Tyr
 1               5                  10                  15

aat gta gtg gcc cat gat cca ttt agt ttt gag cat aaa tca tta gat    96
Asn Val Val Ala His Asp Pro Phe Ser Phe Glu His Lys Ser Leu Asp
            20                  25                  30

acc atc cga aaa gaa tgg atg gag tgg aaa aga aca gat cat agt tta    144
Thr Ile Arg Lys Glu Trp Met Glu Trp Lys Arg Thr Asp His Ser Leu
        35                  40                  45

tat gta gct cct gta gtc gga act gtt tct agc ttt ctg cta aag aag    192
Tyr Val Ala Pro Val Val Gly Thr Val Ser Ser Phe Leu Leu Lys Lys
    50                  55                  60

gtg ggg agt ctt att gga aaa agg ata ttg agt gaa tta tgg ggg tta    240
Val Gly Ser Leu Ile Gly Lys Arg Ile Leu Ser Glu Leu Trp Gly Leu
65                  70                  75                  80

ata ttt cct agt ggt agc aca aat cta atg caa gat att tta agg gag    288
Ile Phe Pro Ser Gly Ser Thr Asn Leu Met Gln Asp Ile Leu Arg Glu
                85                  90                  95

aca gaa caa ttc cta aat caa aga ctt aat aca gac act ctt gct cgt    336
Thr Glu Gln Phe Leu Asn Gln Arg Leu Asn Thr Asp Thr Leu Ala Arg
            100                 105                 110

gta aat gcg gaa ttg aca ggg ctg caa gcg aat ata agg gag ttt aat    384
Val Asn Ala Glu Leu Thr Gly Leu Gln Ala Asn Ile Arg Glu Phe Asn

115                 120                 125

caa caa gta gat aat ttt tta aat cct act caa aac cct gtt cct tta     432
Gln Gln Val Asp Asn Phe Leu Asn Pro Thr Gln Asn Pro Val Pro Leu
    130                 135                 140

tca ata act tct tca gtt aat aca atg cag caa tta ttt cta aat aga     480
Ser Ile Thr Ser Ser Val Asn Thr Met Gln Gln Leu Phe Leu Asn Arg
145                 150                 155                 160

tta ccc cag ttc cag ata caa gga tac caa ctg tta tta tta cct tta     528
Leu Pro Gln Phe Gln Ile Gln Gly Tyr Gln Leu Leu Leu Leu Pro Leu
                165                 170                 175

ttt gca cag gca gcc aat atg cat ctt tct ttt att aga gat gtt att     576
Phe Ala Gln Ala Ala Asn Met His Leu Ser Phe Ile Arg Asp Val Ile
            180                 185                 190

ctt aat gca gat gaa tgg gga att tca gca gca aca cta cgt acg tat     624
Leu Asn Ala Asp Glu Trp Gly Ile Ser Ala Ala Thr Leu Arg Thr Tyr
        195                 200                 205

cga gac tac ctg aga aat tat aca aga gat tat tct aat tat tgt ata     672
Arg Asp Tyr Leu Arg Asn Tyr Thr Arg Asp Tyr Ser Asn Tyr Cys Ile
    210                 215                 220

aat acg tat caa act gcg ttt aga ggg tta aac acc cgt tta cac gat     720
Asn Thr Tyr Gln Thr Ala Phe Arg Gly Leu Asn Thr Arg Leu His Asp
225                 230                 235                 240

atg tta gaa ttt aga aca tat atg ttt tta aat gta ttt gaa tat gta     768
Met Leu Glu Phe Arg Thr Tyr Met Phe Leu Asn Val Phe Glu Tyr Val
                245                 250                 255

tcc att tgg tca ttg ttt aaa tat cag agt ctt atg gta tct tct ggc     816
Ser Ile Trp Ser Leu Phe Lys Tyr Gln Ser Leu Met Val Ser Ser Gly
            260                 265                 270

gct aat tta tat gct agt ggt agt gga cca cag cag acc caa tca ttt     864
Ala Asn Leu Tyr Ala Ser Gly Ser Gly Pro Gln Gln Thr Gln Ser Phe
        275                 280                 285

att tca caa gac tgg cca ttt tta tat tct ctt ttc caa gtt aat tca     912
Ile Ser Gln Asp Trp Pro Phe Leu Tyr Ser Leu Phe Gln Val Asn Ser
    290                 295                 300

aat tat gtg tta aat ggc ttt agt ggc gct agg aat act att aga ttc     960
Asn Tyr Val Leu Asn Gly Phe Ser Gly Ala Arg Asn Thr Ile Arg Phe
305                 310                 315                 320

cca gct ggt ggt ggt tta tta cca cct ggt gtt act aca act cac gca     1008
Pro Ala Gly Gly Gly Leu Leu Pro Pro Gly Val Thr Thr Thr His Ala
                325                 330                 335

ttg ctt gct gca agg gtc aat tac agt gga gga gtt tcg tct ggt tat     1056
Leu Leu Ala Ala Arg Val Asn Tyr Ser Gly Gly Val Ser Ser Gly Tyr
            340                 345                 350

ata ggc gct gtg ttt aat caa aat ttt aat tgt agc aca ctt ctc cca     1104
Ile Gly Ala Val Phe Asn Gln Asn Phe Asn Cys Ser Thr Leu Leu Pro
        355                 360                 365

cct ttg tta aca cca ttt gtt agg agt tgg cta gat tca ggt aca gat     1152

Pro Leu Leu Thr Pro Phe Val Arg Ser Trp Leu Asp Ser Gly Thr Asp
    370                 375                 380

cgg gag ggc gtt acc acc gtt aca aat tgg caa aca gaa tcc ttt aag     1200
Arg Glu Gly Val Thr Thr Val Thr Asn Trp Gln Thr Glu Ser Phe Lys
385                 390                 395                 400

aca act tta ggt tta agg tgt ggt gct ttt aca ccc cgt ggt aat tca     1248
Thr Thr Leu Gly Leu Arg Cys Gly Ala Phe Thr Pro Arg Gly Asn Ser
                405                 410                 415

aac tat ttc cca gat tat ttt atc cgt aat att tct ggc gtt cct tta     1296
Asn Tyr Phe Pro Asp Tyr Phe Ile Arg Asn Ile Ser Gly Val Pro Leu
            420                 425                 430

gtt gtt aga aac gaa gat tta aga aga ccg tta cac tat aat gaa ata     1344
Val Val Arg Asn Glu Asp Leu Arg Arg Pro Leu His Tyr Asn Glu Ile
        435                 440                 445

aga aat ata gaa agt ccc tca gga aca cct ggt gga tta cga gct tat     1392
Arg Asn Ile Glu Ser Pro Ser Gly Thr Pro Gly Gly Leu Arg Ala Tyr
    450                 455                 460

atg gta tct gtg cat aac aga aaa aat aat atc tat gcc gct cat gaa     1440
Met Val Ser Val His Asn Arg Lys Asn Asn Ile Tyr Ala Ala His Glu
465                 470                 475                 480

aat ggt act atg att cat ttg gca ccg gaa gat tat aca gga ttt act     1488
Asn Gly Thr Met Ile His Leu Ala Pro Glu Asp Tyr Thr Gly Phe Thr
                485                 490                 495

atg ttg ccg ata cat gca act caa gtg aat aat caa acg cga aca ttt     1536
Met Leu Pro Ile His Ala Thr Gln Val Asn Asn Gln Thr Arg Thr Phe
            500                 505                 510

att tct gaa aaa ttt gga aat caa ggt gat tcc tta aga ttt gaa caa     1584
Ile Ser Glu Lys Phe Gly Asn Gln Gly Asp Ser Leu Arg Phe Glu Gln
        515                 520                 525

agc gac acg aca gct cgt tat aca ctt aga ggg aat gga aat agt tac     1632
Ser Asp Thr Thr Ala Arg Tyr Thr Leu Arg Gly Asn Gly Asn Ser Tyr
    530                 535                 540

aat ctt tat tta aga gta tct tct cta gga aat tcc act att cga gtt     1680
Asn Leu Tyr Leu Arg Val Ser Ser Leu Gly Asn Ser Thr Ile Arg Val
545                 550                 555                 560

act ata aac gga aga gtt tat act gtt cca aat gtt aat aca aat ata     1728
Thr Ile Asn Gly Arg Val Tyr Thr Val Pro Asn Val Asn Thr Asn Ile
                565                 570                 575

aat aac gat gga gtc att gat aat gga gct cgt ttt tca gat att aat     1776
Asn Asn Asp Gly Val Ile Asp Asn Gly Ala Arg Phe Ser Asp Ile Asn
            580                 585                 590

atc ggt aat gta gta gca agt gat aat act aat gta ccg tta gat ata     1824
Ile Gly Asn Val Val Ala Ser Asp Asn Thr Asn Val Pro Leu Asp Ile
        595                 600                 605

aac gtg aca tta agt tct gga act caa ttt gag ctt atg aat att atg     1872
Asn Val Thr Leu Ser Ser Gly Thr Gln Phe Glu Leu Met Asn Ile Met
    610                 615                 620

ttt gtt cca act aat ctt cca cca ata tat                                    1902
Phe Val Pro Thr Asn Leu Pro Pro Ile Tyr
625                 630

<210> 5
<211> 634
<212> PRT
<213> Bacillus thuringiensis

<400> 5
Met Asn Asn Val Leu Asn Asn Gly Arg Thr Thr Asn Cys Asp Ala Tyr
1               5                   10                  15
Asn Val Val Ala His Asp Pro Phe Ser Phe Glu His Lys Ser Leu Asp
            20                  25                  30
Thr Ile Arg Lys Glu Trp Met Glu Trp Lys Arg Thr Asp His Ser Leu
        35                  40                  45
Tyr Val Ala Pro Val Val Gly Thr Val Ser Ser Phe Leu Leu Lys Lys
    50                  55                  60
Val Gly Ser Leu Ile Gly Lys Arg Ile Leu Ser Glu Leu Trp Gly Leu
65                  70                  75                  80
Ile Phe Pro Ser Gly Ser Thr Asn Leu Met Gln Asp Ile Leu Arg Glu
                85                  90                  95
Thr Glu Gln Phe Leu Asn Gln Arg Leu Asn Thr Asp Thr Leu Ala Arg
            100                 105                 110
Val Asn Ala Glu Leu Thr Gly Leu Gln Ala Asn Ile Arg Glu Phe Asn
        115                 120                 125
Gln Gln Val Asp Asn Phe Leu Asn Pro Thr Gln Asn Pro Val Pro Leu
    130                 135                 140
Ser Ile Thr Ser Ser Val Asn Thr Met Gln Gln Leu Phe Leu Asn Arg
145                 150                 155                 160
Leu Pro Gln Phe Gln Ile Gln Gly Tyr Gln Leu Leu Leu Leu Pro Leu
                165                 170                 175
Phe Ala Gln Ala Ala Asn Met His Leu Ser Phe Ile Arg Asp Val Ile
            180                 185                 190
Leu Asn Ala Asp Glu Trp Gly Ile Ser Ala Ala Thr Leu Arg Thr Tyr
        195                 200                 205
Arg Asp Tyr Leu Arg Asn Tyr Thr Arg Asp Tyr Ser Asn Tyr Cys Ile
    210                 215                 220
Asn Thr Tyr Gln Thr Ala Phe Arg Gly Leu Asn Thr Arg Leu His Asp
225                 230                 235                 240
Met Leu Glu Phe Arg Thr Tyr Met Phe Leu Asn Val Phe Glu Tyr Val
                245                 250                 255
Ser Ile Trp Ser Leu Phe Lys Tyr Gln Ser Leu Met Val Ser Ser Gly
            260                 265                 270
Ala Asn Leu Tyr Ala Ser Gly Ser Gly Pro Gln Gln Thr Gln Ser Phe
        275                 280                 285
Ile Ser Gln Asp Trp Pro Phe Leu Tyr Ser Leu Phe Gln Val Asn Ser
    290                 295                 300
Asn Tyr Val Leu Asn Gly Phe Ser Gly Ala Arg Asn Thr Ile Arg Phe
305                 310                 315                 320
Pro Ala Gly Gly Gly Leu Leu Pro Pro Gly Val Thr Thr Thr His Ala
                325                 330                 335
Leu Leu Ala Ala Arg Val Asn Tyr Ser Gly Gly Val Ser Ser Gly Tyr
            340                 345                 350
Ile Gly Ala Val Phe Asn Gln Asn Phe Asn Cys Ser Thr Leu Leu Pro
        355                 360                 365
Pro Leu Leu Thr Pro Phe Val Arg Ser Trp Leu Asp Ser Gly Thr Asp
    370                 375                 380
Arg Glu Gly Val Thr Thr Val Thr Asn Trp Gln Thr Glu Ser Phe Lys
385                 390                 395                 400
Thr Thr Leu Gly Leu Arg Cys Gly Ala Phe Thr Pro Arg Gly Asn Ser
                405                 410                 415

Asn Tyr Phe Pro Asp Tyr Phe Ile Arg Asn Ile Ser Gly Val Pro Leu
            420                 425                 430
Val Val Arg Asn Glu Asp Leu Arg Arg Pro Leu His Tyr Asn Glu Ile
        435                 440                 445
Arg Asn Ile Glu Ser Pro Ser Gly Thr Pro Gly Gly Leu Arg Ala Tyr
    450                 455                 460
Met Val Ser Val His Asn Arg Lys Asn Asn Ile Tyr Ala Ala His Glu
465                 470                 475                 480
Asn Gly Thr Met Ile His Leu Ala Pro Glu Asp Tyr Thr Gly Phe Thr
                485                 490                 495
Met Leu Pro Ile His Ala Thr Gln Val Asn Asn Gln Thr Arg Thr Phe
            500                 505                 510
Ile Ser Glu Lys Phe Gly Asn Gln Gly Asp Ser Leu Arg Phe Glu Gln
        515                 520                 525
Ser Asp Thr Thr Ala Arg Tyr Thr Leu Arg Gly Asn Gly Asn Ser Tyr
    530                 535                 540
Asn Leu Tyr Leu Arg Val Ser Ser Leu Gly Asn Ser Thr Ile Arg Val
545                 550                 555                 560
Thr Ile Asn Gly Arg Val Tyr Thr Val Pro Asn Val Asn Thr Asn Ile
                565                 570                 575
Asn Asn Asp Gly Val Ile Asp Asn Gly Ala Arg Phe Ser Asp Ile Asn
            580                 585                 590
Ile Gly Asn Val Val Ala Ser Asp Asn Thr Asn Val Pro Leu Asp Ile
        595                 600                 605
Asn Val Thr Leu Ser Ser Gly Thr Gln Phe Glu Leu Met Asn Ile Met
    610                 615                 620
Phe Val Pro Thr Asn Leu Pro Pro Ile Tyr
625                 630


<210> 6
<211> 1902
<212> DNA
<213> Artificial Sequence

<220>
<223> Synthetic sequence encoding AXMI-076 (optaxmi-076)

<221> CDS
<222> (1)...(1902)

<400> 6
atg aac aat gtg ctc aac aat gga aga aca aca aat tgt gat gcc tac      48
Met Asn Asn Val Leu Asn Asn Gly Arg Thr Thr Asn Cys Asp Ala Tyr
 1               5                  10                  15

aat gtg gtg gct cat gat ccc ttc agc ttc gag cac aag agc ttg gac      96
Asn Val Val Ala His Asp Pro Phe Ser Phe Glu His Lys Ser Leu Asp
            20                  25                  30

acc atc agg aag gag tgg atg gaa tgg aag agg aca gat cat tct ctt     144
Thr Ile Arg Lys Glu Trp Met Glu Trp Lys Arg Thr Asp His Ser Leu
        35                  40                  45

tat gtg gcg ccg gtg gtg ggc acc gtc tca tcc ttc ctg ctg aag aag     192
Tyr Val Ala Pro Val Val Gly Thr Val Ser Ser Phe Leu Leu Lys Lys
    50                  55                  60

gtg ggc agc ctc atc ggc aag agg att ctt tct gag ctc tgg ggc ctc     240
Val Gly Ser Leu Ile Gly Lys Arg Ile Leu Ser Glu Leu Trp Gly Leu
 65                  70                  75                  80

atc ttc cca agt gga tca aca aat ttg atg caa gac atc ctc cga gaa     288

Ile Phe Pro Ser Gly Ser Thr Asn Leu Met Gln Asp Ile Leu Arg Glu
                85                  90                  95

aca gag cag ttc ctc aat caa agg ctc aac acc gac acc ttg gca agg     336
Thr Glu Gln Phe Leu Asn Gln Arg Leu Asn Thr Asp Thr Leu Ala Arg
            100                 105                 110

gtg aat gct gag ctg aca ggg cta caa gcc aac atc agg gag ttc aac     384
Val Asn Ala Glu Leu Thr Gly Leu Gln Ala Asn Ile Arg Glu Phe Asn
        115                 120                 125

cag cag gtg gac aac ttc ctc aac cca act caa aat cca gtg ccg ctg     432
Gln Gln Val Asp Asn Phe Leu Asn Pro Thr Gln Asn Pro Val Pro Leu
    130                 135                 140

agc atc acc tcc tct gtt aac acc atg cag cag ctc ttc ctc aac agg     480
Ser Ile Thr Ser Ser Val Asn Thr Met Gln Gln Leu Phe Leu Asn Arg
145                 150                 155                 160

ctg ccg cag ttc cag atc caa ggc tac cag ctg ctg ctg ctg ccg cta     528
Leu Pro Gln Phe Gln Ile Gln Gly Tyr Gln Leu Leu Leu Leu Pro Leu
                165                 170                 175

ttt gct caa gct gcc aac atg cac ctc agc ttc atc aga gat gtc atc     576
Phe Ala Gln Ala Ala Asn Met His Leu Ser Phe Ile Arg Asp Val Ile
            180                 185                 190

ctc aat gct gat gaa tgg ggc atc tcg gcg gcg acg ctg agg acc tac     624
Leu Asn Ala Asp Glu Trp Gly Ile Ser Ala Ala Thr Leu Arg Thr Tyr
        195                 200                 205

aga gat tat ttg agg aac tac aca agg gac tac tca aac tac tgc atc     672
Arg Asp Tyr Leu Arg Asn Tyr Thr Arg Asp Tyr Ser Asn Tyr Cys Ile
    210                 215                 220

aac acc tac cag acg gcc ttc cgc ggc ctc aac aca agg ctg cat gac     720
Asn Thr Tyr Gln Thr Ala Phe Arg Gly Leu Asn Thr Arg Leu His Asp
225                 230                 235                 240

atg ctg gag ttc aga acc tac atg ttc ctc aat gtt ttt gaa tat gtt     768
Met Leu Glu Phe Arg Thr Tyr Met Phe Leu Asn Val Phe Glu Tyr Val
                245                 250                 255

tca att tgg agc ctc ttc aag tac cag agc ttg atg gtg agc agc ggc     816
Ser Ile Trp Ser Leu Phe Lys Tyr Gln Ser Leu Met Val Ser Ser Gly
            260                 265                 270

gcc aac ctc tat gct tct gga agt ggg ccg cag caa act cag agc ttc     864
Ala Asn Leu Tyr Ala Ser Gly Ser Gly Pro Gln Gln Thr Gln Ser Phe
        275                 280                 285

atc agc caa gat tgg ccc ttc ctc tac agc ctc ttc caa gtt aac agc     912
Ile Ser Gln Asp Trp Pro Phe Leu Tyr Ser Leu Phe Gln Val Asn Ser
    290                 295                 300

aac tac gtg ctg aat ggc ttc tca gga gca agg aac acc atc aga ttt     960
Asn Tyr Val Leu Asn Gly Phe Ser Gly Ala Arg Asn Thr Ile Arg Phe
305                 310                 315                 320

cct gct gga gga ggg ctg ctg ccg ccg ggc gtg aca aca act cat gct     1008
Pro Ala Gly Gly Gly Leu Leu Pro Pro Gly Val Thr Thr Thr His Ala
                325                 330                 335

ctg ctg gcg gca aga gtt aac tac tct gga gga gtt tca agc ggc tac     1056
Leu Leu Ala Ala Arg Val Asn Tyr Ser Gly Gly Val Ser Ser Gly Tyr
            340                 345                 350

atc ggc gcc gtc ttc aac cag aac ttc aac tgc tcg acg ctg ctg ccg     1104
Ile Gly Ala Val Phe Asn Gln Asn Phe Asn Cys Ser Thr Leu Leu Pro
        355                 360                 365

ccg ctg ctg acg cca ttt gtg agg agc tgg ctg gac agc ggc acc gac     1152
Pro Leu Leu Thr Pro Phe Val Arg Ser Trp Leu Asp Ser Gly Thr Asp
    370                 375                 380

aga gaa gga gtc acc acc gtc acc aac tgg caa act gag agc ttc aag     1200
Arg Glu Gly Val Thr Thr Val Thr Asn Trp Gln Thr Glu Ser Phe Lys
385                 390                 395                 400

acg acg ctg ggc ctc cgc tgc ggc gcc ttc acg ccg cgc ggc aac tca     1248
Thr Thr Leu Gly Leu Arg Cys Gly Ala Phe Thr Pro Arg Gly Asn Ser
                405                 410                 415

aac tac ttc ccg gac tac ttc atc agg aac atc agc ggc gtg ccg ctg     1296
Asn Tyr Phe Pro Asp Tyr Phe Ile Arg Asn Ile Ser Gly Val Pro Leu
            420                 425                 430

gtg gtg agg aat gaa gat ttg agg agg ccg ctg cac tac aat gag atc     1344
Val Val Arg Asn Glu Asp Leu Arg Arg Pro Leu His Tyr Asn Glu Ile
        435                 440                 445

agg aac atc gag agc cca tca gga act cct gga ggc ctc cgc gcc tac     1392
Arg Asn Ile Glu Ser Pro Ser Gly Thr Pro Gly Gly Leu Arg Ala Tyr
    450                 455                 460

atg gtt tct gtt cac aac agg aag aac aac atc tat gct gct cat gaa     1440
Met Val Ser Val His Asn Arg Lys Asn Asn Ile Tyr Ala Ala His Glu
465                 470                 475                 480

aat ggc acc atg atc cac ctg gcg ccg gag gac tac acc ggc ttc acc     1488
Asn Gly Thr Met Ile His Leu Ala Pro Glu Asp Tyr Thr Gly Phe Thr
                485                 490                 495

atg cta ccc atc cat gca act caa gtc aac aac caa aca agg acc ttc     1536
Met Leu Pro Ile His Ala Thr Gln Val Asn Asn Gln Thr Arg Thr Phe
            500                 505                 510

atc tcc gag aag ttt gga aat caa gga gat tct ttg aga ttt gag cag     1584
Ile Ser Glu Lys Phe Gly Asn Gln Gly Asp Ser Leu Arg Phe Glu Gln
        515                 520                 525

agc gac acc acg gcg cgc tac acc ttg aga gga aat ggc aac agc tac     1632
Ser Asp Thr Thr Ala Arg Tyr Thr Leu Arg Gly Asn Gly Asn Ser Tyr
    530                 535                 540

aac ctc tac ctc cgc gtc agc agc ctc ggc aac tca acc atc agg gtg     1680
Asn Leu Tyr Leu Arg Val Ser Ser Leu Gly Asn Ser Thr Ile Arg Val
545                 550                 555                 560

acc atc aat ggc cgc gtc tac acc gtg cca aat gtc aac acc aac atc     1728
Thr Ile Asn Gly Arg Val Tyr Thr Val Pro Asn Val Asn Thr Asn Ile
                565                 570                 575

aac aat gat ggc gtc atc gac aat gga gca aga ttc tcc gac atc aac     1776
Asn Asn Asp Gly Val Ile Asp Asn Gly Ala Arg Phe Ser Asp Ile Asn
            580                 585                 590

atc ggc aat gtg gtg gcc tcc gac aac acc aac gtg ccg ctg gac atc    1824
Ile Gly Asn Val Val Ala Ser Asp Asn Thr Asn Val Pro Leu Asp Ile
        595                 600                 605

aat gtc acc ttg agc tca gga act caa ttt gag ctg atg aac atc atg    1872
Asn Val Thr Leu Ser Ser Gly Thr Gln Phe Glu Leu Met Asn Ile Met
    610                 615                 620

ttc gtg cca aca aat ctt cct ccc atc tac                            1902
Phe Val Pro Thr Asn Leu Pro Pro Ile Tyr
625                 630


<210> 7
<211> 4
<212> PRT
<213> Artificial Sequence

<220>
<223> Endoplasmic reticulum targeting sequence

<400> 7
Lys Asp Glu Leu
1


<210> 8
<211> 1990
<212> DNA
<213> Bacillus thuringiensis

<400> 8
agacaggttc ttaaacatac ttgttattat caagagagta ttgtctcttt aatgttatta 60
tataacccaa tatttcaagg aggattttat atgaataatg tattgaatag cgaaagaact 120
aataagtgtg atgcgtataa cgtagtggcc catgatccat ttagttttga gcataaatca 180
ttagatacca tacagcaaga atggatggag tggaaaagaa ccgatcatag tttatatgta 240
tctcctattg tgggaactat agctagtttt ctgctaaaga aaatagcggg gcttatagga 300
aaaagaatat taagtgagtt aaagaattta atttttccta gtggtagtat agaatcaatg 360
caagatattt taagaggggc agaacaattc ctaaatcaaa gacttgatgc agacaccttt 420
gctcgggtag aggcagaatt gatagggctt caagcaaatg tagaggaatt taatcaacaa 480
gtggacaatt ttttaaaccc aaatcaaaac cctgttcctt tagcaataat tgattcggtt 540
aatacaatgc aacaattatt cctaagtaga ttaccccagt tccagataca acgctatcag 600
ctattattat tacctttatt tgcacaagca gccaatttac accttacctt tattagagat 660
gttattctta atgcagatga atggggaata ccagcagcaa cagtgcgcac atatagagag 720
cacctaaaaa gatatacacg cgattattcc aattattgta taaacacgta ccaaactgct 780
ttccgaggtt taaacactcg tttacatgat atgttagagt ttagaacatt tatgttttta 840
aatgtattag actatgtatc tatctggtcg ttgtttaaat atcaaagtct gatggttact 900
tcaagtgcta atttatatgc ttcgggaagt ggtagtaatc aaccttttac tgcacaagac 960
tggccatttt tatattctct tttccaagtg aattcaaatt atataatgtc taattttggt 1020
ggtaaccgag agactgctag ttttggtgtt cctattctgg ggggattcat aataaatttt 1080
ttacttagtt ttagggttaa ttatactgga ggagtttcat ctggtctcct aggtgttgaa 1140
ggaatttcaa acaactttaa ttgcaactcc tctttatcaa caccagttgt aagaagttgg 1200
ctagattcag gtgtatatcg aggtgacctg caacacaatt ggcgaacaga catctttatg 1260
aggactaata ttgtaccttg tggtgctttt ctattatctc ttgctatgtt tccagatgtt 1320
aaaagtaatt attttcctga ttatttcatt cgtaacattt ccggaattat tcgaaatatt 1380
gataacatga atttgagtag accattacac tttaatgaag taagagattt aagagacact 1440
gaagttgcta ctttagtatc tgtgcataat agaaaaaata atatctatgc tgctcatgaa 1500
aatggtacta tgattcattt tgcgccggaa ggttatacag gtttcacaat atcaccaata 1560
tatgcaactc aagtaaataa tcaaacacga acgtttattt ctgaaaaatt cggaaatcaa 1620
ggtgattcct tgagatttga acaaactaac acaacggctc gttatacgtt tagagggaat 1680
ggtaataatt ataatcttta tttaagagta tcctcacaag gaaattctac ttttcgagtt 1740
actataaacg gtagggttta tactgtttca aatgttaata ccactacaaa taatgatggg 1800

gttattgata atggggctcg tttttcagat attcacatcg ggaatatagt ggcaagtaac 1860
aatactaatg taccattaga tataaatgtg atacttaact ccggtactca atttgagctt 1920
atgaatatta tttttgttcc aactaacatt ccaccacttt attaaggttt gagtttctga 1980
ggtaaatata 1990

<210> 9
<211> 1872
<212> DNA
<213> Bacillus thuringiensis

<220>
<221> CDS
<222> (1)...(1872)

<400> 9
atg aat aat gta ttg aat agc gaa aga act aat aag tgt gat gcg tat 48
Met Asn Asn Val Leu Asn Ser Glu Arg Thr Asn Lys Cys Asp Ala Tyr
1               5                   10                  15

aac gta gtg gcc cat gat cca ttt agt ttt gag cat aaa tca tta gat 96
Asn Val Val Ala His Asp Pro Phe Ser Phe Glu His Lys Ser Leu Asp
            20                  25                  30

acc ata cag caa gaa tgg atg gag tgg aaa aga acc gat cat agt tta 144
Thr Ile Gln Gln Glu Trp Met Glu Trp Lys Arg Thr Asp His Ser Leu
        35                  40                  45

tat gta tct cct att gtg gga act ata gct agt ttt ctg cta aag aaa 192
Tyr Val Ser Pro Ile Val Gly Thr Ile Ala Ser Phe Leu Leu Lys Lys
    50                  55                  60

ata gcg ggg ctt ata gga aaa aga ata tta agt gag tta aag aat tta 240
Ile Ala Gly Leu Ile Gly Lys Arg Ile Leu Ser Glu Leu Lys Asn Leu
65                  70                  75                  80

att ttt cct agt ggt agt ata gaa tca atg caa gat att tta aga ggg 288
Ile Phe Pro Ser Gly Ser Ile Glu Ser Met Gln Asp Ile Leu Arg Gly
                85                  90                  95

gca gaa caa ttc cta aat caa aga ctt gat gca gac acc ttt gct cgg 336
Ala Glu Gln Phe Leu Asn Gln Arg Leu Asp Ala Asp Thr Phe Ala Arg
            100                 105                 110

gta gag gca gaa ttg ata ggg ctt caa gca aat gta gag gaa ttt aat 384
Val Glu Ala Glu Leu Ile Gly Leu Gln Ala Asn Val Glu Glu Phe Asn
        115                 120                 125

caa caa gtg gac aat ttt tta aac cca aat caa aac cct gtt cct tta 432
Gln Gln Val Asp Asn Phe Leu Asn Pro Asn Gln Asn Pro Val Pro Leu
    130                 135                 140

gca ata att gat tcg gtt aat aca atg caa caa tta ttc cta agt aga 480
Ala Ile Ile Asp Ser Val Asn Thr Met Gln Gln Leu Phe Leu Ser Arg
145                 150                 155                 160

tta ccc cag ttc cag ata caa cgc tat cag cta tta tta tta cct tta 528
Leu Pro Gln Phe Gln Ile Gln Arg Tyr Gln Leu Leu Leu Leu Pro Leu
                165                 170                 175

ttt gca caa gca gcc aat tta cac ctt acc ttt att aga gat gtt att 576
Phe Ala Gln Ala Ala Asn Leu His Leu Thr Phe Ile Arg Asp Val Ile
            180                 185                 190

ctt aat gca gat gaa tgg gga ata cca gca gca aca gtg cgc aca tat 624
Leu Asn Ala Asp Glu Trp Gly Ile Pro Ala Ala Thr Val Arg Thr Tyr
        195                 200                 205

aga gag cac cta aaa aga tat aca cgc gat tat tcc aat tat tgt ata 672
Arg Glu His Leu Lys Arg Tyr Thr Arg Asp Tyr Ser Asn Tyr Cys Ile
    210                 215                 220

aac acg tac caa act gct ttc cga ggt tta aac act cgt tta cat gat 720
Asn Thr Tyr Gln Thr Ala Phe Arg Gly Leu Asn Thr Arg Leu His Asp
225                 230                 235                 240

atg tta gag ttt aga aca ttt atg ttt tta aat gta tta gac tat gta 768
Met Leu Glu Phe Arg Thr Phe Met Phe Leu Asn Val Leu Asp Tyr Val
                245                 250                 255

tct atc tgg tcg ttg ttt aaa tat caa agt ctg atg gtt act tca agt 816
Ser Ile Trp Ser Leu Phe Lys Tyr Gln Ser Leu Met Val Thr Ser Ser
            260                 265                 270

gct aat tta tat gct tcg gga agt ggt agt aat caa cct ttt act gca 864
Ala Asn Leu Tyr Ala Ser Gly Ser Gly Ser Asn Gln Pro Phe Thr Ala
        275                 280                 285

caa gac tgg cca ttt tta tat tct ctt ttc caa gtg aat tca aat tat 912
Gln Asp Trp Pro Phe Leu Tyr Ser Leu Phe Gln Val Asn Ser Asn Tyr
    290                 295                 300

ata atg tct aat ttt ggt ggt aac cga gag act gct agt ttt ggt gtt 960
Ile Met Ser Asn Phe Gly Gly Asn Arg Glu Thr Ala Ser Phe Gly Val
305                 310                 315                 320

cct att ctg ggg gga ttc ata ata aat ttt tta ctt agt ttt agg gtt 1008
Pro Ile Leu Gly Gly Phe Ile Ile Asn Phe Leu Leu Ser Phe Arg Val
                325                 330                 335

aat tat act gga gga gtt tca tct ggt ctc cta ggt gtt gaa gga att 1056
Asn Tyr Thr Gly Gly Val Ser Ser Gly Leu Leu Gly Val Glu Gly Ile
            340                 345                 350

tca aac aac ttt aat tgc aac tcc tct tta tca aca cca gtt gta aga 1104
Ser Asn Asn Phe Asn Cys Asn Ser Ser Leu Ser Thr Pro Val Val Arg
        355                 360                 365

agt tgg cta gat tca ggt gta tat cga ggt gac ctg caa cac aat tgg 1152
Ser Trp Leu Asp Ser Gly Val Tyr Arg Gly Asp Leu Gln His Asn Trp
    370                 375                 380

cga aca gac atc ttt atg agg act aat att gta cct tgt ggt gct ttt 1200
Arg Thr Asp Ile Phe Met Arg Thr Asn Ile Val Pro Cys Gly Ala Phe
385                 390                 395                 400

cta tta tct ctt gct atg ttt cca gat gtt aaa agt aat tat ttt cct 1248
Leu Leu Ser Leu Ala Met Phe Pro Asp Val Lys Ser Asn Tyr Phe Pro
                405                 410                 415

gat tat ttc att cgt aac att tcc gga att att cga aat att gat aac 1296
Asp Tyr Phe Ile Arg Asn Ile Ser Gly Ile Ile Arg Asn Ile Asp Asn
            420                 425                 430

atg aat ttg agt aga cca tta cac ttt aat gaa gta aga gat tta aga 1344
Met Asn Leu Ser Arg Pro Leu His Phe Asn Glu Val Arg Asp Leu Arg
        435                 440                 445

gac act gaa gtt gct act tta gta tct gtg cat aat aga aaa aat aat     1392
Asp Thr Glu Val Ala Thr Leu Val Ser Val His Asn Arg Lys Asn Asn
    450                 455                 460

atc tat gct gct cat gaa aat ggt act atg att cat ttt gcg ccg gaa     1440
Ile Tyr Ala Ala His Glu Asn Gly Thr Met Ile His Phe Ala Pro Glu
465                 470                 475                 480

ggt tat aca ggt ttc aca ata tca cca ata tat gca act caa gta aat     1488
Gly Tyr Thr Gly Phe Thr Ile Ser Pro Ile Tyr Ala Thr Gln Val Asn
                485                 490                 495

aat caa aca cga acg ttt att tct gaa aaa ttc gga aat caa ggt gat     1536
Asn Gln Thr Arg Thr Phe Ile Ser Glu Lys Phe Gly Asn Gln Gly Asp
            500                 505                 510

tcc ttg aga ttt gaa caa act aac aca acg gct cgt tat acg ttt aga     1584
Ser Leu Arg Phe Glu Gln Thr Asn Thr Thr Ala Arg Tyr Thr Phe Arg
        515                 520                 525

ggg aat ggt aat aat tat aat ctt tat tta aga gta tcc tca caa gga     1632
Gly Asn Gly Asn Asn Tyr Asn Leu Tyr Leu Arg Val Ser Ser Gln Gly
    530                 535                 540

aat tct act ttt cga gtt act ata aac ggt agg gtt tat act gtt tca     1680
Asn Ser Thr Phe Arg Val Thr Ile Asn Gly Arg Val Tyr Thr Val Ser
545                 550                 555                 560

aat gtt aat acc act aca aat aat gat ggg gtt att gat aat ggg gct     1728
Asn Val Asn Thr Thr Thr Asn Asn Asp Gly Val Ile Asp Asn Gly Ala
                565                 570                 575

cgt ttt tca gat att cac atc ggg aat ata gtg gca agt aac aat act     1776
Arg Phe Ser Asp Ile His Ile Gly Asn Ile Val Ala Ser Asn Asn Thr
            580                 585                 590

aat gta cca tta gat ata aat gtg ata ctt aac tcc ggt act caa ttt     1824
Asn Val Pro Leu Asp Ile Asn Val Ile Leu Asn Ser Gly Thr Gln Phe
        595                 600                 605

gag ctt atg aat att att ttt gtt cca act aac att cca cca ctt tat     1872
Glu Leu Met Asn Ile Ile Phe Val Pro Thr Asn Ile Pro Pro Leu Tyr
    610                 615                 620

<210> 10
<211> 624
<212> PRT
<213> Bacillus thuringiensis

<400> 10
Met Asn Asn Val Leu Asn Ser Glu Arg Thr Asn Lys Cys Asp Ala Tyr
1               5                   10                  15
Asn Val Val Ala His Asp Pro Phe Ser Phe Glu His Lys Ser Leu Asp
            20                  25                  30
Thr Ile Gln Gln Glu Trp Met Glu Trp Lys Arg Thr Asp His Ser Leu
        35                  40                  45
Tyr Val Ser Pro Ile Val Gly Thr Ile Ala Ser Phe Leu Leu Lys Lys
    50                  55                  60
Ile Ala Gly Leu Ile Gly Lys Arg Ile Leu Ser Glu Leu Lys Asn Leu
65                  70                  75                  80

Ile Phe Pro Ser Gly Ser Ile Glu Ser Met Gln Asp Ile Leu Arg Gly
                85                  90                  95
Ala Glu Gln Phe Leu Asn Gln Arg Leu Asp Ala Asp Thr Phe Ala Arg
            100                 105                 110
Val Glu Ala Glu Leu Ile Gly Leu Gln Ala Asn Val Glu Glu Phe Asn
        115                 120                 125
Gln Gln Val Asp Asn Phe Leu Asn Pro Asn Gln Asn Pro Val Pro Leu
    130                 135                 140
Ala Ile Ile Asp Ser Val Asn Thr Met Gln Gln Leu Phe Leu Ser Arg
145                 150                 155                 160
Leu Pro Gln Phe Gln Ile Gln Arg Tyr Gln Leu Leu Leu Leu Pro Leu
                165                 170                 175
Phe Ala Gln Ala Ala Asn Leu His Leu Thr Phe Ile Arg Asp Val Ile
            180                 185                 190
Leu Asn Ala Asp Glu Trp Gly Ile Pro Ala Ala Thr Val Arg Thr Tyr
        195                 200                 205
Arg Glu His Leu Lys Arg Tyr Thr Arg Asp Tyr Ser Asn Tyr Cys Ile
    210                 215                 220
Asn Thr Tyr Gln Thr Ala Phe Arg Gly Leu Asn Thr Arg Leu His Asp
225                 230                 235                 240
Met Leu Glu Phe Arg Thr Phe Met Phe Leu Asn Val Leu Asp Tyr Val
                245                 250                 255
Ser Ile Trp Ser Leu Phe Lys Tyr Gln Ser Leu Met Val Thr Ser Ser
            260                 265                 270
Ala Asn Leu Tyr Ala Ser Gly Ser Gly Ser Asn Gln Pro Phe Thr Ala
        275                 280                 285
Gln Asp Trp Pro Phe Leu Tyr Ser Leu Phe Gln Val Asn Ser Asn Tyr
    290                 295                 300
Ile Met Ser Asn Phe Gly Gly Asn Arg Glu Thr Ala Ser Phe Gly Val
305                 310                 315                 320
Pro Ile Leu Gly Gly Phe Ile Ile Asn Phe Leu Leu Ser Phe Arg Val
                325                 330                 335
Asn Tyr Thr Gly Gly Val Ser Ser Gly Leu Leu Gly Val Glu Gly Ile
            340                 345                 350
Ser Asn Asn Phe Asn Cys Asn Ser Ser Leu Ser Thr Pro Val Val Arg
        355                 360                 365
Ser Trp Leu Asp Ser Gly Val Tyr Arg Gly Asp Leu Gln His Asn Trp
    370                 375                 380
Arg Thr Asp Ile Phe Met Arg Thr Asn Ile Val Pro Cys Gly Ala Phe
385                 390                 395                 400
Leu Leu Ser Leu Ala Met Phe Pro Asp Val Lys Ser Asn Tyr Phe Pro
                405                 410                 415
Asp Tyr Phe Ile Arg Asn Ile Ser Gly Ile Ile Arg Asn Ile Asp Asn
            420                 425                 430
Met Asn Leu Ser Arg Pro Leu His Phe Asn Glu Val Arg Asp Leu Arg
        435                 440                 445
Asp Thr Glu Val Ala Thr Leu Val Ser Val His Asn Arg Lys Asn Asn
    450                 455                 460
Ile Tyr Ala Ala His Glu Asn Gly Thr Met Ile His Phe Ala Pro Glu
465                 470                 475                 480
Gly Tyr Thr Gly Phe Thr Ile Ser Pro Ile Tyr Ala Thr Gln Val Asn
                485                 490                 495
Asn Gln Thr Arg Thr Phe Ile Ser Glu Lys Phe Gly Asn Gln Gly Asp
            500                 505                 510
Ser Leu Arg Phe Glu Gln Thr Asn Thr Thr Ala Arg Tyr Thr Phe Arg
        515                 520                 525
Gly Asn Gly Asn Asn Tyr Asn Leu Tyr Leu Arg Val Ser Ser Gln Gly
    530                 535                 540
Asn Ser Thr Phe Arg Val Thr Ile Asn Gly Arg Val Tyr Thr Val Ser
545                 550                 555                 560
Asn Val Asn Thr Thr Thr Asn Asn Asp Gly Val Ile Asp Asn Gly Ala
                565                 570                 575
Arg Phe Ser Asp Ile His Ile Gly Asn Ile Val Ala Ser Asn Asn Thr

580                 585                 590
Asn Val Pro Leu Asp Ile Asn Val Ile Leu Asn Ser Gly Thr Gln Phe
        595                 600                 605
Glu Leu Met Asn Ile Ile Phe Val Pro Thr Asn Ile Pro Pro Leu Tyr
    610                 615                 620

<210> 11
<211> 1902
<212> DNA
<213> Artificial Sequence

<220>
<223> Synthetic sequence encoding AXMI-076
      (optaxmi-076v04)

<221> CDS
<222> (1)...(1902)

<400> 11
atg aac aat gtt ctc aac aat gga aga aca aca aac tgt gat gcc tac     48
Met Asn Asn Val Leu Asn Asn Gly Arg Thr Thr Asn Cys Asp Ala Tyr
 1               5                  10                  15

aat gtt gtt gct cat gat cct ttc tca ttt gaa cac aag agc ttg gac     96
Asn Val Val Ala His Asp Pro Phe Ser Phe Glu His Lys Ser Leu Asp
            20                  25                  30

acc atc aga aaa gaa tgg atg gaa tgg aaa aga aca gat cat tct ctc    144
Thr Ile Arg Lys Glu Trp Met Glu Trp Lys Arg Thr Asp His Ser Leu
        35                  40                  45

tat gtt gct cct gtt gtt gga act gtt agc agc ttc ttg ctg aag aag    192
Tyr Val Ala Pro Val Val Gly Thr Val Ser Ser Phe Leu Leu Lys Lys
    50                  55                  60

gtt ggc agc ttg att gga aaa agg att ctt tca gag ctc tgg ggc ttg    240
Val Gly Ser Leu Ile Gly Lys Arg Ile Leu Ser Glu Leu Trp Gly Leu
65                  70                  75                  80

atc ttt cct tct gga agc acc aac ttg atg caa gac atc ttg aga gaa    288
Ile Phe Pro Ser Gly Ser Thr Asn Leu Met Gln Asp Ile Leu Arg Glu
                85                  90                  95

aca gag cag ttc ttg aac caa agg ctc aac aca gac acc ttg gca agg    336
Thr Glu Gln Phe Leu Asn Gln Arg Leu Asn Thr Asp Thr Leu Ala Arg
            100                 105                 110

gtg aat gct gag ctc act ggc ctt caa gca aac atc aga gag ttc aac    384
Val Asn Ala Glu Leu Thr Gly Leu Gln Ala Asn Ile Arg Glu Phe Asn
        115                 120                 125

caa caa gtt gac aac ttc ttg aat cca aca caa aat cct gtt cct ctc    432
Gln Gln Val Asp Asn Phe Leu Asn Pro Thr Gln Asn Pro Val Pro Leu
    130                 135                 140

tcc atc act tct tca gtg aac acc atg cag cag ctc ttc ttg aac agg    480
Ser Ile Thr Ser Ser Val Asn Thr Met Gln Gln Leu Phe Leu Asn Arg
145                 150                 155                 160

ctt cct cag ttc cag att caa gga tat cag ctg ctg ctg ctt cct ctc    528
Leu Pro Gln Phe Gln Ile Gln Gly Tyr Gln Leu Leu Leu Leu Pro Leu
                165                 170                 175

ttt gct caa gct gca aac atg cat ctc tcc ttc atc aga gat gtc atc 576
Phe Ala Gln Ala Ala Asn Met His Leu Ser Phe Ile Arg Asp Val Ile
            180                 185                 190

ttg aat gct gat gaa tgg ggc atc tct gct gcc acc ttg aga aca tac 624
Leu Asn Ala Asp Glu Trp Gly Ile Ser Ala Ala Thr Leu Arg Thr Tyr
        195                 200                 205

aga gat tat ttg aga aac tac aca aga gat tat tca aac tac tgc atc 672
Arg Asp Tyr Leu Arg Asn Tyr Thr Arg Asp Tyr Ser Asn Tyr Cys Ile
    210                 215                 220

aac aca tat caa aca gct ttc aga ggc ctc aac aca agg ctt cat gac 720
Asn Thr Tyr Gln Thr Ala Phe Arg Gly Leu Asn Thr Arg Leu His Asp
225                 230                 235                 240

atg ctg gag ttc aga aca tac atg ttc ttg aat gtt ttt gaa tat gtt 768
Met Leu Glu Phe Arg Thr Tyr Met Phe Leu Asn Val Phe Glu Tyr Val
                245                 250                 255

tcc atc tgg agc ttg ttc aag tac cag agc ttg atg gtt tct tct gga 816
Ser Ile Trp Ser Leu Phe Lys Tyr Gln Ser Leu Met Val Ser Ser Gly
            260                 265                 270

gca aat tta tat gca tct gga tct gga cct caa caa aca cag agc ttc 864
Ala Asn Leu Tyr Ala Ser Gly Ser Gly Pro Gln Gln Thr Gln Ser Phe
        275                 280                 285

atc tct caa gat tgg cca ttt ctc tac agc ttg ttc caa gtg aac agc 912
Ile Ser Gln Asp Trp Pro Phe Leu Tyr Ser Leu Phe Gln Val Asn Ser
    290                 295                 300

aac tat gtt ctt aat ggc ttc tct gga gca aga aac acc atc aga ttt 960
Asn Tyr Val Leu Asn Gly Phe Ser Gly Ala Arg Asn Thr Ile Arg Phe
305                 310                 315                 320

cct gct gga gga ggc ttg ctg cct cct ggt gtc acc acc acc cat gct 1008
Pro Ala Gly Gly Gly Leu Leu Pro Pro Gly Val Thr Thr Thr His Ala
                325                 330                 335

ctt ctg gcg gcg cgc gtc aac tac tca gga gga gtt tct tct gga tac 1056
Leu Leu Ala Ala Arg Val Asn Tyr Ser Gly Gly Val Ser Ser Gly Tyr
            340                 345                 350

att gga gct gtg ttc aac caa aac ttc aac tgc tcc acc ttg ctg ccg 1104
Ile Gly Ala Val Phe Asn Gln Asn Phe Asn Cys Ser Thr Leu Leu Pro
        355                 360                 365

ccg ctg ctg aca cca ttt gtc aga agc tgg ctg gac tca gga act gac 1152
Pro Leu Leu Thr Pro Phe Val Arg Ser Trp Leu Asp Ser Gly Thr Asp
    370                 375                 380

aga gaa gga gtc acc acc gtc acc aac tgg caa aca gag agc ttc aaa 1200
Arg Glu Gly Val Thr Thr Val Thr Asn Trp Gln Thr Glu Ser Phe Lys
385                 390                 395                 400

aca acc ttg ggc ctc cgc tgc ggc gcc ttc acg ccg cgc ggc aac agc 1248
Thr Thr Leu Gly Leu Arg Cys Gly Ala Phe Thr Pro Arg Gly Asn Ser
                405                 410                 415

aac tac ttt cca gat tac ttc atc aga aac att tct gga gtt cct ttg 1296
Asn Tyr Phe Pro Asp Tyr Phe Ile Arg Asn Ile Ser Gly Val Pro Leu

                420                 425                 430

gtg gtg aga aat gaa gat ctg agg agg cct ctt cac tac aat gaa atc    1344
Val Val Arg Asn Glu Asp Leu Arg Arg Pro Leu His Tyr Asn Glu Ile
        435                 440                 445

aga aac att gaa tct cca tca gga act cct gga ggc ctc cgc gcc tac    1392
Arg Asn Ile Glu Ser Pro Ser Gly Thr Pro Gly Gly Leu Arg Ala Tyr
    450                 455                 460

atg gtt tct gtt cac aac agg aag aac aac atc tat gct gct cat gaa    1440
Met Val Ser Val His Asn Arg Lys Asn Asn Ile Tyr Ala Ala His Glu
465                 470                 475                 480

aat gga aca atg att cat ctt gct cca gaa gat tac act ggc ttc acc    1488
Asn Gly Thr Met Ile His Leu Ala Pro Glu Asp Tyr Thr Gly Phe Thr
                485                 490                 495

atg ctg ccc atc cat gca aca caa gtc aac aac caa aca agg acc ttc    1536
Met Leu Pro Ile His Ala Thr Gln Val Asn Asn Gln Thr Arg Thr Phe
            500                 505                 510

att tca gaa aaa ttt gga aat caa gga gat tct ttg aga ttt gaa caa    1584
Ile Ser Glu Lys Phe Gly Asn Gln Gly Asp Ser Leu Arg Phe Glu Gln
        515                 520                 525

agc gac acc aca gca aga tac acc ttg aga gga aat gga aac agc tac    1632
Ser Asp Thr Thr Ala Arg Tyr Thr Leu Arg Gly Asn Gly Asn Ser Tyr
    530                 535                 540

aac ttg tac ctc cgc gtc agc agc ttg gga aac agc acc atc agg gtg    1680
Asn Leu Tyr Leu Arg Val Ser Ser Leu Gly Asn Ser Thr Ile Arg Val
545                 550                 555                 560

acc atc aat gga aga gtc tac act gtt cca aat gtc aac acc aac atc    1728
Thr Ile Asn Gly Arg Val Tyr Thr Val Pro Asn Val Asn Thr Asn Ile
                565                 570                 575

aac aat gat ggt gtc att gac aat gga gca aga ttt tca gac atc aac    1776
Asn Asn Asp Gly Val Ile Asp Asn Gly Ala Arg Phe Ser Asp Ile Asn
            580                 585                 590

att gga aat gtg gtg gct tct gac aac aca aat gtt cct ctg gac atc    1824
Ile Gly Asn Val Val Ala Ser Asp Asn Thr Asn Val Pro Leu Asp Ile
        595                 600                 605

aat gtc acc ctt tct tct gga aca caa ttt gag ctg atg aac atc atg    1872
Asn Val Thr Leu Ser Ser Gly Thr Gln Phe Glu Leu Met Asn Ile Met
    610                 615                 620

ttt gtt cca aca aat ctt cct cca atc tac                            1902
Phe Val Pro Thr Asn Leu Pro Pro Ile Tyr
625                 630


<210> 12
<211> 2130
<212> DNA
<213> Artificial Sequence

<220>
<223> Synthetic sequence encoding AXMI-076 with a
      chloroplast targeting peptide (optaxmi-076v04)

<221> CDS
<222> (1)...(2130)

<400> 12
atg cag ctg ctg aac caa aga caa gct ctt cgc ctt gga aga agc tca    48
Met Gln Leu Leu Asn Gln Arg Gln Ala Leu Arg Leu Gly Arg Ser Ser
 1               5                  10                  15

gca agc aaa aat caa caa gtg gcg ccg ctg gca tca agg cca gct tct    96
Ala Ser Lys Asn Gln Gln Val Ala Pro Leu Ala Ser Arg Pro Ala Ser
            20                  25                  30

tct ctt tct gtt tct gct tct tct gtg gcg ccg gcg ccg gca tgc tct    144
Ser Leu Ser Val Ser Ala Ser Ser Val Ala Pro Ala Pro Ala Cys Ser
        35                  40                  45

gct cct gct gga gct ggt aga aga gct gtg gtg gtg aga gct tct gca    192
Ala Pro Ala Gly Ala Gly Arg Arg Ala Val Val Val Arg Ala Ser Ala
    50                  55                  60

aca aag gag aag gtg gag gag ctc acc atc cag atg aac aat gtt ctc    240
Thr Lys Glu Lys Val Glu Glu Leu Thr Ile Gln Met Asn Asn Val Leu
 65                  70                  75                  80

aac aat gga aga aca aca aac tgt gat gcc tac aat gtt gtt gct cat    288
Asn Asn Gly Arg Thr Thr Asn Cys Asp Ala Tyr Asn Val Val Ala His
                85                  90                  95

gat cct ttc tca ttt gaa cac aag agc ttg gac acc atc aga aaa gaa    336
Asp Pro Phe Ser Phe Glu His Lys Ser Leu Asp Thr Ile Arg Lys Glu
            100                 105                 110

tgg atg gaa tgg aaa aga aca gat cat tct ctc tat gtt gct cct gtt    384
Trp Met Glu Trp Lys Arg Thr Asp His Ser Leu Tyr Val Ala Pro Val
        115                 120                 125

gtt gga act gtt agc agc ttc ttg ctg aag aag gtt ggc agc ttg att    432
Val Gly Thr Val Ser Ser Phe Leu Leu Lys Lys Val Gly Ser Leu Ile
    130                 135                 140

gga aaa agg att ctt tca gag ctc tgg ggc ttg atc ttt cct tct gga    480
Gly Lys Arg Ile Leu Ser Glu Leu Trp Gly Leu Ile Phe Pro Ser Gly
145                 150                 155                 160

agc acc aac ttg atg caa gac atc ttg aga gaa aca gag cag ttc ttg    528
Ser Thr Asn Leu Met Gln Asp Ile Leu Arg Glu Thr Glu Gln Phe Leu
                165                 170                 175

aac caa agg ctc aac aca gac acc ttg gca agg gtg aat gct gag ctc    576
Asn Gln Arg Leu Asn Thr Asp Thr Leu Ala Arg Val Asn Ala Glu Leu
            180                 185                 190

act ggc ctt caa gca aac atc aga gag ttc aac caa caa gtt gac aac    624
Thr Gly Leu Gln Ala Asn Ile Arg Glu Phe Asn Gln Gln Val Asp Asn
        195                 200                 205

ttc ttg aat cca aca caa aat cct gtt cct ctc tcc atc act tct tca    672
Phe Leu Asn Pro Thr Gln Asn Pro Val Pro Leu Ser Ile Thr Ser Ser
    210                 215                 220

gtg aac acc atg cag cag ctc ttc ttg aac agg ctt cct cag ttc cag    720
Val Asn Thr Met Gln Gln Leu Phe Leu Asn Arg Leu Pro Gln Phe Gln

225                 230                 235                 240

att caa gga tat cag ctg ctg ctg ctt cct ctc ttt gct caa gct gca     768
Ile Gln Gly Tyr Gln Leu Leu Leu Leu Pro Leu Phe Ala Gln Ala Ala
                245                 250                 255

aac atg cat ctc tcc ttc atc aga gat gtc atc ttg aat gct gat gaa     816
Asn Met His Leu Ser Phe Ile Arg Asp Val Ile Leu Asn Ala Asp Glu
            260                 265                 270

tgg ggc atc tct gct gcc acc ttg aga aca tac aga gat tat ttg aga     864
Trp Gly Ile Ser Ala Ala Thr Leu Arg Thr Tyr Arg Asp Tyr Leu Arg
        275                 280                 285

aac tac aca aga gat tat tca aac tac tgc atc aac aca tat caa aca     912
Asn Tyr Thr Arg Asp Tyr Ser Asn Tyr Cys Ile Asn Thr Tyr Gln Thr
    290                 295                 300

gct ttc aga ggc ctc aac aca agg ctt cat gac atg ctg gag ttc aga     960
Ala Phe Arg Gly Leu Asn Thr Arg Leu His Asp Met Leu Glu Phe Arg
305                 310                 315                 320

aca tac atg ttc ttg aat gtt ttt gaa tat gtt tcc atc tgg agc ttg    1008
Thr Tyr Met Phe Leu Asn Val Phe Glu Tyr Val Ser Ile Trp Ser Leu
                325                 330                 335

ttc aag tac cag agc ttg atg gtt tct tct gga gca aat tta tat gca    1056
Phe Lys Tyr Gln Ser Leu Met Val Ser Ser Gly Ala Asn Leu Tyr Ala
            340                 345                 350

tct gga tct gga cct caa caa aca cag agc ttc atc tct caa gat tgg    1104
Ser Gly Ser Gly Pro Gln Gln Thr Gln Ser Phe Ile Ser Gln Asp Trp
        355                 360                 365

cca ttt ctc tac agc ttg ttc caa gtg aac agc aac tat gtt ctt aat    1152
Pro Phe Leu Tyr Ser Leu Phe Gln Val Asn Ser Asn Tyr Val Leu Asn
    370                 375                 380

ggc ttc tct gga gca aga aac acc atc aga ttt cct gct gga gga ggc    1200
Gly Phe Ser Gly Ala Arg Asn Thr Ile Arg Phe Pro Ala Gly Gly Gly
385                 390                 395                 400

ttg ctg cct cct ggt gtc acc acc acc cat gct ctt ctg gcg gcg cgc    1248
Leu Leu Pro Pro Gly Val Thr Thr Thr His Ala Leu Leu Ala Ala Arg
                405                 410                 415

gtc aac tac tca gga gga gtt tct tct gga tac att gga gct gtg ttc    1296
Val Asn Tyr Ser Gly Gly Val Ser Ser Gly Tyr Ile Gly Ala Val Phe
            420                 425                 430

aac caa aac ttc aac tgc tcc acc ttg ctg ccg ccg ctg ctg aca cca    1344
Asn Gln Asn Phe Asn Cys Ser Thr Leu Leu Pro Pro Leu Leu Thr Pro
        435                 440                 445

ttt gtc aga agc tgg ctg gac tca gga act gac aga gaa gga gtc acc    1392
Phe Val Arg Ser Trp Leu Asp Ser Gly Thr Asp Arg Glu Gly Val Thr
    450                 455                 460

acc gtc acc aac tgg caa aca gag agc ttc aaa aca acc ttg ggc ctc    1440
Thr Val Thr Asn Trp Gln Thr Glu Ser Phe Lys Thr Thr Leu Gly Leu
465                 470                 475                 480

cgc tgc ggc gcc ttc acg ccg cgc ggc aac agc aac tac ttt cca gat    1488

Arg Cys Gly Ala Phe Thr Pro Arg Gly Asn Ser Asn Tyr Phe Pro Asp
                485                 490                 495

tac ttc atc aga aac att tct gga gtt cct ttg gtg gtg aga aat gaa      1536
Tyr Phe Ile Arg Asn Ile Ser Gly Val Pro Leu Val Val Arg Asn Glu
            500                 505                 510

gat ctg agg agg cct ctt cac tac aat gaa atc aga aac att gaa tct      1584
Asp Leu Arg Arg Pro Leu His Tyr Asn Glu Ile Arg Asn Ile Glu Ser
        515                 520                 525

cca tca gga act cct gga ggc ctc cgc gcc tac atg gtt tct gtt cac      1632
Pro Ser Gly Thr Pro Gly Gly Leu Arg Ala Tyr Met Val Ser Val His
    530                 535                 540

aac agg aag aac aac atc tat gct gct cat gaa aat gga aca atg att      1680
Asn Arg Lys Asn Asn Ile Tyr Ala Ala His Glu Asn Gly Thr Met Ile
545                 550                 555                 560

cat ctt gct cca gaa gat tac act ggc ttc acc atg ctg ccc atc cat      1728
His Leu Ala Pro Glu Asp Tyr Thr Gly Phe Thr Met Leu Pro Ile His
                565                 570                 575

gca aca caa gtc aac aac caa aca agg acc ttc att tca gaa aaa ttt      1776
Ala Thr Gln Val Asn Asn Gln Thr Arg Thr Phe Ile Ser Glu Lys Phe
            580                 585                 590

gga aat caa gga gat tct ttg aga ttt gaa caa agc gac acc aca gca      1824
Gly Asn Gln Gly Asp Ser Leu Arg Phe Glu Gln Ser Asp Thr Thr Ala
        595                 600                 605

aga tac acc ttg aga gga aat gga aac agc tac aac ttg tac ctc cgc      1872
Arg Tyr Thr Leu Arg Gly Asn Gly Asn Ser Tyr Asn Leu Tyr Leu Arg
    610                 615                 620

gtc agc agc ttg gga aac agc acc atc agg gtg acc atc aat gga aga      1920
Val Ser Ser Leu Gly Asn Ser Thr Ile Arg Val Thr Ile Asn Gly Arg
625                 630                 635                 640

gtc tac act gtt cca aat gtc aac acc aac atc aac aat gat ggt gtc      1968
Val Tyr Thr Val Pro Asn Val Asn Thr Asn Ile Asn Asn Asp Gly Val
                645                 650                 655

att gac aat gga gca aga ttt tca gac atc aac att gga aat gtg gtg      2016
Ile Asp Asn Gly Ala Arg Phe Ser Asp Ile Asn Ile Gly Asn Val Val
            660                 665                 670

gct tct gac aac aca aat gtt cct ctg gac atc aat gtc acc ctt tct      2064
Ala Ser Asp Asn Thr Asn Val Pro Leu Asp Ile Asn Val Thr Leu Ser
        675                 680                 685

tct gga aca caa ttt gag ctg atg aac atc atg ttt gtt cca aca aat      2112
Ser Gly Thr Gln Phe Glu Leu Met Asn Ile Met Phe Val Pro Thr Asn
    690                 695                 700

ctt cct cca atc tac taa                                              2130
Leu Pro Pro Ile Tyr  *
705


<210> 13
<211> 709
<212> PRT

<213> Artificial Sequence

<220>
<223> AXMI-076 with a chlorooplast targeting peptide

<400> 13
Met Gln Leu Leu Asn Gln Arg Gln Ala Leu Arg Leu Gly Arg Ser Ser
1               5                   10                  15
Ala Ser Lys Asn Gln Gln Val Ala Pro Leu Ala Ser Arg Pro Ala Ser
            20                  25                  30
Ser Leu Ser Val Ser Ala Ser Ser Val Ala Pro Ala Pro Ala Cys Ser
        35                  40                  45
Ala Pro Ala Gly Ala Gly Arg Arg Ala Val Val Val Arg Ala Ser Ala
    50                  55                  60
Thr Lys Glu Lys Val Glu Glu Leu Thr Ile Gln Met Asn Asn Val Leu
65                  70                  75                  80
Asn Asn Gly Arg Thr Thr Asn Cys Asp Ala Tyr Asn Val Val Ala His
                85                  90                  95
Asp Pro Phe Ser Phe Glu His Lys Ser Leu Asp Thr Ile Arg Lys Glu
            100                 105                 110
Trp Met Glu Trp Lys Arg Thr Asp His Ser Leu Tyr Val Ala Pro Val
        115                 120                 125
Val Gly Thr Val Ser Ser Phe Leu Leu Lys Lys Val Gly Ser Leu Ile
    130                 135                 140
Gly Lys Arg Ile Leu Ser Glu Leu Trp Gly Leu Ile Phe Pro Ser Gly
145                 150                 155                 160
Ser Thr Asn Leu Met Gln Asp Ile Leu Arg Glu Thr Glu Gln Phe Leu
                165                 170                 175
Asn Gln Arg Leu Asn Thr Asp Thr Leu Ala Arg Val Asn Ala Glu Leu
            180                 185                 190
Thr Gly Leu Gln Ala Asn Ile Arg Glu Phe Asn Gln Gln Val Asp Asn
        195                 200                 205
Phe Leu Asn Pro Thr Gln Asn Pro Val Pro Leu Ser Ile Thr Ser Ser
    210                 215                 220
Val Asn Thr Met Gln Gln Leu Phe Leu Asn Arg Leu Pro Gln Phe Gln
225                 230                 235                 240
Ile Gln Gly Tyr Gln Leu Leu Leu Leu Pro Leu Phe Ala Gln Ala Ala
                245                 250                 255
Asn Met His Leu Ser Phe Ile Arg Asp Val Ile Leu Asn Ala Asp Glu
            260                 265                 270
Trp Gly Ile Ser Ala Ala Thr Leu Arg Thr Tyr Arg Asp Tyr Leu Arg
        275                 280                 285
Asn Tyr Thr Arg Asp Tyr Ser Asn Tyr Cys Ile Asn Thr Tyr Gln Thr
    290                 295                 300
Ala Phe Arg Gly Leu Asn Thr Arg Leu His Asp Met Leu Glu Phe Arg
305                 310                 315                 320
Thr Tyr Met Phe Leu Asn Val Phe Glu Tyr Val Ser Ile Trp Ser Leu
                325                 330                 335
Phe Lys Tyr Gln Ser Leu Met Val Ser Ser Gly Ala Asn Leu Tyr Ala
            340                 345                 350
Ser Gly Ser Gly Pro Gln Gln Thr Gln Ser Phe Ile Ser Gln Asp Trp
        355                 360                 365
Pro Phe Leu Tyr Ser Leu Phe Gln Val Asn Ser Asn Tyr Val Leu Asn
    370                 375                 380
Gly Phe Ser Gly Ala Arg Asn Thr Ile Arg Phe Pro Ala Gly Gly Gly
385                 390                 395                 400
Leu Leu Pro Pro Gly Val Thr Thr Thr His Ala Leu Leu Ala Ala Arg
                405                 410                 415
Val Asn Tyr Ser Gly Gly Val Ser Ser Gly Tyr Ile Gly Ala Val Phe
            420                 425                 430
Asn Gln Asn Phe Asn Cys Ser Thr Leu Leu Pro Pro Leu Leu Thr Pro
        435                 440                 445
Phe Val Arg Ser Trp Leu Asp Ser Gly Thr Asp Arg Glu Gly Val Thr

450                 455                 460
Thr Val Thr Asn Trp Gln Thr Glu Ser Phe Lys Thr Thr Leu Gly Leu
465                 470                 475                 480
Arg Cys Gly Ala Phe Thr Pro Arg Gly Asn Ser Asn Tyr Phe Pro Asp
                485                 490                 495
Tyr Phe Ile Arg Asn Ile Ser Gly Val Pro Leu Val Val Arg Asn Glu
            500                 505                 510
Asp Leu Arg Arg Pro Leu His Tyr Asn Glu Ile Arg Asn Ile Glu Ser
        515                 520                 525
Pro Ser Gly Thr Pro Gly Gly Leu Arg Ala Tyr Met Val Ser Val His
    530                 535                 540
Asn Arg Lys Asn Asn Ile Tyr Ala Ala His Glu Asn Gly Thr Met Ile
545                 550                 555                 560
His Leu Ala Pro Glu Asp Tyr Thr Gly Phe Thr Met Leu Pro Ile His
                565                 570                 575
Ala Thr Gln Val Asn Asn Gln Thr Arg Thr Phe Ile Ser Glu Lys Phe
            580                 585                 590
Gly Asn Gln Gly Asp Ser Leu Arg Phe Glu Gln Ser Asp Thr Thr Ala
        595                 600                 605
Arg Tyr Thr Leu Arg Gly Asn Gly Asn Ser Tyr Asn Leu Tyr Leu Arg
    610                 615                 620
Val Ser Ser Leu Gly Asn Ser Thr Ile Arg Val Thr Ile Asn Gly Arg
625                 630                 635                 640
Val Tyr Thr Val Pro Asn Val Asn Thr Asn Ile Asn Asn Asp Gly Val
                645                 650                 655
Ile Asp Asn Gly Ala Arg Phe Ser Asp Ile Asn Ile Gly Asn Val Val
            660                 665                 670
Ala Ser Asp Asn Thr Asn Val Pro Leu Asp Ile Asn Val Thr Leu Ser
        675                 680                 685
Ser Gly Thr Gln Phe Glu Leu Met Asn Ile Met Phe Val Pro Thr Asn
    690                 695                 700
Leu Pro Pro Ile Tyr
705

<210> 14
<211> 633
<212> PRT
<213> Bacillus thuringiensis

<400> 14
Met Asn Asn Val Leu Asn Ser Gly Arg Thr Thr Ile Cys Asp Ala Tyr
1               5                   10                  15
Asn Val Val Ala His Asp Pro Phe Ser Phe Glu His Lys Ser Leu Asp
            20                  25                  30
Thr Ile Gln Lys Glu Trp Met Glu Trp Lys Arg Thr Asp His Ser Leu
        35                  40                  45
Tyr Val Ala Pro Val Val Gly Thr Val Ser Ser Phe Leu Leu Lys Lys
    50                  55                  60
Val Gly Ser Leu Ile Gly Lys Arg Ile Leu Ser Glu Leu Trp Gly Ile
65                  70                  75                  80
Ile Phe Pro Ser Gly Ser Thr Asn Leu Met Gln Asp Ile Leu Arg Glu
                85                  90                  95
Thr Glu Gln Phe Leu Asn Gln Arg Leu Asn Thr Asp Thr Leu Ala Arg
            100                 105                 110
Val Asn Ala Glu Leu Ile Gly Leu Gln Ala Asn Ile Arg Glu Phe Asn
        115                 120                 125
Gln Gln Val Asp Asn Phe Leu Asn Pro Thr Gln Asn Pro Val Pro Leu
    130                 135                 140
Ser Ile Thr Ser Ser Val Asn Thr Met Gln Gln Leu Phe Leu Asn Arg
145                 150                 155                 160
Leu Pro Gln Phe Gln Ile Gln Gly Tyr Gln Leu Leu Leu Leu Pro Leu
                165                 170                 175

Phe Ala Gln Ala Ala Asn Met His Leu Ser Phe Ile Arg Asp Val Ile
            180                 185                 190
Leu Asn Ala Asp Glu Trp Gly Ile Ser Ala Ala Thr Leu Arg Thr Tyr
        195                 200                 205
Arg Asp Tyr Leu Arg Asn Tyr Thr Arg Asp Tyr Ser Asn Tyr Cys Ile
    210                 215                 220
Asn Thr Tyr Gln Thr Ala Phe Arg Gly Leu Asn Thr Arg Leu His Asp
225                 230                 235                 240
Met Leu Glu Phe Arg Thr Tyr Met Phe Leu Asn Val Phe Glu Tyr Val
                245                 250                 255
Ser Ile Trp Ser Leu Phe Lys Tyr Gln Ser Leu Met Val Ser Ser Gly
            260                 265                 270
Ala Asn Leu Tyr Ala Ser Gly Ser Gly Pro Gln Gln Thr Gln Ser Phe
        275                 280                 285
Thr Ala Gln Asn Trp Pro Phe Leu Tyr Ser Leu Phe Gln Val Asn Ser
    290                 295                 300
Asn Tyr Ile Leu Ser Gly Ile Ser Gly Thr Arg Leu Ser Ile Thr Phe
305                 310                 315                 320
Pro Asn Ile Gly Gly Leu Pro Gly Ser Thr Thr Thr His Ser Leu Asn
                325                 330                 335
Ser Ala Arg Val Asn Tyr Ser Gly Gly Val Ser Ser Gly Leu Ile Gly
            340                 345                 350
Ala Thr Asn Leu Asn His Asn Phe Asn Cys Ser Thr Val Leu Pro Pro
        355                 360                 365
Leu Ser Thr Pro Phe Val Arg Ser Trp Leu Asp Ser Gly Thr Asp Arg
    370                 375                 380
Glu Gly Val Ala Thr Ser Thr Asn Trp Gln Thr Glu Ser Phe Gln Thr
385                 390                 395                 400
Thr Leu Ser Leu Arg Cys Gly Ala Phe Ser Ala Arg Gly Asn Ser Asn
                405                 410                 415
Tyr Phe Pro Asp Tyr Phe Ile Arg Asn Ile Ser Gly Val Pro Leu Val
            420                 425                 430
Ile Arg Asn Glu Asp Leu Thr Arg Pro Leu His Tyr Asn Gln Ile Arg
        435                 440                 445
Asn Ile Glu Ser Pro Ser Gly Thr Pro Gly Gly Ala Arg Ala Tyr Leu
    450                 455                 460
Val Ser Val His Asn Arg Lys Asn Asn Ile Tyr Ala Ala Asn Glu Asn
465                 470                 475                 480
Gly Thr Met Ile His Leu Ala Pro Glu Asp Tyr Thr Gly Phe Thr Ile
                485                 490                 495
Ser Pro Ile His Ala Thr Gln Val Asn Asn Gln Thr Arg Thr Phe Ile
            500                 505                 510
Ser Glu Lys Phe Gly Asn Gln Gly Asp Ser Leu Arg Phe Glu Gln Ser
        515                 520                 525
Asn Thr Thr Ala Arg Tyr Thr Leu Arg Gly Asn Gly Asn Ser Tyr Asn
    530                 535                 540
Leu Tyr Leu Arg Val Ser Ser Ile Gly Asn Ser Thr Ile Arg Val Thr
545                 550                 555                 560
Ile Asn Gly Arg Val Tyr Thr Val Ser Asn Val Asn Thr Thr Thr Asn
                565                 570                 575
Asn Asp Gly Val Asn Asp Asn Gly Ala Arg Phe Ser Asp Ile Asn Ile
            580                 585                 590
Gly Asn Ile Val Ala Ser Asp Asn Thr Asn Val Thr Leu Asp Ile Asn
        595                 600                 605
Val Thr Leu Asn Ser Gly Thr Pro Phe Asp Leu Met Asn Ile Met Phe
    610                 615                 620
Val Pro Thr Asn Leu Pro Pro Leu Tyr
625                 630

<210> 15
<211> 633
<212> PRT

<213> Bacillus thuringiensis

<400> 15
Met Asn Ser Val Leu Asn Ser Gly Arg Thr Thr Ile Cys Asp Ala Tyr
1               5                   10                  15
Asn Val Ala Ala His Asp Pro Phe Ser Phe Gln His Lys Ser Leu Asp
            20                  25                  30
Thr Val Gln Lys Glu Trp Thr Glu Trp Lys Lys Asn Asn His Ser Leu
        35                  40                  45
Tyr Leu Asp Pro Ile Val Gly Thr Val Ala Ser Phe Leu Leu Lys Lys
    50                  55                  60
Val Gly Ser Leu Val Gly Lys Arg Ile Leu Ser Glu Leu Arg Asn Leu
65                  70                  75                  80
Ile Phe Pro Ser Gly Ser Thr Asn Leu Met Gln Asp Ile Leu Arg Glu
                85                  90                  95
Thr Glu Lys Phe Leu Asn Gln Arg Leu Asn Thr Asp Thr Leu Ala Arg
            100                 105                 110
Val Asn Ala Glu Leu Thr Gly Leu Gln Ala Asn Val Glu Glu Phe Asn
        115                 120                 125
Arg Gln Val Asp Asn Phe Leu Asn Pro Asn Arg Asn Ala Val Pro Leu
    130                 135                 140
Ser Ile Thr Ser Ser Val Asn Thr Met Gln Gln Leu Phe Leu Asn Arg
145                 150                 155                 160
Leu Pro Gln Phe Gln Met Gln Gly Tyr Gln Leu Leu Leu Leu Pro Leu
                165                 170                 175
Phe Ala Gln Ala Ala Asn Leu His Leu Ser Phe Ile Arg Asp Val Ile
            180                 185                 190
Leu Asn Ala Asp Glu Trp Gly Ile Ser Ala Ala Thr Leu Arg Thr Tyr
        195                 200                 205
Arg Asp Tyr Leu Lys Asn Tyr Thr Arg Asp Tyr Ser Asn Tyr Cys Ile
    210                 215                 220
Asn Thr Tyr Gln Ser Ala Phe Lys Gly Leu Asn Thr Arg Leu His Asp
225                 230                 235                 240
Met Leu Glu Phe Arg Thr Tyr Met Phe Leu Asn Val Phe Glu Tyr Val
                245                 250                 255
Ser Ile Trp Ser Leu Phe Lys Tyr Gln Ser Leu Leu Val Ser Ser Gly
            260                 265                 270
Ala Asn Leu Tyr Ala Ser Gly Ser Gly Pro Gln Gln Thr Gln Ser Phe
        275                 280                 285
Thr Ser Gln Asp Trp Pro Phe Leu Tyr Ser Leu Phe Gln Val Asn Ser
    290                 295                 300
Asn Tyr Val Leu Asn Gly Phe Ser Gly Ala Arg Leu Ser Asn Thr Phe
305                 310                 315                 320
Pro Asn Ile Val Gly Leu Pro Gly Ser Thr Thr Thr His Ala Leu Leu
                325                 330                 335
Ala Ala Arg Val Asn Tyr Ser Gly Gly Ile Ser Ser Gly Asp Ile Gly
            340                 345                 350
Ala Ser Pro Phe Asn Gln Asn Phe Asn Cys Ser Thr Phe Leu Pro Pro
        355                 360                 365
Leu Leu Thr Pro Phe Val Arg Ser Trp Leu Asp Ser Gly Ser Asp Arg
    370                 375                 380
Glu Gly Val Ala Thr Val Thr Asn Trp Gln Thr Glu Ser Phe Glu Thr
385                 390                 395                 400
Thr Leu Gly Leu Arg Ser Gly Ala Phe Thr Ala Arg Gly Asn Ser Asn
                405                 410                 415
Tyr Phe Pro Asp Tyr Phe Ile Arg Asn Ile Ser Gly Val Pro Leu Val
            420                 425                 430
Val Arg Asn Glu Asp Leu Arg Arg Pro Leu His Tyr Asn Glu Ile Arg
        435                 440                 445
Asn Ile Ala Ser Pro Ser Gly Thr Pro Gly Gly Ala Arg Ala Tyr Met
    450                 455                 460
Val Ser Val His Asn Arg Lys Asn Asn Ile His Ala Val His Glu Asn
465                 470                 475                 480

Gly Ser Met Ile His Leu Ala Pro Asn Asp Tyr Thr Gly Phe Thr Ile
                485                 490                 495
Ser Pro Ile His Ala Thr Gln Val Asn Asn Gln Thr Arg Thr Phe Ile
            500                 505                 510
Ser Glu Lys Phe Gly Asn Gln Gly Asp Ser Leu Arg Phe Glu Gln Asn
        515                 520                 525
Asn Thr Thr Ala Arg Tyr Thr Leu Arg Gly Asn Gly Asn Ser Tyr Asn
    530                 535                 540
Leu Tyr Leu Arg Val Ser Ser Ile Gly Asn Ser Thr Ile Arg Val Thr
545                 550                 555                 560
Ile Asn Gly Arg Val Tyr Thr Ala Thr Asn Val Asn Thr Thr Thr Asn
                565                 570                 575
Asn Asp Gly Val Asn Asp Asn Gly Ala Arg Phe Ser Asp Ile Asn Ile
            580                 585                 590
Gly Asn Val Val Ala Ser Ser Asn Ser Asp Val Pro Leu Asp Ile Asn
        595                 600                 605
Val Thr Leu Asn Ser Gly Thr Gln Phe Asp Leu Met Asn Ile Met Leu
    610                 615                 620
Val Pro Thr Asn Ile Ser Pro Leu Tyr
625                 630

<210> 16
<211> 622
<212> PRT
<213> Bacillus thuringiensis

<400> 16
Met Asn Thr Val Leu Asn Asn Gly Arg Asn Thr Thr Cys His Ala His
1               5                   10                  15
Asn Val Val Ala His Asp Pro Phe Ser Phe Glu His Lys Ser Leu Asn
            20                  25                  30
Thr Ile Glu Lys Glu Trp Lys Glu Trp Lys Arg Thr Asp His Ser Leu
        35                  40                  45
Tyr Val Ala Pro Ile Val Gly Thr Val Gly Ser Phe Leu Leu Lys Lys
    50                  55                  60
Val Gly Ser Leu Val Gly Lys Arg Ile Leu Ser Glu Leu Gln Asn Leu
65                  70                  75                  80
Ile Phe Pro Ser Gly Ser Ile Asp Leu Met Gln Glu Ile Leu Arg Ala
                85                  90                  95
Thr Glu Gln Phe Ile Asn Gln Arg Leu Asn Ala Asp Thr Leu Gly Arg
            100                 105                 110
Val Asn Ala Glu Leu Ala Gly Leu Gln Ala Asn Val Ala Glu Phe Asn
        115                 120                 125
Arg Gln Val Asp Asn Phe Leu Asn Pro Asn Gln Asn Pro Val Pro Leu
    130                 135                 140
Ala Ile Ile Asp Ser Val Asn Thr Leu Gln Gln Leu Phe Leu Ser Arg
145                 150                 155                 160
Leu Pro Gln Phe Gln Ile Gln Gly Tyr Gln Leu Leu Leu Leu Pro Leu
                165                 170                 175
Phe Ala Gln Ala Ala Asn Phe Asn Leu Ser Phe Ile Arg Gly Val Ile
            180                 185                 190
Leu Asn Ala Asp Glu Trp Gly Ile Ser Ala Ala Thr Val Arg Thr Tyr
        195                 200                 205
Arg Asp His Leu Arg Lys Phe His Arg Asp Tyr Ser Asn Tyr Cys Ile
    210                 215                 220
Asn Pro Tyr Gln Thr Ala Phe Arg Gly Leu Asn His Arg Leu Pro Asp
225                 230                 235                 240
Met Leu Glu Phe Arg Thr Tyr Met Phe Leu Asn Val Phe Glu Tyr Val
                245                 250                 255
Ser Ile Trp Ser Leu Phe Lys Tyr Gln Ser Leu Leu Val Ser Ser Gly
            260                 265                 270
Ala Asn Leu Tyr Ala Ser Gly Ser Gly Pro Thr Gln Ser Phe Thr Ala

275                 280                 285
Gln Asn Trp Pro Phe Leu Tyr Ser Leu Phe Gln Val Asn Ser Asn Tyr
    290                 295                 300
Val Leu Asn Gly Leu Ser Gly Ala Arg Thr Thr Ile Thr Phe Pro Asn
305                 310                 315                 320
Ile Gly Gly Leu Pro Val Tyr His Asn Ser Thr Leu His Phe Ala Arg
                325                 330                 335
Ile Asn Tyr Arg Gly Gly Val Ser Ser Ser Arg Ile Gly Gln Ala Asn
            340                 345                 350
Leu Asn Gln Asn Phe Asn Ile Ser Thr Leu Phe Asn Pro Leu Gln Thr
        355                 360                 365
Pro Phe Ile Arg Ser Trp Leu Asp Ser Gly Thr Asp Arg Glu Gly Val
    370                 375                 380
Ala Thr Ser Thr Asn Trp Gln Ser Gly Ala Phe Glu Thr Thr Leu Leu
385                 390                 395                 400
Arg Phe Ser Ile Phe Ser Ala Arg Gly Asn Ser Asn Phe Phe Pro Asp
                405                 410                 415
Tyr Phe Ile Arg Asn Ile Ser Gly Val Val Gly Thr Ile Ser Asn Ala
            420                 425                 430
Asp Leu Ala Arg Pro Leu His Phe Asn Glu Ile Arg Asp Ile Gly Thr
        435                 440                 445
Thr Ala Val Ala Ser Leu Val Thr Val His Asn Arg Lys Asn Asn Ile
    450                 455                 460
Tyr Asp Thr His Glu Asn Gly Thr Met Ile His Leu Ala Pro Asn Asp
465                 470                 475                 480
Tyr Thr Gly Phe Thr Val Ser Pro Ile His Ala Thr Gln Val Asn Asn
                485                 490                 495
Gln Ile Arg Thr Phe Ile Ser Glu Lys Tyr Gly Asn Gln Gly Asp Ser
            500                 505                 510
Leu Arg Phe Glu Leu Ser Asn Pro Thr Ala Arg Tyr Thr Leu Arg Gly
        515                 520                 525
Asn Gly Asn Ser Tyr Asn Leu Tyr Leu Arg Val Ser Ser Ile Gly Ser
    530                 535                 540
Ser Thr Ile Arg Val Thr Ile Asn Gly Arg Val Tyr Thr Ala Asn Val
545                 550                 555                 560
Asn Thr Thr Thr Asn Asn Asp Gly Val Leu Asp Asn Gly Ala Arg Phe
                565                 570                 575
Ser Asp Ile Asn Ile Gly Asn Val Val Ala Ser Ala Asn Thr Asn Val
            580                 585                 590
Pro Leu Asp Ile Gln Val Thr Phe Asn Gly Asn Pro Gln Phe Glu Leu
        595                 600                 605
Met Asn Ile Met Phe Val Pro Thr Asn Leu Pro Pro Leu Tyr
    610                 615                 620

<210> 17
<211> 633
<212> PRT
<213> Bacillus thuringiensis

<400> 17
Met Asn Ser Val Leu Asn Ser Gly Arg Asn Thr Ile Cys Asp Ala Tyr
1               5                   10                  15
Asn Val Val Val His Asp Pro Phe Ser Phe Gln His Lys Ser Leu Asp
            20                  25                  30
Thr Ile Gln Lys Glu Trp Met Glu Trp Lys Lys Asp Asn His Ser Leu
        35                  40                  45
Tyr Val Asp Pro Ile Val Gly Thr Val Ala Ser Phe Leu Leu Lys Lys
    50                  55                  60
Leu Gly Ser Leu Ile Gly Lys Arg Ile Leu Ser Glu Leu Arg Asn Leu
65                  70                  75                  80
Ile Phe Pro Ser Gly Ser Thr Asn Leu Met Glu Asp Ile Leu Arg Glu
                85                  90                  95

Thr Glu Lys Phe Leu Asn Gln Lys Leu Asn Thr Asp Thr Leu Ser Arg
            100                 105                 110
Val Asn Ala Glu Leu Thr Gly Leu Gln Ala Asn Val Glu Glu Phe Asn
        115                 120                 125
Arg Gln Val Asp Asn Phe Leu Asn Pro Asn Arg Asn Ala Val Pro Leu
    130                 135                 140
Ser Ile Thr Ser Ser Val Asn Thr Met Gln Gln Leu Phe Leu Asn Arg
145                 150                 155                 160
Leu Ser Gln Phe Gln Met Gln Gly Tyr Gln Leu Leu Leu Leu Pro Leu
                165                 170                 175
Phe Ala Gln Ala Ala Asn Leu His Leu Ser Phe Ile Arg Asp Val Ile
            180                 185                 190
Leu Asn Ala Glu Glu Trp Gly Ile Ser Ala Ala Thr Leu Arg Thr Tyr
        195                 200                 205
Gln Asn His Leu Arg Asn Tyr Thr Arg Asp Tyr Ser Asn Tyr Cys Ile
    210                 215                 220
Asp Thr Tyr Gln Thr Ala Phe Arg Gly Leu Asn Thr Arg Leu His Asp
225                 230                 235                 240
Met Leu Glu Phe Arg Thr Tyr Met Phe Leu Asn Val Phe Glu Tyr Val
                245                 250                 255
Ser Ile Trp Ser Leu Phe Lys Tyr Gln Ser Leu Leu Val Ser Ser Gly
            260                 265                 270
Ala Asn Leu Tyr Ala Ser Gly Ser Gly Pro Gln Gln Thr Gln Leu Phe
        275                 280                 285
Thr Ser Gln Asp Trp Pro Phe Leu Tyr Ser Leu Phe Gln Val Asn Ser
    290                 295                 300
Asn Tyr Val Leu Ser Gly Phe Ser Gly Ala Ser Leu Phe Thr Thr Phe
305                 310                 315                 320
Pro Asn Ile Gly Gly Leu Pro Gly Ser Thr Thr Thr Gln Ala Leu Leu
                325                 330                 335
Ala Ala Arg Val Asn Tyr Ser Gly Gly Ile Thr Ser Gly Ser Ile Gly
            340                 345                 350
Gly Ser Asn Phe Asn Gln Asn Phe Asn Cys Asn Thr Ile Ser Pro Pro
        355                 360                 365
Leu Ser Thr Ser Phe Val Arg Ser Trp Leu Asp Ser Gly Ser Asp Arg
    370                 375                 380
Gln Gly Val Asn Thr Val Thr Asn Trp Gln Thr Glu Ser Phe Glu Thr
385                 390                 395                 400
Thr Ser Gly Leu Arg Cys Gly Ala Phe Thr Pro Arg Gly Asn Ser Asn
                405                 410                 415
Tyr Tyr Pro Gly Tyr Phe Ile Arg Asn Ile Ser Gly Val Ser Leu Val
            420                 425                 430
Leu Arg Asn Glu Asp Leu Lys Arg Pro Leu Tyr Tyr Asn Glu Lys Arg
        435                 440                 445
Asn Ile Glu Ser Pro Ser Gly Thr Pro Gly Gly Ala Arg Ala Tyr Met
    450                 455                 460
Val Ser Val His Asn Lys Lys Asn Asn Ile Tyr Ala Val His Glu Asn
465                 470                 475                 480
Gly Thr Met Ile His Leu Ala Pro Glu Asp Asn Thr Gly Phe Thr Ile
                485                 490                 495
Ser Pro Ile His Ala Thr Gln Val Asn Asn Gln Thr Arg Thr Phe Ile
            500                 505                 510
Ser Glu Lys Phe Gly Asn Gln Gly Asp Ser Leu Arg Phe Glu Gln Ser
        515                 520                 525
Asn Thr Thr Ala Arg Tyr Thr Leu Arg Gly Asn Gly Asn Ser Tyr Asn
    530                 535                 540
Leu Tyr Leu Arg Val Ser Ser Ile Gly Asn Ser Thr Ile Arg Val Thr
545                 550                 555                 560
Ile Asn Gly Arg Val Tyr Thr Ala Ser Asn Val Asn Thr Thr Thr Asn
                565                 570                 575
Asn Asp Gly Val Asn Asp Asn Gly Ala Arg Phe Ser Asp Ile Asn Ile
            580                 585                 590
Gly Asn Val Val Ala Ser Ser Asn Ser Asp Val Pro Leu Asp Ile Asn

595                 600                 605
Val Thr Leu Asn Ser Gly Thr Gln Phe Asp Leu Met Asn Ile Met Leu
    610                 615                 620
Val Pro Thr Asn Ile Ser Pro Leu Tyr
625                 630

<210> 18
<211> 632
<212> PRT
<213> Bacillus thuringiensis

<400> 18
Met Asn Asn Val Leu Asn Asn Gly Arg Thr Thr Ile Cys Asp Ala Tyr
 1               5                  10                  15
Asn Val Val Ala His Asp Pro Phe Ser Phe Glu His Lys Ser Leu Asp
            20                  25                  30
Thr Ile Arg Lys Glu Trp Met Glu Trp Lys Arg Thr Asp His Ser Leu
        35                  40                  45
Tyr Val Ala Pro Ile Val Gly Thr Val Ser Ser Phe Leu Leu Lys Lys
    50                  55                  60
Val Gly Ser Leu Ile Gly Lys Arg Ile Leu Ser Glu Leu Trp Gly Leu
65                  70                  75                  80
Ile Phe Pro Ser Gly Ser Thr Asn Leu Met Gln Asp Ile Leu Arg Glu
                85                  90                  95
Thr Glu Gln Phe Leu Asn Gln Arg Leu Asn Thr Asp Thr Leu Ala Arg
            100                 105                 110
Val Asn Ala Glu Leu Glu Gly Leu Gln Ala Asn Ile Arg Glu Phe Asn
        115                 120                 125
Gln Gln Val Asp Asn Phe Leu Asn Pro Thr Gln Asn Pro Val Pro Leu
    130                 135                 140
Ser Ile Thr Ser Ser Val Asn Thr Met Gln Gln Leu Phe Leu Asn Arg
145                 150                 155                 160
Leu Pro Gln Phe Arg Val Gln Gly Tyr Gln Leu Leu Leu Leu Pro Leu
                165                 170                 175
Phe Ala Gln Ala Ala Asn Met His Leu Ser Phe Ile Arg Asp Val Val
            180                 185                 190
Leu Asn Ala Asp Glu Trp Gly Ile Ser Ala Ala Thr Leu Arg Thr Tyr
        195                 200                 205
Gln Asn Tyr Leu Lys Asn Tyr Thr Thr Glu Tyr Ser Asn Tyr Cys Ile
    210                 215                 220
Asn Thr Tyr Gln Thr Ala Phe Arg Gly Leu Asn Thr Arg Leu His Asp
225                 230                 235                 240
Met Leu Glu Phe Arg Thr Tyr Met Phe Leu Asn Val Phe Glu Tyr Val
                245                 250                 255
Ser Ile Trp Ser Leu Phe Lys Tyr Gln Ser Leu Leu Val Ser Ser Gly
            260                 265                 270
Ala Asn Leu Tyr Ala Ser Gly Ser Gly Pro Gln Gln Thr Gln Ser Phe
        275                 280                 285
Thr Ser Gln Asp Trp Pro Phe Leu Tyr Ser Leu Phe Gln Val Asn Ser
    290                 295                 300
Asn Tyr Val Leu Asn Gly Phe Ser Gly Ala Arg Leu Thr Gln Thr Phe
305                 310                 315                 320
Pro Asn Ile Gly Gly Leu Pro Gly Thr Thr Thr Thr His Ala Leu Leu
                325                 330                 335
Ala Ala Arg Val Asn Tyr Ser Gly Gly Val Ser Ser Gly Asp Ile Gly
            340                 345                 350
Ala Val Phe Asn Gln Asn Phe Ser Cys Ser Thr Phe Leu Pro Pro Leu
        355                 360                 365
Leu Thr Pro Phe Val Arg Ser Trp Leu Asp Ser Gly Ser Asp Arg Gly
    370                 375                 380
Gly Val Asn Thr Val Thr Asn Trp Gln Thr Glu Ser Phe Glu Ser Thr
385                 390                 395                 400

Leu Gly Leu Arg Cys Gly Ala Phe Thr Ala Arg Gly Asn Ser Asn Tyr
                405                 410                 415
Phe Pro Asp Tyr Phe Ile Arg Asn Ile Ser Gly Val Pro Leu Val Val
            420                 425                 430
Arg Asn Glu Asp Leu Arg Arg Pro Leu His Tyr Asn Glu Ile Arg Asn
        435                 440                 445
Ile Glu Ser Pro Ser Gly Thr Pro Gly Gly Leu Arg Ala Tyr Met Val
    450                 455                 460
Ser Val His Asn Arg Lys Asn Asn Ile Tyr Ala Val His Glu Asn Gly
465                 470                 475                 480
Thr Met Ile His Leu Ala Pro Glu Asp Tyr Thr Gly Phe Thr Ile Ser
                485                 490                 495
Pro Ile His Ala Thr Gln Val Asn Asn Gln Thr Arg Thr Phe Ile Ser
            500                 505                 510
Glu Lys Phe Gly Asn Gln Gly Asp Ser Leu Arg Phe Glu Gln Ser Asn
        515                 520                 525
Thr Thr Ala Arg Tyr Thr Leu Arg Gly Asn Gly Asn Ser Tyr Asn Leu
    530                 535                 540
Tyr Leu Arg Val Ser Ser Leu Gly Asn Ser Thr Ile Arg Val Thr Ile
545                 550                 555                 560
Asn Gly Arg Val Tyr Thr Ala Ser Asn Val Asn Thr Thr Thr Asn Asn
                565                 570                 575
Asp Gly Val Asn Asp Asn Gly Ala Arg Phe Leu Asp Ile Asn Met Gly
            580                 585                 590
Asn Val Val Ala Ser Asp Asn Thr Asn Val Pro Leu Asp Ile Asn Val
        595                 600                 605
Thr Phe Asn Ser Gly Thr Gln Phe Glu Leu Met Asn Ile Met Phe Val
    610                 615                 620
Pro Thr Asn Leu Pro Pro Ile Tyr
625                 630

<210> 19
<211> 1178
<212> PRT
<213> Bacillus thuringiensis

<400> 19
Met Asp Asn Asn Pro Asn Ile Asn Glu Cys Ile Pro Tyr Asn Cys Leu
1               5                   10                  15
Ser Asn Pro Glu Val Glu Val Leu Gly Gly Glu Arg Ile Glu Thr Gly
            20                  25                  30
Tyr Thr Pro Ile Asp Ile Ser Leu Ser Leu Thr Gln Phe Leu Leu Ser
        35                  40                  45
Glu Phe Val Pro Gly Ala Gly Phe Val Leu Gly Leu Val Asp Ile Ile
    50                  55                  60
Trp Gly Ile Phe Gly Pro Ser Gln Trp Asp Ala Phe Leu Val Gln Ile
65                  70                  75                  80
Glu Gln Leu Ile Asn Gln Arg Ile Glu Glu Phe Ala Arg Asn Gln Ala
                85                  90                  95
Ile Ser Arg Leu Glu Gly Leu Ser Asn Leu Tyr Gln Ile Tyr Ala Glu
            100                 105                 110
Ser Phe Arg Glu Trp Glu Ala Asp Pro Thr Asn Pro Ala Leu Arg Glu
        115                 120                 125
Glu Met Arg Ile Gln Phe Asn Asp Met Asn Ser Ala Leu Thr Thr Ala
    130                 135                 140
Ile Pro Leu Phe Ala Val Gln Asn Tyr Gln Val Pro Leu Leu Ser Val
145                 150                 155                 160
Tyr Val Gln Ala Ala Asn Leu His Leu Ser Val Leu Arg Asp Val Ser
                165                 170                 175
Val Phe Gly Gln Arg Trp Gly Phe Asp Ala Ala Thr Ile Asn Ser Arg
            180                 185                 190
Tyr Asn Asp Leu Thr Arg Leu Ile Gly Asn Tyr Thr Asp Tyr Ala Val

        195                 200                 205
Arg Trp Tyr Asn Thr Gly Leu Glu Arg Val Trp Gly Pro Asp Ser Arg
    210                 215                 220
Asp Trp Val Arg Tyr Asn Gln Phe Arg Arg Glu Leu Thr Leu Thr Val
225                 230                 235                 240
Leu Asp Ile Val Ala Leu Phe Pro Asn Tyr Asp Ser Arg Arg Tyr Pro
                245                 250                 255
Ile Arg Thr Val Ser Gln Leu Thr Arg Glu Ile Tyr Thr Asn Pro Val
            260                 265                 270
Leu Glu Asn Phe Asp Gly Ser Phe Arg Gly Ser Ala Gln Gly Ile Glu
        275                 280                 285
Arg Ser Ile Arg Ser Pro His Leu Met Asp Ile Leu Asn Ser Ile Thr
    290                 295                 300
Ile Tyr Thr Asp Ala His Arg Gly Tyr Tyr Tyr Trp Ser Gly His Gln
305                 310                 315                 320
Ile Met Ala Ser Pro Val Gly Phe Ser Gly Pro Glu Phe Thr Phe Pro
                325                 330                 335
Leu Tyr Gly Thr Met Gly Asn Ala Ala Pro Gln Gln Arg Ile Val Ala
            340                 345                 350
Gln Leu Gly Gln Gly Val Tyr Arg Thr Leu Ser Ser Thr Leu Tyr Arg
        355                 360                 365
Arg Pro Phe Asn Ile Gly Ile Asn Asn Gln Gln Leu Ser Val Leu Asp
    370                 375                 380
Gly Thr Glu Phe Ala Tyr Gly Thr Ser Ser Asn Leu Pro Ser Ala Val
385                 390                 395                 400
Tyr Arg Lys Ser Gly Thr Val Asp Ser Leu Asp Glu Ile Pro Pro Gln
                405                 410                 415
Asn Asn Asn Val Pro Pro Arg Gln Gly Phe Ser His Arg Leu Ser His
            420                 425                 430
Val Ser Met Phe Arg Ser Gly Phe Ser Asn Ser Ser Val Ser Ile Ile
        435                 440                 445
Arg Ala Pro Met Phe Ser Trp Ile His Arg Ser Ala Glu Phe Asn Asn
    450                 455                 460
Ile Ile Ala Ser Asp Ser Ile Thr Gln Ile Pro Ala Val Lys Gly Asn
465                 470                 475                 480
Phe Leu Phe Asn Gly Ser Val Ile Ser Gly Pro Gly Phe Thr Gly Gly
                485                 490                 495
Asp Leu Val Arg Leu Asn Ser Ser Gly Asn Asn Ile Gln Asn Arg Gly
            500                 505                 510
Tyr Ile Glu Val Pro Ile His Phe Pro Ser Thr Ser Thr Arg Tyr Arg
        515                 520                 525
Val Arg Val Arg Tyr Ala Ser Val Thr Pro Ile His Leu Asn Val Asn
    530                 535                 540
Trp Gly Asn Ser Ser Ile Phe Ser Asn Thr Val Pro Ala Thr Ala Thr
545                 550                 555                 560
Ser Leu Asp Asn Leu Gln Ser Ser Asp Phe Gly Tyr Phe Glu Ser Ala
                565                 570                 575
Asn Ala Phe Thr Ser Ser Leu Gly Asn Ile Val Gly Val Arg Asn Phe
            580                 585                 590
Ser Gly Thr Ala Gly Val Ile Ile Asp Arg Phe Glu Phe Ile Pro Val
        595                 600                 605
Thr Ala Thr Leu Glu Ala Glu Tyr Asn Leu Glu Arg Ala Gln Lys Ala
    610                 615                 620
Val Asn Ala Leu Phe Thr Ser Thr Asn Gln Leu Gly Leu Lys Thr Asn
625                 630                 635                 640
Val Thr Asp Tyr His Ile Asp Gln Val Ser Asn Leu Val Thr Tyr Leu
                645                 650                 655
Ser Asp Glu Phe Cys Leu Asp Glu Lys Arg Glu Leu Ser Glu Lys Val
            660                 665                 670
Lys His Ala Lys Arg Leu Ser Asp Glu Arg Asn Leu Leu Gln Asp Ser
        675                 680                 685
Asn Phe Lys Asp Ile Asn Arg Gln Pro Glu Arg Gly Trp Gly Gly Ser
    690                 695                 700

Thr Gly Ile Thr Ile Gln Gly Gly Asp Asp Val Phe Lys Glu Asn Tyr
705                 710                 715                 720
Val Thr Leu Ser Gly Thr Phe Asp Glu Cys Tyr Pro Thr Tyr Leu Tyr
                725                 730                 735
Gln Lys Ile Asp Glu Ser Lys Leu Lys Ala Phe Thr Arg Tyr Gln Leu
            740                 745                 750
Arg Gly Tyr Ile Glu Asp Ser Gln Asp Leu Glu Ile Tyr Leu Ile Arg
        755                 760                 765
Tyr Asn Ala Lys His Glu Thr Val Asn Val Pro Gly Thr Gly Ser Leu
    770                 775                 780
Trp Pro Leu Ser Ala Gln Ser Pro Ile Gly Lys Cys Gly Glu Pro Asn
785                 790                 795                 800
Arg Cys Ala Pro His Leu Glu Trp Asn Pro Asp Leu Asp Cys Ser Cys
                805                 810                 815
Arg Asp Gly Glu Lys Cys Ala His His Ser His His Phe Ser Leu Asp
            820                 825                 830
Ile Asp Val Gly Cys Thr Asp Leu Asn Glu Asp Leu Gly Val Trp Val
        835                 840                 845
Ile Phe Lys Ile Lys Thr Gln Asp Gly His Ala Arg Leu Gly Asn Leu
    850                 855                 860
Glu Phe Leu Glu Glu Lys Pro Leu Val Gly Glu Ala Leu Ala Arg Val
865                 870                 875                 880
Lys Arg Ala Glu Lys Lys Trp Arg Asp Lys Arg Glu Lys Leu Glu Trp
                885                 890                 895
Glu Thr Asn Ile Val Tyr Lys Glu Ala Lys Glu Ser Val Asp Ala Leu
            900                 905                 910
Phe Val Asn Ser Gln Tyr Asp Gln Leu Gln Ala Asp Thr Asn Ile Ala
        915                 920                 925
Met Ile His Ala Ala Asp Lys Arg Val His Ser Ile Arg Glu Ala Tyr
    930                 935                 940
Leu Pro Glu Leu Ser Val Ile Pro Gly Val Asn Ala Ala Ile Phe Glu
945                 950                 955                 960
Glu Leu Glu Gly Arg Ile Phe Thr Ala Phe Ser Leu Tyr Asp Ala Arg
                965                 970                 975
Asn Val Ile Lys Asn Gly Asp Phe Asn Asn Gly Leu Ser Cys Trp Asn
            980                 985                 990
Val Lys Gly His Val Asp Val Glu Glu Gln Asn Asn Gln Arg Ser Val
        995                 1000                1005
Leu Val Val Pro Glu Trp Glu Ala Glu Val Ser Gln Glu Val Arg Val
    1010                1015                1020
Cys Pro Gly Arg Gly Tyr Ile Leu Arg Val Thr Ala Tyr Lys Glu Gly
1025                1030                1035                1040
Tyr Gly Glu Gly Cys Val Thr Ile His Glu Ile Glu Asn Asn Thr Asp
                1045                1050                1055
Glu Leu Lys Phe Ser Asn Cys Val Glu Glu Glu Ile Tyr Pro Asn Asn
            1060                1065                1070
Thr Val Thr Cys Asn Asp Tyr Thr Val Asn Gln Glu Glu Tyr Gly Gly
        1075                1080                1085
Ala Tyr Thr Ser Arg Asn Arg Gly Tyr Asn Glu Ala Pro Ser Val Pro
    1090                1095                1100
Ala Asp Tyr Ala Ser Val Tyr Glu Glu Lys Ser Tyr Thr Asp Gly Arg
1105                1110                1115                1120
Arg Glu Asn Pro Cys Glu Phe Asn Arg Gly Tyr Arg Asp Tyr Thr Pro
                1125                1130                1135
Leu Pro Val Gly Tyr Val Thr Lys Glu Leu Glu Tyr Phe Pro Glu Thr
            1140                1145                1150
Asp Lys Val Trp Ile Glu Ile Gly Glu Thr Glu Gly Thr Phe Ile Val
        1155                1160                1165
Asp Ser Val Glu Leu Leu Leu Met Glu Glu
    1170                1175

<210> 20

<211> 652
<212> PRT
<213> Bacillus thuringiensis

<400> 20
Met Ile Arg Lys Gly Gly Arg Lys Met Asn Pro Asn Asn Arg Ser Glu
1               5                   10                  15
His Asp Thr Ile Lys Thr Thr Glu Asn Asn Glu Val Pro Thr Asn His
            20                  25                  30
Val Gln Tyr Pro Leu Ala Glu Thr Pro Asn Pro Thr Leu Glu Asp Leu
        35                  40                  45
Asn Tyr Lys Glu Phe Leu Arg Met Thr Ala Asp Asn Asn Thr Glu Ala
    50                  55                  60
Leu Asp Ser Ser Thr Thr Lys Asp Val Ile Gln Lys Gly Ile Ser Val
65                  70                  75                  80
Val Gly Asp Leu Leu Gly Val Val Gly Phe Pro Phe Gly Gly Ala Leu
                85                  90                  95
Val Ser Phe Tyr Thr Asn Phe Leu Asn Thr Ile Trp Pro Ser Glu Asp
            100                 105                 110
Pro Trp Lys Ala Phe Met Glu Gln Val Glu Ala Leu Met Asp Gln Lys
        115                 120                 125
Ile Ala Asp Tyr Ala Lys Asn Lys Ala Leu Ala Glu Leu Gln Gly Leu
    130                 135                 140
Gln Asn Asn Val Glu Asp Tyr Val Ser Ala Leu Ser Ser Trp Gln Lys
145                 150                 155                 160
Asn Pro Val Ser Ser Arg Asn Pro His Ser Gln Gly Arg Ile Arg Glu
                165                 170                 175
Leu Phe Ser Gln Ala Glu Ser His Phe Arg Asn Ser Met Pro Ser Phe
            180                 185                 190
Ala Ile Ser Gly Tyr Glu Val Leu Phe Leu Thr Thr Tyr Ala Gln Ala
        195                 200                 205
Ala Asn Thr His Leu Phe Leu Leu Lys Asp Ala Gln Ile Tyr Gly Glu
    210                 215                 220
Glu Trp Gly Tyr Glu Lys Glu Asp Ile Ala Glu Phe Tyr Lys Arg Gln
225                 230                 235                 240
Leu Lys Leu Thr Gln Glu Tyr Thr Asp His Cys Val Lys Trp Tyr Asn
                245                 250                 255
Val Gly Leu Asp Lys Leu Arg Gly Ser Ser Tyr Glu Ser Trp Val Asn
            260                 265                 270
Phe Asn Arg Tyr Arg Arg Glu Met Thr Leu Thr Val Leu Asp Leu Ile
        275                 280                 285
Ala Leu Phe Pro Leu Tyr Asp Val Arg Leu Tyr Pro Lys Glu Val Lys
    290                 295                 300
Thr Glu Leu Thr Arg Asp Val Leu Thr Asp Pro Ile Val Gly Val Asn
305                 310                 315                 320
Asn Leu Arg Gly Tyr Gly Thr Thr Phe Ser Asn Ile Glu Asn Tyr Ile
                325                 330                 335
Arg Lys Pro His Leu Phe Asp Tyr Leu His Arg Ile Gln Phe His Thr
            340                 345                 350
Arg Phe Gln Pro Gly Tyr Tyr Gly Asn Asp Ser Phe Asn Tyr Trp Ser
        355                 360                 365
Gly Asn Tyr Val Ser Thr Arg Pro Ser Ile Gly Ser Asn Asp Ile Ile
    370                 375                 380
Thr Ser Pro Phe Tyr Gly Asn Lys Ser Ser Glu Pro Val Gln Asn Leu
385                 390                 395                 400
Glu Phe Asn Gly Glu Lys Val Tyr Arg Ala Val Ala Asn Thr Asn Leu
                405                 410                 415
Ala Val Trp Pro Ser Ala Val Tyr Ser Gly Val Thr Lys Val Glu Phe
            420                 425                 430
Ser Gln Tyr Asn Asp Gln Thr Asp Glu Ala Ser Thr Gln Thr Tyr Asp
        435                 440                 445
Ser Lys Arg Asn Val Gly Ala Val Ser Trp Asp Ser Ile Asp Gln Leu
    450                 455                 460

Pro Pro Glu Thr Thr Asp Glu Pro Leu Glu Lys Gly Tyr Ser His Gln
465                 470                 475                 480
Leu Asn Tyr Val Met Cys Phe Leu Met Gln Gly Ser Arg Gly Thr Ile
                485                 490                 495
Pro Val Leu Thr Trp Thr His Lys Ser Val Asp Phe Phe Asn Met Ile
            500                 505                 510
Asp Ser Lys Lys Ile Thr Gln Leu Pro Leu Val Lys Ala Tyr Lys Leu
        515                 520                 525
Gln Ser Gly Ala Ser Val Val Ala Gly Pro Arg Phe Thr Gly Gly Asp
    530                 535                 540
Ile Ile Gln Cys Thr Glu Asn Gly Ser Ala Ala Thr Ile Tyr Val Thr
545                 550                 555                 560
Pro Asp Val Ser Tyr Ser Gln Lys Tyr Arg Ala Arg Ile His Tyr Ala
                565                 570                 575
Ser Thr Ser Gln Ile Thr Phe Thr Leu Ser Leu Asp Gly Ala Pro Phe
            580                 585                 590
Asn Gln Tyr Tyr Phe Asp Lys Thr Ile Asn Lys Gly Asp Thr Leu Thr
        595                 600                 605
Tyr Asn Ser Phe Asn Leu Ala Ser Phe Ser Thr Pro Phe Glu Leu Ser
    610                 615                 620
Gly Asn Asn Leu Gln Ile Gly Val Thr Gly Leu Ser Ala Gly Asp Lys
625                 630                 635                 640
Val Tyr Ile Asp Lys Ile Glu Phe Ile Pro Val Asn
                645                 650

<210> 21
<211> 35
<212> DNA
<213> Artificial Sequence

<220>
<223> oligonucleotide primer

<221> misc_feature
<222> 15, 17, 18, 20
<223> n = A,T,C or G

<400> 21
cttccttcgg cgtgnwnnwn cccatcctcg gcggc                                35

<210> 22
<211> 35
<212> DNA
<213> Artificial Sequence

<220>
<223> oligonucleotide primer

<221> misc_feature
<222> 15, 17, 18, 20
<223> n = A,T,C or G

<400> 22
cttccttcgg cgtgnsnnsn cccatcctcg gcggc                                35

<210> 23
<211> 35
<212> DNA
<213> Artificial Sequence

<220>
<223> oligonucleotide primer

<221> misc_feature
<222> 15, 17, 18, 20
<223> n = A,T,C or G

<400> 23
cttccttcgg cgtgnwnnsn cccatcctcg gcggc                                35

<210> 24
<211> 35
<212> DNA
<213> Artificial Sequence

<220>
<223> oligonucleotide primer

<221> misc_feature
<222> 15, 17, 18, 20
<223> n = A,T,C or G

<400> 24
cttccttcgg cgtgnsnnwn cccatcctcg gcggc                                35

<210> 25
<211> 40
<212> DNA
<213> Artificial Sequence

<220>
<223> oligonucleotide primer

<221> misc_feature
<222> 17, 19, 20, 22
<223> n = A,T,C or G

<400> 25
cggcgtctac agagganwnn wngatcttca gcacaactgg                           40

<210> 26
<211> 40
<212> DNA
<213> Artificial Sequence

<220>
<223> oligonucleotide primer

<221> misc_feature
<222> 17, 19, 20, 22
<223> n = A,T,C or G

<400> 26
cggcgtctac agaggansnn sngatcttca gcacaactgg                           40

<210> 27
<211> 40
<212> DNA
<213> Artificial Sequence

<220>
<223> oligonucleotide primer

<221> misc_feature
<222> 17, 19, 20, 22

<223> n = A,T,C or G

<400> 27
cggcgtctac agaggansnn wngatcttca gcacaactgg                                40

<210> 28
<211> 40
<212> DNA
<213> Artificial Sequence

<220>
<223> oligonucleotide primer

<221> misc_feature
<222> 17, 19, 20, 22
<223> n = A,T,C or G

<400> 28
cggcgtctac agagganwnn sngatcttca gcacaactgg                                40

<210> 29
<211> 35
<212> DNA
<213> Artificial Sequence

<220>
<223> oligonucleotide primer

<400> 29
cgccttcctc ctctcagtga agagcaacta cttcc                                     35